# EUROPEAN PATENT SPECIFICATION

(11) **EP 1 546 322 B1**
(45) Date of publication and mention of the grant of the patent: **05.01.2011**
(21) Application number: 03748973.9
(22) Date of filing: 24.07.2003
(51) Int. Cl.: C12N 15/00, C12N 15/09, C12N 15/63, C12N 15/70, C12N 15/74

(54) **TRANSPOSON-BASED VECTORS AND METHODS OF NUCLEIC ACID INTEGRATION**
VEKTOREN AUF TRANSPOSONBASIS UND VERFAHREN ZUR INTEGRATION VON NUKLEINSÄUREN
VECTEURS ET PROCEDES D'INTEGRATION D'ACIDE NUCLEIQUE BASES SUR DES TRANSPOSONS

(30) Priority: 24.07.2002 US 398628 P
(43) Date of publication of application: 29.06.2005
(73) Proprietor: Manoa Biosciences Inc., Honolulu HI 96822 (US)
(72) Inventor: KAMINSKI, Joseph, M., Med. College of Georgia, Augusta, GA 30912 (US)
(74) Representative: Neuefeind, Regina
(86) International application number: PCT/US2003/023090
(87) International publication number: WO 2004/009792

(56) References cited:
- EP-A- 1 308 516
- WO-A-01/30965
- WO-A-02/08286
- WO-A-96/35777
- WO-A-96/37626
- WO-A-97/20038
- US-A1- 2002 103 152
- US-A1- 2002 132 350
- US-B1- 6 225 121
- US-B1- 6 461 864
- YANT S R ET AL: "SOMATIC INTEGRATION AND LONG-TERM TRANSGENE EXPRESSION IN NORMAL AND HAEMOPHILIC MICE USING A DNA TRANSPOSON SYSTEM" NATURE GENETICS, NATURE AMERICA, NEW YORK, US, vol. 25, May 2000 (2000-05), pages 35-41, XP002946960 ISSN: 1061-4036
- MATTHEAKIS L C ET AL: "Expression of Cre recombinase as a reporter of signal transduction in mammalian cells" CHEMISTRY AND BIOLOGY, CURRENT BIOLOGY, LONDON, GB, vol. 6, no. 11, November 1999 (1999-11), pages 835-844, XP002265769 ISSN: 1074-5521
- DORGAI L ET AL: "IDENTIFYING DETERMINANTS OF RECOMBINATION SPECIFICITY: CONTRUCTION AND CHARACTERIZATION OF MUTANT BACTERIOPHAGE INTEGRASES" JOURNAL OF MOLECULAR BIOLOGY, LONDON, GB, vol. 252, 1995, pages 178-188, XP002941865 ISSN: 0022-2836
- HOLMES-SON M L ET AL: "MOLECULAR GENETICS AND TARGET SITE SPECIFICITY OF RETROVIRAL INTEGRATION" ADVANCES IN GENETICS, ACADEMIC PRESS, XX, vol. 43, 2001, pages 33-69, XP009048763 ISSN: 0065-2660
- VEGA M A: "PROSPECTS FOR HOMOLOGOUS RECOMBINATION IN HUMAN GENE THERAPY" HUMAN GENETICS, BERLIN, DE, vol. 87, no. 3, 1991, pages 245-253, XP002052226 ISSN: 0340-6717
- KAMINSKI J M ET AL: "DESIGN OF A NONVIRAL VECTOR FOR SITE-SELECTIVE, EFFICIENT INTEGRATION INTO THE HUMAN GENOME" FASEB JOURNAL (FEDERATION OF AMERICAN SOCIETIES FOR EXPERIMENTAL BIOLOGY), BETHESDA, US, vol. 16, no. 10, August 2002 (2002-08), pages 1242-1247, XP009033354 ISSN: 0892-6638
- AKOPIAN A ET AL: "Chimeric recombinases with designed DNA sequence recognition" PROCEEDINGS OF THE NATIONAL ACADEMY OF SCIENCES OF USA, NATIONAL ACADEMY OF SCIENCE, WASHINGTON, DC, US, vol. 100, no. 15, 22 July 2003 (2003-07-22), pages 8688-8691, XP002289806 ISSN: 0027-8424
- ANTON M. ET AL.: 'Site-specific recombination mediated by an adenovirus vector expressing the cre recombinase protein: a molecular switch for control of gene expression' JOURNAL OF VIROLOGY vol. 69, no. 8, August 1995, pages 4600 - 4606, XP002912537

### SEQUENCE LISTING

<110> Kaminski, Joseph
<120> TRANSPOSON-BASED VECTORS AND METHODS OF
   NUCLEIC ACID INTEGRATION
<130> 11000.0001P1
<150> 60/398,628
   <151> 2002-07-24
<160> 7
<170> FastSEQ for Windows Version 4.0
<210> 1
   <211> 19
   <212> DNA
   <213> Artificial Sequence
<220>
   <223> Description of Artificial Sequence:/note = synthetic construct
<400> 1
   tccggccgga accggcttt 19
<210> 2
   <211> 33
   <212> DNA
   <213> Artificial Sequence
<220>
   <223> Description of Artificial Sequence:/note = synthetic construct
<400> 2
   cgggatccca cctatggaag gaagatcaga ttt 33
<210> 3
   <211> 35
   <212> DNA
   <213> Artificial Sequence
<220>
   <223> Description of Artificial Sequence:/note =
   synthetic construct
<400> 3
   agattactcg agtcaaagtg ttttgtatga tctcg 35
<210> 4
   <211> 18
   <212> DNA
   <213> Artificial Sequence
<220>
   <223> Description of Artificial Sequence:/note =
   synthetic construct
<400> 4
   aagatctgat ccgtcgac 18
<210> 5
   <211> 33
   <212> DNA
   <213> Artificial Sequence
<220>
   <223> Description of Artificial Sequence:/note =
   synthetic construct
<400> 5
   cgggatccca cctatgaatt atggcgtgga gaa 33
<210> 6
   <211> 35
   <212> DNA
   <213> Artificial Sequence
<220>
   <223> Description of Artificial Sequence:/note =
   synthetic construct
<400> 6
   agattactcg agtcagttgt acagctgcaa tccca 35
<210> 7
   <211> 19
   <212> DNA
   <213> Artificial Sequence
<220>
   <223> Description of Artificial Sequence:/note =
   synthetic construct
<400> 7
   ggaagccctg caaagtaaa 19

## Description

### I. BACKGROUND OF THE INVENTION

1. Research has revealed three major components for efficient transport of viral and non-viral vectors through the cytoplasmic membrane and into the nucleus of eukaryotic cells. These include a specific ligand for receptor mediated endocytosis, an endosomal disruption factor, and a nuclear localizing signal. These components have been employed successfully in non-viral vectors (1-6). In vectors that lack or fail to interact with a nuclear localizing signal, efficient transfection will only occur in those cells that are actively dividing. The three DNA requirements for integration are (1) the sequence of DNA, (2) a local host DNA structure, and (3) the associated endogenous DNA-binding proteins [45]. For integration to occur an enzyme (e.g., transposase) is required to mediate the process. This enzyme can be a transposase or a sitespecific recombinase. Site-specific recombinases allow recombination, and some do not require cofactors thereby allowing activity outside their normal environment. For example, Cre recombinase, although derived from *Escherichia coli* phage P1, acts efficiently in plant, yeast, and mammalian cells (18). Site-selective recombinases such as FLP, Cre, and β-recombinase perform both integration and excision efficiently with the same target sites; however, the net integration frequency is low (e.g. 0.03% for Cre) (18-20).

2. Limitations of viral vectors such as pathogenicity, expense in production, and systemic instability have proved to be major obstacles to the use of viral based systems. In fact, re-administration of viral based vectors can promote immune responses that can result in life threatening systemic effects and limit gene-transfer efficacy (64-65). Non-viral vectors (i.e., lipid-based, polymer-based, lipid-polymer-based, and polylysine) are a synthetic means of encapsulating transgenic DNA until it reaches the cellular target. Compared to viral vectors, non-viral vectors are safer to prepare; the risk of pathogenic and immunologic complications is diminished. Non-viral vectors have been designed by modifying the surface of the non-viral vector for targeted therapy (7-12). Liposomes are typically internalized into endosomes, which are then frequently directed to lysosomes, thus degrading the plasmid. Endosomal disruption factors and nuclear localizing signals have been employed in these vectors. However, the lipoplexes (plasmid DNA and liposome) are mainly limited to transfecting dividing cells unless a nuclear localizing factor is present or interacts with the vector (16). Furthermore, efficient host integration does not occur except in transposon-based plasmids (17-20). Nevertheless, liposomes have demonstrated their safety in human gene therapy trials (21-24).

3. Transposons are mobile, in that they can move from one position on DNA to a second position on DNA in the presence of a transposase. There are two fundamental components of any mobile cut-and-paste type transposon system, a source of an active transposase and the DNA sequences that are recognized and mobilized by the transposase. Mobilization of the DNA sequences permits the intervening nucleic acid between the recognized DNA sequences to also be mobilized.

4. Integrase and retrotransposase depend upon their own DNA-binding domain or an interaction with a host DNA directing factor to direct the DNA-enzyme complex (e.g., transposon/transposase) in juxtaposition to the host DNA for integration to occur (25, 35-37).
If the host does not have this directing factor or a specific host-DNA sequence recognized by the transposon/transposase complex, the efficiency of integration decreases substantially (25, 38). For example, a specific human endogenous protein, integrase interacting 1, has been shown to affiliate with integrase and stimulate integration in vitro and possibly in vivo by binding and directing integrase to DNase 1 hypersensitive sites (25). Alternatively, the yeast retrovirus-like element Ty3 inserts at the transcription start sites of genes transcribed by RNA polymerase III because of its affiliation with this complex (37). Furthermore, some transposases or integrases require certain sites in the host DNA for catalytic activity even if the DNA-enzyme complex is brought into the vicinity of the host-DNA. For example, Tc1/mariner transposon integrates into a TA dinucleotide (32).

5. DNA transposable elements for genetic manipulation have been available for over 15 years. This technology has been applied in both bacteria and eukaryotes to verify whether or not a cloned DNA fragment contains the whole functional gene of interest. Rubin and Spradling first demonstrated this for P elements of D. melanogaster. A fragment of DNA carrying the rosy gene was inserted within the terminal repeats of a P element and then cloned into a plasmid. This plasmid and another encoding the transposase were injected into the embryos of an M strain with a deletion in the rosy gene. About 50% of the flies derived from the injected embryos possessed rosy phenotype, thereby suggesting that the rosy gene inserted into the chromosome and maintained its function at various sites within the genome. Furthermore, none of the flanking plasmid DNA was integrated in the host genome suggesting that excision from the plasmid only took place at the terminal repeats (39).

6. In order for a vector encoded nucleic acid to be incorporated into the target DNA, integration must occur. The putative model of integration is similar in retroviruses, transposons, and retrovirus-like retrotransposons. For example, the catalytic domain is conserved in integrases and transposases. In vitro reactions have shown that integrase or transposase are the only enzymes necessary for integration (25-28) Integrase and many transposases in bacteria and eukaryotes have been shown to bind specifically to the att site at the ends of the terminal repeats. They require the presence of CA at the 3' end for both processing and cleavage/ligation (29-30).

7. Transposons have many applications in genetic manipulation of a host genome, including transgenic delivery and insertional mutagenesis. However, the efficiency of transposon integration can vary substantially among cell lines, suggesting the involvement of host factors. Based upon the requirements for integration of the transposable elements, it appears a host DNA directing factor is necessary for efficient integration by juxtaposing the transposon-transposase complex adjacent to the host DNA. The requirement for a host DNAdirecting factor has been established in retroviruses and retroviral-like retrotransposons. For example, the yeast retrovirus-like element Ty3 inserts at the transcription start sites of genes transcribed by RNA polymerase III because of its interaction with this complex [82]. Alternatively, integrase of the human immunodeficieny virus affiliates with the human endogenous protein integrase interacting 1 to stimulate integration in vitro and possibly in vivo [83, 25]. In fact, Tc1/mariner transposases also have DNA binding domains. However, these DNA binding domains apparently are not site selective (35), possibly lack strong recognition sites in certain host genomes, and may require other host proteins for efficient integration by docking the transposon-transposase to the host DNA.

8. In many cases, the host does not have the required docking factor such as a DNA sequence recognized by the transposase or an endogenous factor that juxtaposes the transposon-transposase complex to the host DNA. Thus, the efficiency of integration in these hosts will be markedly reduced. Furthermore, even if the transposon-transposase complex is docked to the host DNA, integration may still not occur because the DNA site has to be permissive. The present invention overcomes the problems associated with the currently known non-viral vector systems.

WO 2002/08286 describes DNA binding proteins comprising zinc finger domains. Three separate nucleic acid constructs are required for assembling of a zinc finger protein with the respective zinc finger domains. WO 2001/30965 relates to methods and compositions for introducing a nucleic acid into the genome of at least one cell, wherein a non-chimeric Sleeping Beauty transposon is used which does not comprise a DNA binding domain.

### II. SUMMARY OF THE INVENTION

9. In accordance with the purposes of this invention, as embodied and broadly described herein, this invention, in one aspect, relates to compositions comprising a nucleic acid construct for site-selective integration into the genome of a subject and methods of their use.

10. Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### III. BRIEF DESCRIPTION OF THE DRAWINGS

11. The accompanying drawings, which are incorporated in and constitute a part of this specification, illustrate several embodiments of the invention and together with the description, serve to explain the principles of the invention. Specific examples of the invention are seen in Examples 1-9.

12. Figure 1 shows one plasmid encoding a site-selective transposase. The diagram provides a general explanation of an aspect of the invention. IR=Inverted Repeats; E,P=Enhancer, Promoter.

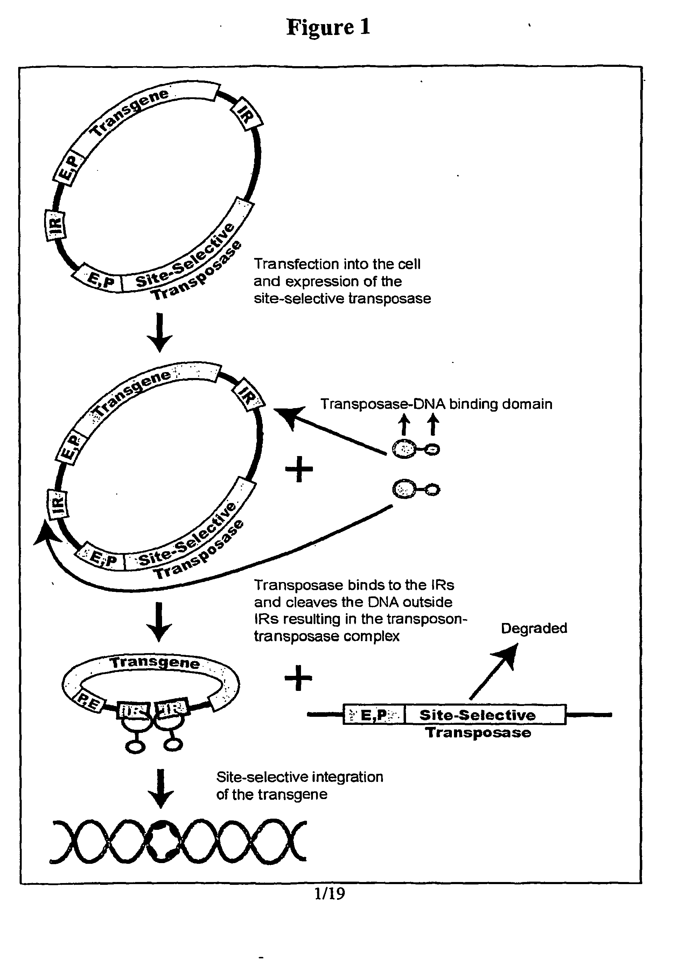

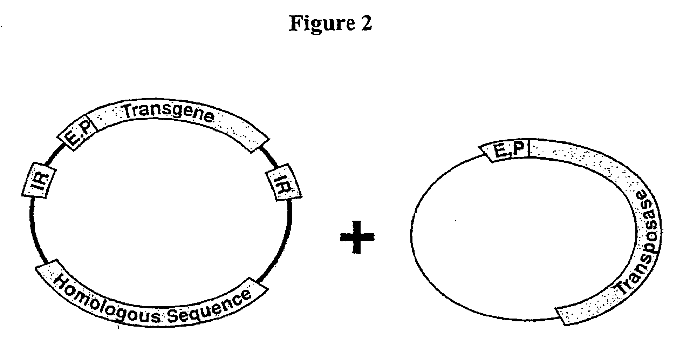

13. Figure 2 shows two plasmids, one coding for the transgene and the other the transposase (or chimeric transposase-host directing factor). The former plasmid has a transgene flanked by inverted terminal repeats (or the like) and also contains a sequence similar to the host. In this representation, the similar sequence acts primarily to dock this plasmid to the complimentary host DNA. The transposase plasmid could be under an inducible promoter that would allow temporal regulation of the transposase. Cleavage of the DNA outside the terminal repeats and integration would occur as diagrammed in Figure 1. IR=Inverted
Repeats; E,P=Enhancer, Promoter.

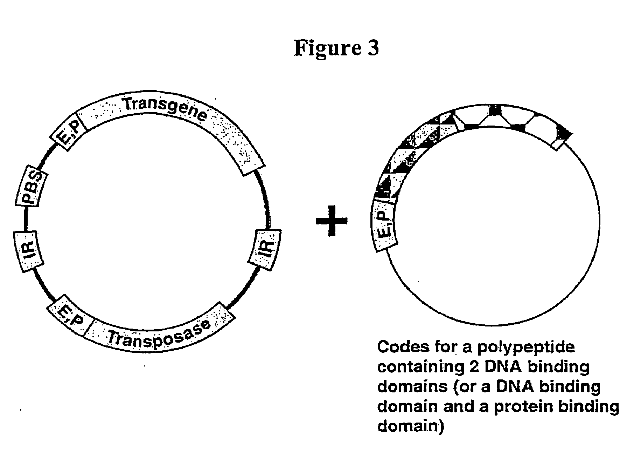

14. Figure 3 shows two plasmids, the transposon-based [coding for the transgene and containing a protein binding site (PBS)] and the other for a fusion polypeptide containing two DNA binding domains (or a DNA binding and a protein binding domains). The fusion polypeptide would then bind to the PBS and direct the transposon plasmid to the host DNA site recognized by the DNA binding domain of the fusion protein (or a protein that is associated with the host DNA). The transposase could be under an inducible promoter that would allow temporal regulation of the transposase. Cleavage of the DNA outside the terminal repeats and integration would occur as diagrammed in Figure 1. IR=Inverted Repeats; E,P=Enhancer, Promoter; PBS= Protein Binding Site.

15. Figure 4 shows the nonviral nucleic acid construct contained within a non-specific cationic coat with transposase bound to the inverted terminal repeats which are flanking the transgene. Minus signs= negative charge of the DNA; Positive Signs= Postive charge of the non-viral package (e.g. lipid bilayer composed of DOPE, a cationic lipid, and polyethylene glycol-ceramide).

16. Figure 5 shows the nonviral nucleic acid construct with a packaging system including a specific ligand, endosomal disruption factor, and nuclear localizing signal. Minus signs= negative charge of the DNA; Positive Signs= Postive charge of the non-viral package (e.g. lipid bilayer composed of DOPE, a cationic lipid, and polyethylene glycol-ceramide); Three Pronged Circles=Ligand for selective cell targeting; Squiggly lines=Endosomal disruption factor; Solid Circles=Nuclear localizing signal.

17. Figure 6 shows the nonviral nucleic acid construct with said packaging system of figure 5; however, the transposase contains a host-DNA directing factor. Minus signs= negative charge of the DNA; Positive Signs= Postive charge of the non-viral package (e.g. lipid bilayer composed of DOPE, a cationic lipid, and polyethylene glycol-ceramide); Three Pronged Circles=Ligand for selective cell targeting; Squiggly lines=Endosomal disruption factor; Solid Circles=Nuclear localizing signal; Triangle on the transposase=DNA-binding domain.

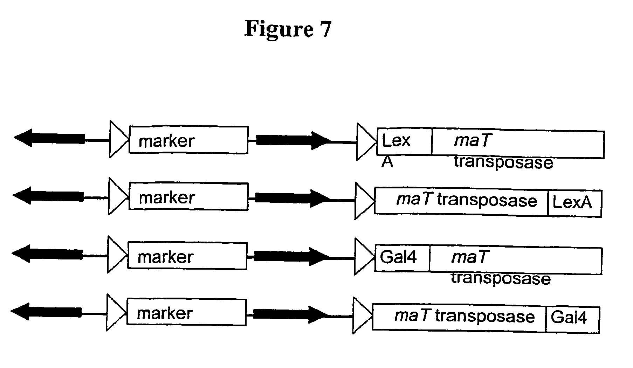

18. Figure 7 shows genetic constructs to assess targeted maT integration in insect cells. Four different plasmids are introduced into insect cells, each carrying 1) a modified maT transposon, with intact ITRs (solid black arrows) and an interrupted transposase ORF containing a selectable marker gene and inducible promoter (triangle) and 2) a chimeric transposase with a LexA or Ga14 DNA binding domain fused to either the 5' or 3' ends of the transposase sequence. An additional target plasmid (not shown), carrying LexA or Gal4 target sites will be co-delivered into the cells or embryos, and following induction of the transposase, recombinant target plasmids carrying the modified transposon will be examined for targeted integrations.

19. Figure 8 shows a construct schematic of a Mos1 chimeric transposon with the recognition sequences for the Ga14 and LexA DNA binding domains and a nuclear localization signal.

20. Figure 9 shows the domain organization of gpNu1

21. Figure 10 shows the chimeric construt of the gpNu1 DNA binding domains and the integrase catalytic domain.

22. Figure 11 shows agarose gel analysis of purified DNA fragments. Lane M, molecular weight markers. Lane 1, pKKT7(-H) linearized with *Eco*RI and *Hind*III. Lane 2, PCR product of gpNulΔE85 sequence digested with *Eco*RI and *Hpa*I. Lane 3, PCR product of gpNulΔP141 sequence digested with *Eco*RI and *Hpa*I. Lane 4, PCR product of IntC170 sequence digested with *Not*I and *Hind*III. Note that the fluorescence of this PCR product is quenched by the loading dye in the gel.

23. Figure 12 shows the cloning strategy for the construction of pNu1ΔE85-IntC170 and gpNu1ΔP141-IntC170.

24. Figure 13 shows a schematic representation of the MBP-Zif-Cre fusion protein.

25. Figure 14 shows over-expression and purification of the MBP-Zif-Cre fusion protein.
The position of the expressed fusion protein is indicated.

26. Figure 15 shows an analysis of the biological activity of MBP-Zif-Cre and MBP-Cre proteins. 5µg of plasmid DNA were mixed with purified fusion protein and incubated at 37°C for 15 min. The reactions were subsequently purified as described and aliquots were digested with the restriction enzyme AflIII.

27. Figure 16 shows a schematic representation of the MBP-Zif-Cre protein. The surface probability is indicated as is the structure of the DNA target sites, which can be used to assess the recombinatorial activity of the MBP-Zif-Cre fusion protein.

28. Figure 17 shows a schematic representation of the sleeping beauty construct and a chimeric transposase using sleeping beauty.

29. Figure 18 shows the introduction of a unique restriction site at the N-terminus of Sleeping Beauty.

30. Figure 19 shows the introduction of a DNA binding domain (either from zif268 or similar), along with a flexible peptide linker, into that restriction site

### IV. DETAILED DESCRIPTION

31. The present invention may be understood more readily by reference to the following detailed description of preferred embodiments of the invention and the Examples included therein and to the Figures and their previous and following description.

32. Before the present compounds, compositions, articles, devices, and/or methods are disclosed and described, it is to be understood that this invention is not limited to specific synthetic methods, specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

33. Throughout this application, reference is made to various proteins and nucleic acids. It is understood that any names used for proteins or nucleic acids are art-recognized names, such that the reference to the name constitutes a disclosure of the molecule itself.

### A. Definitions

34. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

35. Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

36. In this specification and in the claims which follow, reference will be made to a number of terms which shall be defined to have the following meanings:

37. "Optional" or "optionally" means that the subsequently described event or circumstance may or may not occur, and that the description includes instances where said event or circumstance occurs and instances where it does not.

38. By "treating" is meant that an improvement in the disease state, i.e., genetic disorder, autoimmune disease, cancer, viral infection, bacterial infection, or parasitic infection is observed and/or detected upon administration of a substance of the present invention to a subject. Treatment can range from a positive change in a symptom or symptoms of the disease to complete amelioration of the genetic disorder, autoimmune disease, cancer, viral infection, bacterial infection, or parasitic infection, (e.g., reduction in severity or intensity of disease, alteration of clinical parameters indicative of the subject's condition, relief of discomfort or increased or enhanced function), as detected by art-known techniques. The methods of the present invention can be utilized to treat an established genetic disorder, autoimmune disease, cancer, viral infection, bacterial infection, or parasitic infection. One of skill in the art would recognize that genetic disorder, autoimmune disease, cancer, viral infection, bacterial infection, or parasitic infection refer to conditions characterized by the presence of a foreign pathogen or abnormal cell growth. Clinical symptoms will depend on the particular condition and are easily recognizeable by those skilled in the art of treating the specific condition.

39. By "preventing" is meant that after administration of a substance of the present invention to a subject, the subject does not develop the full symptoms of the condition (e.g., genetic disorder, autoimmune disease, cancer, viral, bacterial, or parasitic infection, and/or does not develop the genetic disorder, autoimmune disease, cancer, viral, bacterial, or parasitic infection). Thus, the condition is completely prevented or some recognized symptom or indicia of the condition is prevented or its full manifestation prevented.

40. By "transposable elements" is meant any genetic construct including but not limited to any gene, gene fragment, or nucleic acid that can be integrated into a target DNA sequence under control of an integrating enzyme.

41. By "terminal repeat" is meant any repetitive sequence within a sequence of nucleic acids including but not limited to inverted repeats and direct repeats.

42. By "vector" is meant any composition capable of delivering a nucleic acid, peptide, polypeptide, or protein into a target nucleic acid, cell, tissue, or organism including but not limited to plasmid, phage, transposons, retrotransposons, viral vector, and retroviral vector.

43. By "non-viral vector" is meant any vector that does not comprise a virus or retrovirus.

### B. Compositions

44. The invention provides compositions comprising a nucleic acid comprising a transgene
flanked by two terminal repeats and a nucleic acid encoding an integrating enzyme under the control of a promoter element, wherein the integrating enzyme is a chimeric integrating enzyme comprising a host-specific DNA binding domain and wherein the nucleic acid encoding the transgene and the nucleic acid encoding the chimeric integrating enzyme are the same nucleic acid, and wherein the chimeric integrating enzyme is a transposase.

45. As used herein, plasmids are agents that transport the disclosed nucleic acids into the cell without degradation and allow promoter-driven expression of the protein-encoding nucleic acids (e.g., transgene and integrating enzyme) in the cells into which they are delivered. In some embodiments the promoters and enhancers are derived from either a virus or a retrovirus.

46. Also disclosed are compositions of the invention, wherein the nucleic acid is present in a non-viral vector.

47. Also disclosed are compositions of the invention, wherein the promoter element is a promoter/enhancer.

48. Also disclosed are compositions of the invention, wherein the promoter is a sitespecific promoter.

49. It has been shown that all specific regulatory elements can be cloned and used to construct expression vectors that are selectively expressed in specific cell types. The sitespecific promoter can be selected at least from the group consisting of the glial fibrillary acetic protein (GFAP) promoter, myelin basic promoter (MBP), MCK promoter, NSE promoter, nestin promoter, synapsin promoter, Insulin 2 (Ins2) promoter, PSA promoter, albumin promoter, TRP-1 promoter and the tyrosinase promoter. Also disclosed is a promoter specific for breast tissue, such as the WAP promoter, a promoter specific for ovarian tissue, such as the ACTB promoter, or a promoter specific for bone tissue. Any tissues specific promoter can be used.

50. Also disclosed are compositions of the invention, wherein the promoter is inducible.
The inducible promoter can be selected at least from the group consisting of human heat shock promoter, Egr-1 promoter, tetracycline promoter, and the human glandular kallikrein 2 (hK2) promoter.

51. As the transposable element will need to be integrated into the host genome, an intergrating enzyme is needed. Intergrating enzymes can be any transposase.

52. The chimeric integrating enzymes of the present invention comprise two components:
DNA docking factor (first domain) (e.g., DNA Binding Domain (DBD)) and an integrating (enzymatic) domain (second domain). The DNA docking factor can be be arranged anywhere in relation to the integrating domain (e.g. internally, or at the amino or carboxy termini).
Furthermore, a portion of the wild-type integrating enzyme, for example, the portion that has the DBD of the native enzyme, could be deleted and replaced with a DBD that recognizes DNA of the target cell. The chimeric proteins of the invention comprise a first domain that attaches the chimeric protein to target nucleic acid, and a second domain that integrates donor nucleic acid (transgene) into the target nucleic acid. As employed herein, the phrase "chimeric protein" refers to a genetically engineered recombinant protein wherein the domains thereof are derived from heterologous coding regions (i.e., coding regions obtained from different genes). General molecular methods, and specifically those of Katz et al. (U.S. Patent No. 6,150,511,) can be used to construct a chimeric transposase of the invention.

53. The chimeric integrating enzyme proteins of the invention are prepared by recombinant DNA methods, in which the DNA sequences encoding each domain are "operably linked" together such that upon expression, a fusion protein is generated having the targeting and transposase functions described previously. As used herein, the term "operably linked" means that the DNA segments encoding the fusion protein are assembled with respect to each other, and with respect to an expression vector in which they are inserted, in such a manner that a functional fusion protein is effectively expressed.

54. As used herein, "first domain" refers to the domain within the chimeric protein that functions to attach the chimeric protein to a specific recognition sequence on a target nucleic acid. The first domain is at least 5 amino acids in length and can be located anywhere within the chimeric protein, e.g., internally, or at the amino or carboxy termini thereof. The first domain can be a DNA docking factor, either a "DNA-binding domain" or a "protein-binding domain" that is operative to couple and/or associate the chimeric protein with a recognition sequence on the target nucleic acid.

55. By "DNA docking factor" is meant any amino acid sequence that associates with DNA directly or indirectly. Thus when the association of the chimeric integrating enzyme with the target nucleic acid occurs by indirect binding, a protein-binding domain is employed as the docking factor. Suitable protein-binding domains may be obtained from viral transcription factors (e.g., HSV-VP16 and adenovirus E1A) and cellular transcription factors. Throughout the present disclosure, the terms DNA binding domain, DNA directing factor, and protein binding domain are used to refer to DNA docking factors. It is understood that these terms may be used interchangeably thoughout the present invention without affecting the overall goal of the invention.

56. As used herein, the term "DNA-binding domain" encompasses a minimal peptide sequence of a DNA-binding protein, up to the entire length of a DNA-binding protein without losing function. When a DNA-binding domain is employed in the invention, the association of the chimeric integrating enzyme with the target nucleic acid occurs by direct interaction with the host nucleic acid. The DNA-binding domain brings the second domain (i.e., the integrating domain) in close proximity to a specific recognition sequence on the target nucleic acid so that a desired donor nucleic acid can be integrated into the target nucleic acid sequence.

57. DNA-binding domains are typically derived from DNA-binding proteins. Such DNAbinding domains are known to function heterologously in combination with other functional protein domains by maintaining the ability to bind the natural DNA recognition sequence (see, e.g., Brent and Ptashne, 1985, Cell, 43:729-736).
For example, hormone receptors are known to have interchangeable DNA-binding domains that function in chimeric proteins (see, e.g., U.S. Pat. No. 4,981,784; and Evans, R., 1988, Science, 240:889-895.

58. "DNA-binding protein(s)" utilized herein belong to a well-known class of proteins that are able to directly bind DNA and perform a variety of functions, such as facilitate initiation of transcription or repression of transcription. Exemplary DNA-binding proteins for use herein include transcription control proteins (e.g., transcription factors and the like; Conaway and Conaway, 1994, "Transcription Mechanisms and Regulation", Raven Press Series on Molecular and Cellular Biology, Vol. 3, Raven Press, Ltd., New York, N.Y.;
); recombination enzymes (e.g., hin recombinase, and the like); and DNA modifying enzymes (e.g., restriction enzymes, and the like).

59. Transcription factors with DNA-binding proteins suitable for use herein include, e.g., homeobox proteins, zinc finger proteins, hormone receptors, helix-turn-helix proteins, helix-loop-helix proteins, basic-Zip proteins (bZip), beta-ribbon factors, and the like. See, for example, Harrison, S., "A Structural Taxonomy of DNA-binding Domains," Nature, 353:715-719.

60. Homeobox DNA-binding proteins suitable for use herein include, but are not limited to HOX, STF-1 (Leonard et al., 1993, Mol. Endo., 7:1275-1283), Antp, Mat, alpha.-2, INV, (see, also, Scott et al. (1989), Biochem. Biophys. Acta, 989:25-48). It has been found by Leonart et al., that a fragment of 76 amino acids (corresponding to a.a. 140-215 described in Leonard et al.,1993, Mol. Endo., 7:1275-1283) containing the STF-1 homeodomain binds DNA as tightly as wild-type STF-1 .

61. Zinc fingers can be manipulated to recognize a broad range of sequences. As such, these enzymes have the potential to direct cleavage to arbitrarily chosen targets. A double-strand break (DSB) in the chromosomal target greatly enhances the frequency of localized recombination events. Zinc-finger nucleases (ZFNs) have a DNA recognition domain composed of three Cys₂His₂ zinc fingers linked to a nonspecific DNA cleavage domain (Y.G. Kim et al. (1996) Proc. Natl. Acad. Sci. U.S.A. 93, 1156). To act as a nuclease, the cleavage domain can dimerize (J. Smith et al. (2000) Nucleic Acids Res. 28, 3361). This can be achieved by providing binding sites for two sets of zinc fingers in close proximity and in the appropriate orientations (J. Smith et al. (2000) Nucleic Acids Res. 28, 3361; M. Bibikova et al. (2001) Mol. Cell. Biol. 21, 289). Suitable zinc finger DNA-binding proteins provided for use herein include but are not limited to Zif268, GLI, and XFin. These proteins may be found throughout the literature via Klug and Rhodes (1987), Trends Biochem. Sci., 12:464; Jacobs and Michaels (1990), New Biol., 2:583; and Jacobs (1992), EMBO J., 11:4507-4517 .

62. Exemplary hormone receptor DNA-binding proteins for use herein include but are not limited to glucocorticoid receptor, thyroid hormone receptor, and estrogen receptor are described in the literature(U.S. Pat. Nos. 4,981,784; 5,171,671; and 5,071,773,).

63. Suitable helix-turn-helix DNA-binding proteins for use herein include but are not limited to lambda-repressor, cro-repressor, 434 repressor, and 434-cro. These helix-turn-helix DNA-binding proteins are provided (Pabo and Sauer, 1984, Annu. Rev. Biochem., 53:293-321).

64. Exemplary helix-loop-helix DNA-binding proteins for use herein include but are not limited to MRF4 (Block et al., 1992, Mol. and Cell Biol., 12(6): 2484-2492,), CTF4 (Tsay et al., 1992, NAR, 20(10): 2624), NSCL, PAL2, and USF. See, for review, Wright (1992), Current Opinion in Genetics and Development, 2(2):243-248; Kadesch, T. (1992), Immun. Today, 13(1): 31-36; and Garell and Campuzano (1991), Bioessays, 13(10): 493-498.

65. Exemplary basic Zip DNA-binding proteins for use herein include but are not limited to GCN4, fos, and jun (see, for review, Lamb and McKnight, 1991, Trends Biochem. Sci., 16:417-422). Exemplary .beta.-ribbon factors provided for use herein include, Met-J, ARC, and MNT.

66. Recombination enzymes with suitable DNA-binding proteins for use herein include but are not limited to the hin family of recombinases (e. g., hin, gin, pin, and cin; see, Feng et al., 1994, Science, 263:348-355,), the .lambda.-integrase family, flp-recombinase, TN916 transposons, and the resolvase family (e.g., TN21 resolvase).

67. DNA-modifying enzymes with suitable DNA-binding proteins for use herein include, for example, restriction enzymes, DNA-repair enzymes, and site-specific methylases. For use in the instant invention, restriction enzymes are modified using methods well-known in the art to remove the restriction digest function from the protein while maintaining the DNA-binding function (see, e.g., King et al., 1989, J. Biol. Chem., 264 (20):11807-11815,). Thus, any restriction enzyme may be employed herein. The utilization of a restriction enzyme recognizing a rare DNA sequence permits attachment of the invention chimeric protein to relatively few sites on a particular stretch of genomic DNA.

68. The modification of existing DNA-binding domains to recognize new target recognition sequences is also contemplated herein. It has been found that in vitro evolution methods can be applied to modify and improve existing DNA-binding domains. Devlin et al., 1990, Science, 249:404-406; and Scott and Smith, 1990, Science, 249:386-390 for teachings on modification of existing DNA-binding domains.

69. "Protein-binding domain(s)" suitable for use as the "first domain" of the invention chimeric protein is typically derived from proteins able to bind another protein (e.g., a transcription factor) that is either directly or indirectly attached (coupled) to the target nucleic acid sequence. Thus, when a protein-binding domain is employed as the first domain, the association of the invention chimeric protein with the target nucleic acid
occurs by indirect binding. Suitable protein-binding domains may be obtained, for example, from viral transcription factors (e.g., HSV-VP16, adenovirus E1A, and the like), cellular transcription factors, and the like using routine molecular methods.

70. In addition to readily available protein-binding domains, small protein-binding domains, e.g., in the range of about 5-25 amino acids, can be obtained employing "phage display library" methods described (Rebar and Pabo, 1994, Science, 263:671-673). It has been found that short peptides can be isolated using phage display libraries that bind to a selected protein. For example, a peptide was obtained from a library displaying random amino-acid hexamers on the surface of a phage that bound specifically to avidin; this peptide bore no similarity to any known avidin ligands (Devlin et al., 1990, Science, 249:404-406). This well-known method is used to create protein-binding domains that bind to proteins already bound in vivo to desired target nucleic acid.

71. Microsatellite regions are repetitive sequences in the genome. By targeting repetitive sequences whether through a chimeric integrating enzyme or through homologous sequences one can target integration into non-transcribed regions of the genome (i.e. eliminating the risk of insertional mutagenesis) and by having more targets increasing the efficiency of integration, i.e. many targets are better than one. There are repetitive, non-coding regions in the genome that allow integration as described herein, followed by transcription of the transgene driven by the promoter provided in the construct.

72. The chimeric integrating enzyme of the invention comprises an integrating (enzymatic) domain (second domain). The integrating domain comprises or is derived from an integrating enzyme. Intergrating enzymes can be any.

73. Disclosed are compositions, wherein the integrating enzyme is a transposase. It is understood and herein contemplated that the transposase of the composition is not limited and to any one transposase and can be selected from at least the group consisting of *Sleeping Beauty* (SB), *Tn7, Tn5, mos1, piggybac, Himar1, Hermes,* To12 element, *Pokey, Minos,* S elements, P-element, ICESt1, Quetzal elements, *Tn916. maT,* Tc1/mariner and Tc3.

74. Where the integrating enzyme is a transposase, it is understood that the transposases of the composition is not limited and to any one transposases and can be selected from at least the group consisting of Sleeping Beauty (SB), *Tn7, Tn5, Tn916, Tc1*/*mariner,* Minos and S elements, Quetzal elements, Txr elements, *maT, mos1, piggybac,* Himar1, Hermes, Tol2 element, Pokey, P-element, and Tc3. Additional transposases may be found throughout the art, for example, U.S. Patent No. 6,225,121, U.S. Patent No. 6,218,185 U.S. Patent No. 5,792,924 U.S. Patent No. 5,719,055, U.S. Patent Application No. 20020028513, and U.S. Patent Application No. 20020016975. Since the applicable principal of the invention remains the same, the compositions of the invention can include chimeric transposases constructed from transposases not yet identified.

75. Also disclosed are integrating enzymes wherein the enzyme is an integrase. For example, the integrating enzyme can be a bacteriophage integrase. Such integrase can include any bacteriophage integrase and can include but is not limited to lamda (λ) bacteriophage and mu (µ) bacteriophage, as well as Hong Kong 022 (Cheng Q., et al. Specificity determinants for bacteriophage Hong Kong 022 integrase: analysis of mutants with relaxed core-binding specificities. (2000) Mol Microbiol. 36(2):424-36.), HP1 (Hickman, A. B., et al. (1997). Molecular organization in sitespecific recombination: The catalytic domain of bacteriophage HP1 integrase at 2.7 A resolution. Cell 89: 227-237), P4 (Shoemaker, NB, et al. (1996). The Bacteroides mobilizable insertion element, NBU1, integrates into the 3' end of a Leu-tRNA gene and has an integrase that is a member of the lambda integrase family. J Bacteriol. 178(12):3594-600.), P1 (Li Y, and Austin S. (2002) The P1 plasmid in action: timelapse photomicroscopy reveals some unexpected aspects of plasmid partition. Plasmid. 48(3):174-8.), and T7 (Rezende, L.F., et al. (2002) Essential Amino Acid Residues in the Single-stranded DNA-binding Protein of Bacteriophage T7. Identification of the Dimer Interface. J. Biol. Chem. 277, 50643-50653.).

76. Integrase maintains its activity when fused to other proteins. This has been demonstrated by the use of the lambda repressor-integrase (40) and maltose binding proteinintegrase fusion proteins (41). Additionally, chimeric recombinases, transcription factors, oncogenes, etc. have maintained their activity when fused to other protein domains (42). However, attempts of in vivo targeting of site-selective retroviruses that included sequences encoding integrase fusion proteins have not yet been demonstrated (43-45). The Tc1/mariner elements are promiscuous and have been successfully used as transgene vectors from one species to another in flies (49-53), mosquitoes (54), bacteria (55), protozoa (56), and vertebrates.

77. Also disclosed are integrating enzymes wherein the enzyme is a recombinase. For example, the recombinase can be a Cre recombinase, Flp recombinase, HIN recombinase, or any other recombinase. Recombinases are well-known in the art. An extensive list of recombinases can be found in Nunes-Duby SE, et al. (1998) Nuc. Acids Res. 26(2): 391-406, for its teachings on recombinases and their sequences.

78. Also disclosed are integrating enzymes wherein the enzyme is a retrotransposase. For example, the retrotransposase can be a Gate retrotransposase (Kogan GL, et al. (2003) The GATE retrotransposon in Drosophila melanogaster: mobility in heterochromatin and aspects of its expression in germline tissues. Mol Genet Genomics. 269(2):234-42).

79. The chimeric integrating enzyme of the invention can have the host specific binding domain fused to the transposase's N-terminus.

80. The chimeric integrating enzyme of the invention can have the host specific binding domain is fused to the transposase's C-terminus.

81. Also provided are compositions comprising a nucleic acid construct encoding a transgene under the control of a promoter element flanked by two internal repeats and a nucleic acid enocoding a integrating enzyme under the control of a promoter element. Some internal repeats (e.g., some short and long interspersed nuclear elements), are permissive for site-selective integration (68-69) and would allow for transgene expression even without nuclear matrix attachment regions flanking the transgene (66-67). Proteins that selectively bind to interspersed repeat elements have been identified (70-73) and are herein incorporated by reference. Development of fusion proteins incorporating DNA binding domains to known transcription-permissive, repetitive DNA sequences allow targeted integration as described earlier.

82. In the transgene flanked by the terminal repeats, the terminal repeats can be derived from known transposons. Examples of transposons include, but are not limited to the following:
Sleeping Beauty (Izsvak Z, Ivics Z, and Plasterk RH. (2000) Sleeping Beauty, a wide host-range transposon vector for genetic transformation in vertebrates. J. Mol. Biol. 302:93-102), *mos1* (Bessereau JL, et al. (2001) Mobilization of a Drosophila transposon in the Caenorhabditis elegans germ line. Nature. 413(6851):70-4; Zhang L. et al. (2001) DNA-binding activity and subunit interaction of the mariner transposase. Nucleic Acids Res. 29(17):3566-75, *piggybac* (Tamura T, et al. Germline transformation of the silkworm Bombyx mori L. using a piggyBac transposon-derived vector. Nat Biotechnol. 2000 Jan; 18(1):81-4), Himar1 (Lampe DJ, et al. (1998) Factors affecting transposition of the Himar1 mariner transposon in vitro. Genetics. 149(1):179-87), Hermes, Tol2 element, Pokey, Tn5 (Bhasin A, et al. (2000) Characterization of a Tn5 pre-cleavage synaptic complex. J Mol Biol 302:49-63), Tn7 (Kuduvalli PN, Rao JE, Craig NL. (2001) Target DNA structure plays a critical role in Tn7 transposition. EMBO J 20:924-932) , Tn916 (Marra D, Scott JR. (1999) Regulation of excision of the conjugative tranposon Tn916. Mol Microbiol 2:609-621), Tc1/mariner (Izsvak Z, Ivics Z, Hackett PB. (1995) Characterization of a Tc-1 like transposable element in zebrafish (Danio rerio). Mol. Gen. Genet. 247:312-322), Minos and S elements (Franz G and Savakis C. (1991) Minos, a new transposable element from Drosophila hydei, is a member of the Tc1-like family of transposons. Nucl. Acids Res. 19:6646; Merriman PJ, Grimes CD, Ambroziak J, Hackett DA, Skinner P, and Simmons MJ. (1995) S elements: a family of Tc1-like transposons in the genome of Drosophila melanogaster. Genetics 141:1425-1438), Quetzal elements (Ke Z, Grossman GL, Cornel AJ, Collins FH. (1996) Quetzal: a transposon of the Tc1 family in the mosquito Anopheles albimanus. Genetica 98:141-147); Txr elements (Lam WL, Seo P, Robison K, Virk S, and Gilbert W. (1996) Discovery of amphibian Tc1-like transposon families. J Mol Biol 257:359-366), Tc1-like transposon subfamilies (Ivics Z, Izsvak Z, Minter A, Hackett PB. (1996) Identification of functional domains and evolution of Tc1-like transposable elements. Proc. Natl. Acad Sci USA 93: 5008-5013), Tc3 (Tu Z, Shao H. (2002) Intra- and inter-specific diversity of Tc-3 like tranposons in nematodes and insects and implications for their evolution and transposition. Gene 282:133-142), ICESt1 (Burrus V et al. (2002) The ICESt1 element of Streptococcus thermophilus belongs to alarge family of integrative and conjugative elements that exchange modules and change their specificity of integration. Plasmid. 48(2): 77-97), *maT*, and P-element (Rubin GM and Spradling AC. (1983) Vectors for P element mediated gene transfer in Drosophila. Nucleic Acids Res. 11:6341-6351).

83. Translocation of Sleeping Beauty (SB) transposon requires specific binding of SB transposase to inverted terminal repeats (ITRs) of about 230 bp at each end of the transposon, which is followed by a cut-and-paste transfer of the transposon into a target DNA sequence. The ITRs contain two imperfect direct repeats (DRs) of about 32 bp. The outer DRs are at the extreme ends of the transposon whereas the inner DRs are located inside the transposon, 165-166 bp from the outer DRs. Cui et al. (J. Mol Biol 318:1221-1235) investigated the roles of the DR elements in transposition. Within the 1286-bp element, the essential regions are contained in the intervals bounded by coordinates 229-586, 735-765, and 939-1066, numbering in base pairs from the extreme 5' end of the element. These regions may contain sequences that are necessary for transposase binding or that are needed to maintain proper spacing between binding sites.

84. Transposons are bracketed by terminal inverted repeats that contain binding sites for the transposase. Elements of the IR/DR subgroup of the Tc1/mariner superfamily have a pair of transposase-binding sites at the ends of the 200-250 bp long inverted repeats (IRs) (Izsvak, et al. 1995). The binding sites contain short, 15-20 bp direct repeats (DRs). This characteristic structure can be found in several elements from evolutionarily distant species, such as Minos and S elements in flies (Franz and Savakis, 1991; Merriman et al, 1995), Quetzal elements in mosquitos (Ke et al, 1996), Txr elements in frogs (Lam et al, 1996) and at least three Tc1-like transposon subfamilies in fish (Ivics et al., 1996), including SB [Sleeping Beauty] .

85. Whereas Tc1 transposons require one binding site for their transposase in each IR, Sleeping Beauty requires two direct repeat (DR) binding sites within each IR, and is therefore classified with Tc3 in an IR/DR subgroup of the Tc1/mariner superfamily (96,97). Sleeping Beauty transposes into TA dinucleotide sites and leaves the Tc1/mariner characteristic footprint, i.e., duplication of the TA, upon excision. The nucleic acid construct contains the transgene that is flanked by IR/DR sequences, which act as the binding sites for the transposase. The catalytically active tranposase may be expressed from a separate (trans) or same (cis) plasmid system. The transposase binds to the IR/DRs, catalyzes the excision of the flanked transgene, and mediates its integration into the target host genome.

86. Tc3 of Caenorhabditis elegans is one of the founding members of the Tc1 family which includes DNA transposons in vertebrates, insects, nematodes and fungi. Tu A, et al. (Gene 282:133-142) present the characterization of a number of Tc3-like transposons in C. elegans, Caenorhabditis briggsae, and Drosophila melanogaster, which has revealed high levels of inter-and intra-specific diversity and further suggests a broad distribution of the Tc3-like transposons. These newly defined transposons and the previously described Tc3 and MsqTc3 form a highly divergent yet distinct clade in the Tc1 family. The majority of the Tc3-like transposons contain two putative binding sites for their transposases. The first is near the terminus and the second is approximately 164-184 bp from the first site. There is a large amount of variation in the length (27-566 bp) and structure of the terminal inverted repeats (TIRs) of Tc3-like transposons.

87. *Mos1* is a member of the *mariner*/Tc*1* family of transposable elements originally identified in *Drosophila mauritiana.* It has 28 bp terminal inverted repeats and like other elements of this type it transposes by a cut and paste mechanism, inserts at TA dinucleotides and codes for a transposase. This is the only protein required for transposition *in vitro.* Zhang and colleagues (Nucleic Acids Res 29:3566-3575) have investigated the DNA binding properties of *Mos1* transposase and the role of transposase-transposase interactions in transposition. Purified transposase recognises the terminal inverted repeats of *Mos1* due to a DNA-binding domain in the N-terminal 120 amino acids. This requires a putative helix-turn-helix motif between residues 88 and 108. Binding is preferentially to the right hand end, which differs at four positions from the repeat at the left end. Cleavage of *Mos1* by transposase is also preferentially at the right hand end.

88. Based upon the requirements for integration of the transposable elements, it appears a host DNA directing factor is necessary for efficient integration by juxtaposing the transposon-transposase complex adjacent to the host DNA. Indeed, Tc1/mariner transposases do have DNA binding domains. However, these DNA binding domains apparently are not site selective (35), possibly lack strong recognition sites in certain host genomes, and may require other host proteins for efficient integration by docking the transposon-transposase to the host DNA.

89. The invention overcomes this shortcoming by providing compositions comprising a nucleic acid construct further comprising a chimeric integrating enzyme (i.e., integrating enzyme-host DNA binding domain) to bypass the potential requirement of a host DNA directing factor(s) for efficient, site-selective integration. It is understood that the chimeric integrating enzyme can be any chimeric transposase.

90. Thus, disclosed are compositions comprising a transgene flanked by terminal repeats of a transposable element, e.g. Sleeping Beauty, and a required chimeric enzyme (e.g., host DNA binding domain-transposase) in a non-viral packaging system for targeted integration into the host genome. It is an embodiment of the present invention that this chimeric enzyme would substitute the native DNA binding domain of the integrating enzyme with one that is host specific and site-selective, thereby bypassing the requirement of a host-DNA directing factor.

91. Also disclosed are compositions of the invention, wherein the transposase is a chimeric transposase comprising a host-specific or site-specific DNA binding domain.

92. Thus, the present invention relates to novel chimeric transposases and the transposons that are used to introduce nucleic acid sequences into the DNA of a cell. A transposase is an enzyme that is capable of binding to DNA at regions of DNA termed inverted repeats. Transposons typically contain at least one, and preferably two, inverted repeats that flank an intervening nucleic acid sequence. The transposase binds to recognition sites in the inverted repeats and catalyzes the incorporation of the transposon into host DNA. Transposon function is frequently limited to the host species. Even in those transposons that are not limited to their "normal host" the efficiency of integration varies dramatically. This invention increases the efficiency of integration by modifying a transposase to include a host DNAbinding domain (whether for the purpose of site selectiveness or not) as described herein. The novel DNA binding domain of this chimeric transposase can be added to the native transposases or it can substitute for the DNA binding domain of the native transposase. Thus, the host DNA [directing factor] chimeric transposase, recognition sites on the plasmid that would recognize an endogenous protein (or a newly introduced protein) that would then direct the complex to the vicinity of the host-DNA, incorporating host-like sequences (e.g., repetitive sequences) or a combination of the above play roles in the site-selective and/or efficient transgene integration provided by the present invention.

93. Gene transfer vectors for gene therapy can be broadly classified as viral vectors or non-viral vectors. The use of the nucleic acid constructs comprising a chimeric integrating enzyme provides an important and suprising improvement over the non-viral DNA-mediated gene transfer. Up to the present time, viral vectors have been the focus of gene therapy efforts, because they have been found to be more efficient at introducing and expressing genes in cells than non-viral vectors. Once the efficiency problems of the prior art are overcome, as taught herein, there are several advantages to non-viral gene transfer over virus-mediated gene transfer for the development of new gene therapies. For example, adapting viruses as agents for gene therapy restricts genetic design to the constraints of that virus genome in terms of size, structure and regulation of expression. Non-viral vectors are generated largely from synthetic starting materials and are therefore more easily manufactured than viral vectors. Non-viral reagents are less likely to be immunogenic than viral agents making repeat administration possible. Non-viral vectors are more stable than viral vectors and therefore are better suited for pharmaceutical formulation and application than are viral vectors.

94. In past embodiements, non-viral gene transfer systems have not been equipped to promote integration of nucleic acid into the DNA of a cell, including host chromosomes. As a result, stable gene transfer frequencies using non-viral systems have been very low; 0.1% at best in tissue culture cells and much less in primary cells and tissues. The prior art efforts at transposon-based non-viral vectors have attempted to provide a non-viral nucleic acid construct gene transfer system that facilitates integration and markedly improves the frequency of stable gene transfer. However, the integration is not site specific and is not uniformly efficient, and may vary markedly depending upon the host cell line. This invention allows for site-selective integration into the host genome, and provides the suprising advantage of efficient integration in those hosts that do not have the required DNA directing factor as mentioned herein.

95. In the gene transfer system of this invention, the chimeric integrating enzyme can be introduced into the cell as a protein or as nucleic acid encoding the protein. In one embodiment the nucleic acid encoding the protein is RNA and in another, the nucleic acid is DNA. Further, nucleic acid encoding the chimeric transposase protein can be incorporated into a cell through a viral vector, cationic lipid, or other standard transfection mechanisms including electroporation or particle bombardment used for eukaryotic cells. Following or concurrent with introduction of the nucleic acid encoding chimeric transposae, the nucleic acid fragment of this invention can be introduced into the same cell. Alternatively the nucleic acid encoding the chimeric transposase can be the same nucleic acid that includes the trangene and terminal repeats.

96. Similarly, the nucleic acid fragment can be introduced into the cell as a linear fragment or as a circularized fragment. Preferably the nucleic acid sequence comprises at least a portion of an open reading frame to produce a functional amino-acid containing product. In a preferred embodiment the nucleic acid sequence encodes at least one active or functional peptide, polypeptide, or protein, and includes at least one promoter selected to direct expression of the open reading frame or coding region of the nucleic acid sequence. The protein encoded by the nucleic acid sequence can be any of a variety of recombinant proteins new or known in the art. In one embodiment the protein encoded by the nucleic acid sequence is a marker protein such as green fluorescent protein (GFP), chloramphenicol acetyltransferase (CAT), growth hormones, for example to promote growth in a transgenic animal, beta-galactosidase (lacZ), luciferase (LUC), and insulin-like growth factors (IGFs).

97. The gene transfer system of this invention can readily be used to produce transgenic animals that carry a particular marker or express a particular protein in one or more cells of the animal. Methods for producing transgenic animals are known in the art and the incorporation of the gene transfer system of this invention into these techniques does not require undue experimentation. Further, a review of the production of biopharmaceutical proteins in the milk of transgenic dairy animals (see Young et al., BIO PHARM (1997), 10, 34-38) and the references provided therein, detail methods and strategies for producing recombinant proteins in milk and are encorporated herein in their entirety for teachings related to production of biopharmaceutical proteins. The methods and the gene transfer system of this invention can be readily incorporated into these transgenic techniques without undue experimentation in view of what is known in the art and particularly in view of this disclosure.

98. Also disclosed is a transgenic animal, wherein the transgenic animal acts as a bioreactor, the protein is a product for isolation from a cell. Transgenic animals as bioreactors are known. Protein can be produced in quantity in milk, urine, blood or eggs. Promoters are known that promote expression in milk, urine, blood or eggs and these include, but are not limited to, casein promoter, the mouse urinary protein promoter, beta-globin promoter and the ovalbumin promoter respectively. Recombinant growth hormone, recombinant insulin, and a variety of other recombinant proteins have been produced using other methods for producing protein in a cell. Nucleic acids encoding these or other proteins can be incorporated into the nucleic acid fragment of this invention and introduced into a cell. Efficient incorporation of the nucleic acid fragment into the DNA of a cell occurs when a chimeric transposase as described herein is present. Where the cell is part of a tissue or part of a transgenic animal, large amounts of recombinant protein can be obtained. There are a variety of methods for producing transgenic animals for research or for protein production. The following references are for their teachings on methods of producing transgenic animals (Hackett et al. (1993). The molecular biology of transgenic fish. In Biochemistry and Molecular Biology of Fishes (Hochachka & Mommsen, eds) Vol.2, pp. 207-240. Other methods for producing transgenic animals include the teachings of M. Markkula et al., Rev. Reprod., 1, 97-106 (1996); R. T. Wall et al., J. Dairy Sci, 80, 2213-2224 (1997); J. C. Dalton, et al., Adv. Exp. Med. Biol., 411, 419-428 (1997); and H. Lubon et al., Transfus. Med. Rev.,10, 131-143 (1996). Transgenic zebrafish were made, as described by Hackett et al (Patent Application #20020016975). Transposon-based systems have also been tested through the introduction of the nucleic acid with a marker protein into mouse embryonic stem cells (ES) and it is known that these cells can be used to produce transgenic mice (A. Bradley et al., Nature, 309, 255-256 (1984)).

99. In general, there are two methods to achieve improved stocks of commercially important animals. The first is classical breeding, which has worked well for land animals, but it takes decades to make major changes. A review by Hackett et al. (1997) points out that by controlled breeding, growth rates in coho salmon (Oncorhynchus kisutch) increased 60% over four generations and body weights of two strains of channel catfish (Ictalurus punctatus) were increased 21 to 29% over three generations. The second method is genetic engineering, a selective process by which genes are introduced into the chromosomes of animals or plants to give these organisms a new trait or characteristic, like improved growth or greater resistance to disease. The results of genetic engineering have exceeded those of breeding in some cases. In a single generation, increases in body weight of 58% in common carp (Cyprinus carpio) with extra rainbow trout growth hormone I genes, more than 1000% in salmon with extra salmon growth hormone genes, and less in trout were obtained. The advantage of genetic engineering in fish, for example, is that an organism can be altered directly in a very short periods of time if the appropriate gene has been identified (see Hackett, 1997). The disadvantage of genetic engineering in fish is that few of the many genes that are involved in growth and development have been identified and the interactions of their protein products is poorly understood. Procedures for genetic manipulation are lacking many economically important animals. The present invention provides an efficient system for performing insertional mutagenesis (gene tagging) and efficient procedures for producing transgenic animals.

100. The transposon-based system of this invention has applications to many areas of biotechnology. Development of transposable elements for vectors in animals permits the following: 1) efficient insertion of genetic material into animal chromosomes using the methods given in this application; 2) identification, isolation, and characterization of genes involved with growth and development through the use of transposons as insertional mutagens (e.g., see Kaiser et al., 1995, "Eukaryotic transposable elements as tools to study gene structure and function." In Mobile Genetic Elements, IRL Press, pp. 69-100); 3) identification, isolation and characterization of transcriptional regulatory sequences controlling growth and development; 4) use of marker constructs for quantitative trait loci (QTL) analysis; and 5) identification of genetic loci of economically important traits, besides those for growth and development, i.e., disease resistance (e.g., Anderson et al., 1996, Mol. Mar. Biol. Biotech., 5,105-113). In one example, the system of this invention can be used to produce sterile transgenic fish. Broodstock with inactivated genes could be mated to produce sterile offspring for either biological containment or for maximizing growth rates in aquacultured fish.

101. In yet another use of the gene transfer system of this invention, the nucleic acid fragment includes a gene to provide a gene therapy to a cell. The gene is placed under the control of a tissue specific promoter or of a ubiquitous promoter or one or more other expression control regions for the expression of a gene in a cell in need of that gene. Therapeutic nucleic acids of interest include genes that replace defective genes in the target host cell, such as those responsible for genetic defect based diseased conditions, genes which have therapeutic utility in the treatment of cancer, and the like. A variety of genes are being tested for a variety of gene therapies including, but not limited to, the cystic fibrosis transmembrane regulator (CFTR) gene, adenosine deaminase (ADA) for immune system disorders, factor IX and interleukin-2 (IL-2) for blood cell diseases, alpha-1-antitrypsin for lung disease, and tumor necrosis factors (TNFs) and multiple drug resistance (MDR) proteins for cancer therapies. Other specific therapeutic genes for use in the treatment of genetic defect based disease conditions include genes encoding the following products: factor VIII, beta.-globin, low-density protein receptor, purine nucleoside phosphorylase, sphingomyelinase, glucocerebrosidase, cystic fibrosis transmembrane regulator, CD-18, ornithine transcarbamylase, arginosuccinate synthetase, phenylalanine hydroxylase, branched-chain alpha.-ketoacid dehydrogenase, fumarylacetoacetate hydrolase, glucose 6-phosphatase, .alpha.-L-fucosidase, .beta.-glucuronidase, .alpha.-L-iduronidase, galactose 1-phosphate uridyltransferase, and the like. Cancer therapeutic genes that may be delivered via the subject vectors include: genes that enhance the antitumor activity of lymphocytes, genes whose expression product enhances the immunogenicity of tumor cells, tumor suppressor genes, toxin genes, suicide genes, multiple-drug resistance genes, antisense sequences, small interfering RNAs and the like. Because of the length of nucleic acid that can be carried by the subject vectors, the subject vectors can be used to not only introduce a therapeutic gene of interest, but also any expression regulatory elements, such as promoters, and the like, which may be desired so as to obtain the desired temporal and spatial expression of the therapeutic gene. These and a variety of human or animal specific gene sequences including gene sequences to encode marker proteins and a variety of recombinant proteins are available in the known gene databases such as GenBank, and the like.

102. The invention can be particularly useful for vaccine delivery. In this aspect of the invention, the antigen or immunogen can be expressed heterologously (e.g., by recombinant insertion of a nucleic acid sequence which encodes the antigen) or as an immunogen (including antigenic or immunogenic fragments) in a viral vector. Alternatively, the antigen or immunogen can be expressed in a live attenuated, pseudotyped virus vaccine, for example. It is also understood that the nucleic acid constructs disclosed herein can be used for vaccine delivery. Generally, the methods can be used to generate humoral and cellular immune responses, e.g. via expression of heterologous pathogen-derived proteins or fragments thereof in specific target cells.

103. A problem overcome by the present invention is non-selective integration as seen in the majority of transposon systems (e.g., Patent Application #20020016975) that creates the potential for insertional mutagenesis of vital genes (e.g., disruption of an anti-oncogene, thus potentially leading to carcinogeneis).

104. The compositions and methods of the present invention are also useful for the introduction of a nucleic acid sequence of interest into a plant cells to produce transgenic plants. As used herein, the term "transgenic plant" refers to the introduction of foreign nucleic acid sequences into the nuclear, mitochondrial or plastid genome of a plant. As used herein, the term "plant" is defined as a unicellular or multicellular organism capable of photosynthesis. This includes the prokaryotic and eukaryotic algae (including cyanophyta and blue-green algae), eukaryotic photosynthetic protists, non-vascular and vascular multicellular photosynthetic organisms, including angiosperms (monocots and dicots), gymnosperms, spore-bearing and vegetatively-reproducing plants. Also included are unicellular and multicellular fungi.

105. Production of a transgenic plant can be accomplished by modifying an isolated transposable element of the type described herein to include the nucleic acid sequence of interest flanked by the termini of the isolated transposable element. The modified transposable element can be introduced into a plant cell in the presence of a transposase protein or a nucleic acid sequence encoding a transposase or a virus encoding a transposase protein (e.g., helper plasmid) using techniques well known in the art. Exemplary techniques are discussed in detail in Gelvin et al., "Plant Molecular Biology Manual", 2nd Ed., Kluwen Academic Publishers, Boston (1995), The transposase (along with DNA directing protein as described herein) catalyzes the transposition of the modified transposable element containing the nucleic acid sequence of interest into the genomic DNA of the plant. The present invention therefore increases the efficiency of integration.

106. For example, for grasses such as maize, the elements of the transposon-based method can be introduced into a cell using, for example, microprojectile bombardment (see e.g Sanford J. C., et al. U.S. Pat. No. 5,100,792 (1992). In this approach, the elements of the transposon-based compositions are coated onto small particles which are then introduced into the targeted tissue (cells) via high velocity ballistic penetration. The transformed cells are then cultivated under conditions appropriate for the regeneration of plants, resulting in production of transgenic plants. Transgenic plants carrying a nucleic acid sequence of interest are examined for the desired phenotype using a variety of methods including, but not limited to, an appropriate phenotypic marker, such as antibiotic resistance or herbicide resistance, or visual observation of the time of floral induction compared to naturally-occurring plants.

107. Further, the gene transfer system of this invention can be used as part of a process for working with or for screening a library of recombinant sequences, for example, to assess the function of the sequences or to screen for protein expression, or to assess the effect of a particular protein or a particular expression control region on a particular cell type. In this example, a library of recombinant sequences, such as the product of a combinatorial library or the product of gene shuffling, both techniques now known in the art, can be incorporated into the nucleic acid fragment of this invention to produce a library of nucleic acid fragments with varying nucleic acid sequences positioned between constant inverted repeat sequences.

108. An advantage of this system is that it is not limited to a significant extent by the size of the intervening nucleic acid sequence positioned between the inverted repeats. For example, the SB protein has been used to incorporate transposons ranging from 1.3 kilobases (kb) to about 5.0 kb and the mariner transposase has mobilized transposons up to about 13 kb. There is no known limit on the size of the nucleic acid sequence that can be incorporated into DNA of a cell using the SB protein.

109. The transposon-based vectors approach has several advantages over the recombination techniques currently in use such as the Cre/LoxP system. For example, the introduction of nucleic acids sequences of interest is performed directly by the Minos transposon. No additional components, such as target sites, are required. In addition, using the present method, a single copy of a nucleic acid sequence of interest can be integrated and precisely excised from the genetic material of a cell in each integration step.

110. This invention has significant advantages over current transposon-based vectors for targeted integration (see for example, U.S. Patent #5,958,775 Inventor: E. Wickstrom and Stephen Cleaver; Wickstrom E, et al. Gene (2000) 254:37-44), which describes the uses and limitations of the attTn7 site or of similar sequence which may or may not be similar enough in certain species. The present invention allows for the potential to increase the efficiency of site-selective integration by inserting host-like sequences as described herein. Furthermore, this invention could be used to bypass Tn7 transposase's normal target site(s) by subsituting its host DNA directing factor with another. Also, this invention allows for the potential to utilize the targeting protein of Tn7 (i.e., TnsD) in a simpler and more efficient system, e.g. making a chimeric Tn5-TnsD transposase by recombinant methods described herein.

111. What has also been limiting the use of transposon-based therapies is the method by which the gene transfer system of this invention is introduced into cells. Viral-mediated strategies have limited the length of the nucleic acid sequence positioned between the inverted repeats, according to this invention. In contrast, for the present non-viral transposon based method microinjection is used and there is very little restraint on the size of the intervening sequence of the nucleic acid fragment of this invention. Similarly, the lipid-mediated strategies described herein for delivering the present nucleic acids do not have substantial size limitations.

112. There are several potential combinations of delivery mechanisms for the transposon portion containing the transgene of interest flanked by the inverted terminal repeats (IRs) and the gene encoding the transposase. For example, both the transposon and the chimeric transposase gene can be contained together on the same recombinant viral genome (or plasmid); a single infection delivers both parts of the present transposon system such that expression of the transposase then directs cleavage of the transposon from the recombinant viral genome for subsequent integration into a cellular chromosome. In another example, the chimeric transposase and the transposon can be delivered separately by a combination of viruses and/or non-viral systems such as lipid-containing reagents. In these cases either the chimeric transposon and/or the transposase gene can be delivered by a recombinant virus. In every case, the expressed transposase gene directs liberation of the transposon from its carrier DNA (viral genome) for site-specific integration into chromosomal DNA.

113. This invention also relates to compositions for use in the gene transfer system of this invention. Thus, the invention relates to the introduction of a nucleic acid fragment comprising a nucleic acid sequence positioned between at least two inverted repeats into a cell. In a preferred embodiment, efficient incorporation of the nucleic acid fragment into the DNA of a cell occurs when the cell also contains a chimeric transposase as described herein. As discussed above, the chimeric transposase can be provided to the cell as a chimeric transposase or as nucleic acid encoding the chimeric transposase. Nucleic acid encoding me chimeric transposase can take the form of RNA or DNA. The protein can be introduced into the cell alone or in a vector, such as a plasmid or a viral vector. Further, the nucleic acid encoding the chimeric transposase protein can be stably or transiently incorporated into the genome of the cell to facilitate temporary or prolonged expression of the chimeric transposase in the cell. Further, promoters or other expression control regions can be operably linked with the nucleic acid encoding the chimeric transposase to regulate expression of the protein in a quantitative or in a tissue-specific manner. Many transposases have a nuclear localizing signal (NLS). The NLS is required for transport into the nucleus after translation in the cytosol in those cells that are nondividing. For example, the SB protein contains a DNA-binding domain, a catalytic domain (having transposase activity) and an NLS signal.

114. The nucleic acid construct of this invention is introduced into one or more cells using any of a variety of techniques known in the art such as, but not limited to, microinjection, combining the nucleic acid fragment with lipid vesicles, such as cationic lipid vesicles, particle bombardment, electroporation, DNA condensing reagents (e.g., calcium phosphate, polylysine or polyethyleneimine) or incorporating the nucleic acid fragment into a viral vector and contacting the viral vector with the cell. Where a viral vector is used, the viral vector can include any of a variety of viral vectors known in the art including viral vectors selected from the group consisting of a retroviral vector, an adenovirus vector or an adeno-associated viral vector.

115. P element derived vectors that include at least the P element transposase recognized insertion sequences of the DrosophilaP element are provided. As such, this invention includes a pair of the 31 base pair inverted repeat domain of the P element, or the functional equivalent thereof, i.e. a domain recognized by the P element encoded chimeric transposase. The 31 base pair inverted repeat is disclosed in Beall et al., "Drosophila P-element transposase is a novel site-specific endonuclease," Genes Dev (Aug 15, 1997)11(16):2137-51. The amino acid sequence of the P element transposase is disclosed in Rio et al., Cell (Jan. 17, 1986) 44: 21-32).

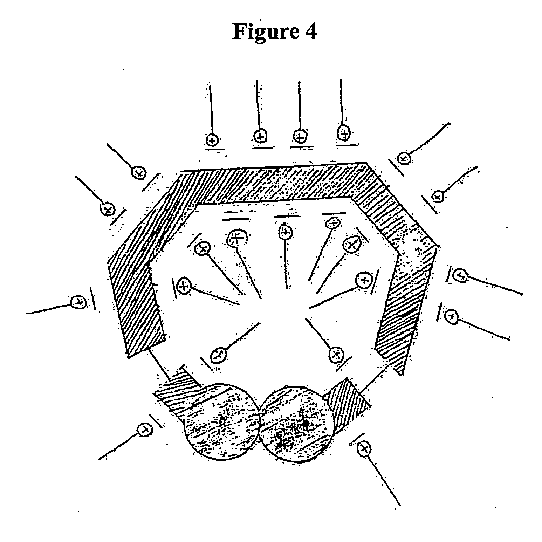

116. Figure 4-6 are schematics of linear nucleic acid constructs in non-viral delivery vehicles. Note: the non-viral nucleic acid construct packaging as outlined can obviously be applied to a plasmid construct. Non-viral packaging systems (e.g., lipid based, polymer based, lipid-polymer-based, and polylysine, among others) are well known to those in the field of non-viral transgenic delivery. Further techniques, to augment the delivery into the nucleus are well known and have been employed in non-viral vectors. Methods of assembling in vitro a transposon-transposase complex have been described in the literature (Lamberg, A, et al. (2002) Efficient insertion mutagenesis strategy for bacterial genomes involving electroporation of in vitro-assembeled DNA transposition complexes of bacteriophage Mu. Applied and Environmental Microbiology).

117. Examples of specific ligands for cellular targeting in the packaging of the invention are well known in the art. The following references are for their teachings on specific ligands: (1) Lestina, B.J., Sagnella, S.M., Xu, Z., Shive, M.S., Richter, N.J., Jayaseharan, J., Case, A.J., Kottke-Marchant, K., Anderson, J.M., and Marchant, R.E. (2002) Surface modification of liposomes for selective cell targeting in cardiovascular drug delivery. J. Control Release 78:235-247. (2) Moreira, J.N., Gaspar, R., and Allen, T.M. (2001) Targeting stealth liposomes in a murine model of human small cell lung cancer. Biochim. Biophys. Acta. 1515:167-176; (3) Xu, L., Tang, W.H., Huang, C.C., Alexander, W., Xiang, L.M., Pirollo, K.F., Rait, A., and Chang, E.H. (2001) Systemic p53 gene therapy of cancer with immunolipoplexes targeted by anti-transferrin receptor scFv. Mol. Med. 7:723-734; (4) Sudhan Shaik, M., Kanikkannan, N., and Singh, M. (2001) Conjugation of anti-My9 antibody to stealth monensin liposomes and the effect of conjugated liposomes on the cytotoxicity of immunotoxin. J. Control Realease 76:285-295; (5) Li, X., Stuckert, P., Bosch, I., Marks, J.D., and Marasco, W.A. (2001) Single-chain antibody-mediated gene delivery into ErbB2-positive human breast cancer cells. Cancer Gene Ther. 8:555-565; (6) Park, J.W., Kirpotin, D.B., Hong, K., Shalaby, R., Shao, Y., Nielsen, U.B., Marks, J.D., Papahadjopoules, D., and Benz, C.C. (2001) Tumor targeting using anti-her2 immunoliposomes. J. Control Release 74:95-113.

118. Examples of endosomal disruption factors that are used in the present vector packaging are well known in the art. The following references are for their teachings on endosomal disruption factors: (1) Farhood, H., Gao, X., Son, K., Yang, Y.Y., Lazo, J.S., Huang, L., Barsoum, J., Bottega, R., and Epand, R.M. (1994) Cationic liposomes for direct gene transfer in therapy of cancer and other diseases. Ann. NY Acad. Sci. 716:23-35; (2) Tachibana R, Harashima H, Shono M, Azumano M, Niwa M, Futaki S, and Kiwada H. (1998) Intracellular regulation of macromolecules using pH-sensitive liposomes and nuclear localization signal: qualitative and quantitative evaluation of intracellular trafficking. Biochem. Biophys. Res. Commun. 251:538-544; (3) E1 Ouahabi A, Thiry M, Pector V, Fuks R, Ruysschaert JM, and Vandenbranden M. (1997) The role of endosome destabilization activity in the gene transfer process mediated by cationic lipids. FEBS Lett 414:187-192.

119. Nuclear localization factors for use in delivering the present nucleic acid constructs known in the art. The following references are for their teachings on nuclear localization factors: (1) Subramanian A, Ranganathan P, and Diamond SL. (1999) Nuclear targeting peptide scaffolds for lipofection of nondividing mammalian cells. Nat Biotechnol 17:873-877; (2) Tachibana R, Harashima H, Shono M, Azumano M, Niwa M, Futaki S, and Kiwada H. (1998) Intracellular regulation of macromolecules using pH-sensitive liposomes and nuclear localization signal: qualitative and quantitative evaluation of intracellular trafficking. Biochem. Biophys. Res. Commun. 251:538-544. (3) Aronsohn AI and Hughes JA. (1998) Nuclear localization signal peptides enhance cationic liposome-mediated gene transfer. J Drug Target 5:163-169; (4) Boehm U, Heinlein M, Behrens U, and Kunze R. (1995) One of three nuclear localization signals of maize Activator (Ac) transposase overlaps the DNA-binding domain. Plant J 7:441-451.

120. Also disclosed are compositions of the invention, wherein the nucleic acid sequence encoding the chimeric integrating enzyme is located outside the terminal repeats.

121. Also disclosed are compositions of the invention, wherein the nucleic acid encoding the transgene and the nucleic acid encoding the chimeric integrating enzyme are the same nucleic acid.

122. Also disclosed are compositions, wherein the transgene and the integrating enzyme are encoded on a separate nucleic acids.

123. Also disclosed are compositions, further comprising a homologous sequence that is homologous to the host DNA.

124. Also disclosed are compositions, wherein the homologous sequence is located outside the terminal repeats.

125. Also disclosed are compositions, further comprising a protein binding sequence and a separate nucleic acid encoding two DNA binding domains.

126. Also disclosed are compositions, further comprising a protein binding sequence and a separate nucleic acid encoding a DNA binding domain and a protein-binding domain.

127. Also disclosed are compositions of the invention, wherein the nucleic acid present in the non-viral vector is at least one functional protein.

128. Also disclosed are compositions of the invention, wherein the transgene encodes a biologically active molecule. The transgene can encode multiple and different biologically active molecules. The transgene can be selected at least from the group consisting of reporter genes (e.g., luciferase, chloramphenicol-acetyl transferase, GFP), oncogenes (e.g., ras and c-myc), and antioncogenes (e.g. p53 and retinoblastoma). A variety of other genes are being tested for gene therapy including CFTR for cystic fibrosis, adenosine deaminase (ADA) for immune disorders, factor IX, factor VIII and interleukin-2 (IL-2) for blood cell diseases, alpha-1-antitrypsin for lung disease, and tumor necrosis factor, endostatin, sodium/iodide symporter, angiostatin, and multiple drug resistance (MDR) for cancer therapies. Other examples of genes include, e.g., bax, bak, E2F-1, BRCA-1, BRCA-2, bak, ras, p21, CDKN2A, pHyde, FAS-ligand, TNF-related apoptosis inducing ligand, DOC-2, E-cadherin, caspases, clusterin, ATM, granulocyte macrophage colony stimulating factor, B7, tumor necrosis factor-alpha, interleuken 12, interleuken 15, interferon-gamma, interferon-beta, MUC-1, PSA, WT1, WT2, myc, MDM2, DCC, VEGFB, VEGFC, VWF, NEFL, NEF3, TUBB, MAPT, SGNE1, RTN1, GAD1, PYGM, AMPD1, TNNT3, TNNT2, ACTC, MYH7, SFTPB, TPO, NGF, connexin 43.

129. Compounds disclosed herein may also be used for the treatment of precancer conditions such as cervical and anal dysplasias, other dysplasias, severe dysplasias, hyperplasias, atypical hyperplasias, and neoplasias.

130. Also disclosed are nucleic acid constructs of the invention, wherein the transgene is an antigen from a virus. The viral antigen can be selected from the group consisting of Herpes simplex virus type-1, Herpes simplex virus type-2, Cytomegalovirus, Epstein-Barr virus, Varicella-zoster virus, Human herpesvirus 6, Human herpesvirus 7, Human herpesvirus 8, Variola virus, Vesicular stomatitis virus, Hepatitis A virus, Hepatitis B virus, Hepatitis C virus, Hepatitis D virus, Hepatitis E virus, Rhinovirus, Coronavirus, Influenza virus A, Influenza virus B, Measles virus, Polyomavirus, Human Papillomavirus, Respiratory syncytial virus, Adenovirus, Coxsackie virus, Dengue virus, Mumps virus, Poliovirus, Rabies virus, Rous sarcoma virus, Yellow fever virus, Ebola virus, Marburg virus, Lassa fever virus, Eastern Equine Encephalitis virus, Japanese Encephalitis virus, St. Louis Encephalitis virus, Murray Valley fever virus, West Nile virus, Rift Valley fever virus, Rotavirus A, Rotavirus B, Rotavirus C, Sindbis virus, Simian Immunodeficiency cirus, Human T-cell Leukemia virus type-1, Hantavirus, Rubella virus, Simian Immunodeficiency virus, Human Immunodeficiency virus type-1, and Human Immunodeficiency virus type-2.

131. Also disclosed are nucleic acid constructs of the invention, wherein the transgene is an antigen from a bacterium. The bacterial antigen can be selected from the group consisting of *M*. *tuberculosis, M. bovis, M. bovis* strain BCG, BCG substrains, *M. avium, M. intracellulare, M. africanum. M. kansasii, M. marinum, M. ulcerans, M. avium* subspecies *paratuberculosis. Nocardia asteroides,* other *Nocardia* species, *Legionella pneumophila,* other *Legionella* species, *Salmonella typhi,* other *Salmonella* species. *Shigella* species, *Yersinia pestis, Pasteurella haemolytica, Pasteurella multocida,* other *Pasteurella species, Actinobacillus pleuropneumoniae, Listeria monocytogenes, Listeria ivanovii, Brucella abortus,* other *Brucella* species, *Cowdria ruminantium, Chlamydia pneumoniae, Chlamydia trachomatis, Chlamydia psittaci, Coxiella burnetti,* other *Rickettsial* species, *Ehrlichia* species, *Staphylococcus aureus, Staphylococcus epidermidis, Streptococcus pyogenes, Streptococcus agalactiae, Bacillus anthracis, Escherichia coli, Vibrio cholerae, Campylobacter* species, *Neiserria meningitidis, Neiserria gonorrhea, Pseudomonas aeruginosa,* other *Pseudomonas* species, *Haemophilus influenzae, Haemophilus ducreyi,* other *Hemophilus* species, *Clostridium tetani,* other *Clostridium species, Yersinia enterolitica,* and other *Yersinia species.*

132. Also disclosed are nucleic acid constructs of the invention, wherein the transgene is antigen from a parasite. The parasitic antigen can be selected from the group consisting of *Toxoplasma gondii, Plasmodium falciparum, Plasmodium vivax, Plasmodium malariae,* other *Plasmodium* species., *Trypanosoma brucei, Trypanosoma cruzi, Leishmania major,* other *Leishmania* species., *Schistosoma mansoni,* other *Schistosoma* species., and *Entamoeba histolytica.*

133. Also disclosed are nucleic acid constructs of the invention, wherein the transgene is a tumor antigen. The tumor antigen can be selected from the list consisting of human epithelial cell mucin (Muc-1; a 20 amino acid core repeat for Muc-1 glycoprotein, present on breast cancer cells and pancreatic cancer cells), the Ha-ras oncogene product, p53, carcino-embryonic antigen (CEA), the raf oncogene product, gp100/pmel17, GD2, GD3, GM2, TF, sTn, MAGE-1, MAGE-3, BAGE, GAGE, tyrosinase, gp75, Melan-A/Mart-1, gp100, HER2/neu, EBV-LMP 1 & 2, HPV-F4, 6, 7, prostate-specific antigen (PSA), HPV-16, MUM, alpha-fetoprotein (AFP), CO17 1A, GA733, gp72, p53, the ras oncogene product, HPV E7, Wilm's tumor antigua-1, telomerase, and melanoma gangliosides.

134. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular chimeric transposase is disclosed and discussed and a number of modifications that can be made to a number of molecules including the chimeric transposase are discussed, specifically contemplated is each and every combination and permutation of chimeric transposase and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

135. Also disclosed are methods of docking the transposon-based vector adjacent to the host DNA, utilizing repetitive sequences for homologous recombination to promote efficient site-selective integration, as well as other site-selective non-viral approaches.

136. Also disclosed are methods that employ recognition site(s) on the plasmid that can recognize an endogenous protein (or a newly introduced protein, e.g. produced from a gene located on the plasmid) that can then direct the complex into the vicinity of the host-DNA for site-selective integration.

137. Also disclosed are methods of incorporating repetitive elements (e.g., Alu-like sequences) in the transposon-based plasmid. It is understood that such methods can enhance docking and at the same time allow for either homologous recombination (66-67) or integration of the transgene into the host genome.

138. Incorporating repetitive elements (e.g., Alu-like sequences) in the transposon-based plasmid can enhance docking and at the same time allow for either homologous recombination or integration of the transgene into the host genome.

139. Also disclosed are methods that employ recognition sites on the plasmid that can recognize an endogenous protein (or a newly introduced protein) that can then direct the complex to the vicinity of the host-DNA.

### 1. Delivery of the vector compositions to cells

140. There are a number of compositions and methods which can be used to deliver nucleic acids to cells, either in vitro or in vivo. For example, the nucleic acids can be delivered through a number of direct delivery systems such as, electroporation, lipofection, calcium phosphate precipitation, plasmids, cosmids, or via transfer of genetic material in cells or carriers such as cationic liposomes. Appropriate means for transfection, including chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991). Such methods are well known in the art and readily adaptable for use with the compositions and methods described herein. In certain cases, the methods will be modifed to specifically function with large DNA molecules. Further, these methods can be used to target certain diseases and cell populations by using the targeting characteristics of the carrier.

141. The disclosed compositions can be delivered to the target cells in a variety of ways. For example, the compositions can be delivered through electroporation, or through lipofection, or through calcium phosphate precipitation. The delivery mechanism chosen will depend in part on the type of cell targeted and whether the delivery is occurring for example in vivo or in vitro.

142. Thus, the compositions can comprise, in addition to the disclosed non-viral nucleic acid construct vectors for example, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a compound and a cationic liposome can be administered to the blood afferent to a target organ or inhaled into the respiratory tract to target cells of the respiratory tract. Regarding liposomes, see, e.g., Brigham et al. Am. J. Resp. Cell. Mol. Biol. 1:95-100 (1989); Felgner et al. Proc. Natl. Acad. Sci USA 84:7413-7417 (1987); U.S. Pat. No.4,897,355. Furthermore, the compound can be administered as a component of a microcapsule that can be targeted to specific cell types, such as macrophages, or where the diffusion of the compound or delivery of the compound from the microcapsule is designed for a specific rate or dosage.

143. In the methods described above which include the administration and uptake of exogenous DNA into the cells of a subj ect (i.e., gene transduction or transfection), delivery of the compositions to cells can be via a variety of mechanisms. As one example, delivery can be via a liposome, using commercially available liposome preparations such as LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art. In addition, the nucleic acid or vector of this invention can be delivered *in vivo* by electroporation, the technology for which is available from Genetronics, Inc. (San Diego, CA) as well as by means of a SONOPORATION machine (ImaRx Pharmaceutical Corp., Tucson, AZ).

144. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). These techniques can be used for a variety of other speciifc cell types. Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

145. Nucleic acids that are delivered to cells which are to be integrated into the host cell genome, typically contain integration sequences. These sequences are often viral related sequences, particularly when viral based systems are used. These viral intergration systems can also be incorporated into nucleic acids which are to be delivered using a non-nucleic acid based system of deliver, such as a liposome, so that the nucleic acid contained in the delivery system can be come integrated into the host genome.

146. Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

147. The 3 requirements for efficient cell-selective delivery of a vector into the nucleus of a cell are a ligand (or receptor) for selective cell targeting, an endosomal disruption factor if the vector is taken up via receptor mediated endocytosis, and a nuclear localizing signal. These have been employed in gene therapy and the methods of construction and implementation are well known in the literature.

148. Surface modifications to liposomes for selective cell targeting have been described in detail and employed with success (Lestini, B.J., et al (2002) Surface modification of liposomes for selective cell targeting in cardiovascular drug delivery. J. Control Release 78:235-247; Moreira, J.N., et al. (2001) Targeting stealth liposomes in a murine model of human small cell lung cancer. Biochim. Biophys. Acta. 1515:167-176.; Xu, L., et al. (2001) Systemic p53 gene therapy of cancer with immunolipoplexes targeted by anti-transferrin receptor scFv. Mol. Med. 7:723-734. Sudhan Shaik, M., et al. (2001) Conjugation of anti-My9 antibody to stealth monensin liposomes and the effect of conjugated liposomes on the cytotoxicity of immunotoxin. J. Control Realease 76:285-295.; Li, X., et al. (2001) Single-chain antibody-mediated gene delivery into ErbB2-positive human breast cancer cells. Cancer Gene Ther. 8:555-565.; Park, J.W., et al. (2001) Tumor targeting using anti-her2 immunoliposomes. J. Control Release 74:95-113). For example, a cationic immunolipolex incorporating a biosynthetically lipid-tagged, anti-transferrrin receptor could be utilized as described by Xu and colleagues.

149. Endosomal disruption factors have been employed in cationic lipids and are well known to those who are skilled in the art (Tachibana R, et al. (1998) Intracellular regulation of macromolecules using pH-sensitive liposomes and nuclear localization signal: qualitative and quantitative evaluation of intracellular trafficking. Biochem. Biophys. Res. Commun. 251:538-544.; E1 Ouahabi A, et al. (1997) The role of endosome destabilization activity in the gene transfer process mediated by cationic lipids. FEBS Lett 414:187-192). For example, Tachibana and colleagues utilized pH-sensitive liposomes in order to achieve endosomal disruption and subsequent release into the cytosol.

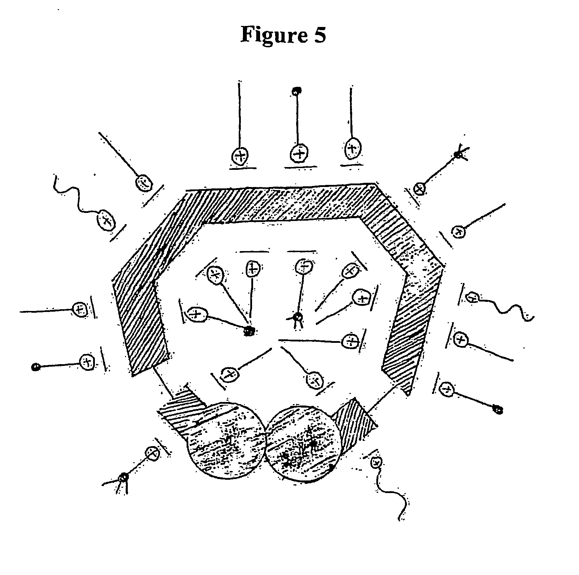

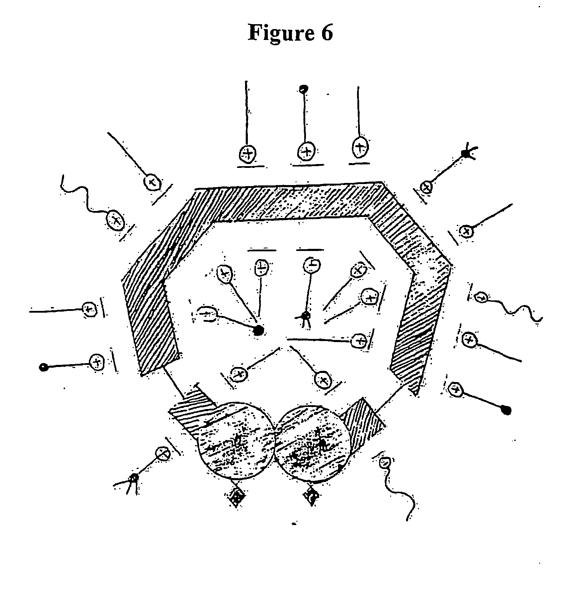

150. Nuclear localization factors can also be incorporated as diagrammed in the schematic (Figure 5 and 6) (Subramanian A, et al. (1999) Nuclear targeting peptide scaffolds for lipofection of nondividing mammalian cells. Nat Biotechnol 17:873-877.; Aronsohn AI, et al. (1998) Nuclear localization signal peptides enhance cationic liposome-mediated gene transfer. J Drug Target 5:163-169.; Boehm U, et al. (1995) One of three nuclear localization signals of maize Activator (Ac) transposase overlaps the DNA-binding domain. Plant J 7:441-451.) For example, Aronsohn and colleagues constructed a non-viral delivery vehicle consisting of a conglomerate of a synthetic nuclear localizing peptide derived from the SV40 virus, a luciferase encoding PGL3 plasmid, and a cationic lipid DOTAP:DOPE liposome.

### 2. Expression systems

151. The nucleic acids that are delivered to cells typically contain expression controlling systems. For example, the inserted genes in non-viral and viral systems usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### a) Promoters and Enhancers

152. Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature. 273: 113 (1978)). The immediate early promoter of the human cytomegalovirus is conveniently obtained as a HindIII E restriction fragment (Greenway, P.J. et al., Gene 18: 355-360 (1982)). Of course, promoters from the host cell or related species also are useful herein.

153. Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers f unction to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, -fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

154. The promotor and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

155. In certain embodiments the promoter and/or enhancer region can act as a constitutive promoter and/or enhancer to maximize expression of the region of the transcription unit to be transcribed. In certain constructs the promoter and/or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTF.

156. It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

157. Suitable promoters for use in plants are also well known in the art. For example, constitutive promoters for plant gene expression include the octopine synthase, nopaline synthase, or mannopine synthase promoters from Agrobacterium, the cauliflower mosaic virus (35S) promoter, the figwort mosaic virus (FMV) promoter, and the tobacco mosaic virus (TMV) promoter. Specific examples of regulated promoters in plants include the low temperature Kin1 and cor6.6 promoters (Wang, et al., Plant Mol. Biol. 28:605 (1995); Wang, et al., Plant Mol. Biol. 28:619-634 (1995)), the ABA inducible promoter (Marcotte et al., Plant Cell 1:969-976 (1989)), heat shock promoters, and the cold inducible promoter from B. napus (White et al., Plant Physiol. 106:917 (1994)).

158. Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases. It is also preferred that the transcribed units contain other standard sequences alone or in combination with the above sequences improve expression from, or stability of, the construct.

### b) Markers

159. The vector can include nucleic acid sequence encoding a marker product. The term "marker gene", as used herein, refers to a nucleic acid sequence whose product can be easily assayed, for example, colorimetrically as an enzymatic reaction product, such as the lacZ gene which encodes for .beta.-galactosidase. The marker gene can be operably linked to a suitable promoter which is optionally linked to a nucleic acid sequence of interest so that expression of the marker gene can be used to assay integration of the transposon into the genome of a cell and thereby integration of the nucleic acid sequence of interest into the genome of the cell. Examples of widely-used marker molecules include enzymes such as beta-galactosidase, beta-glucoronidase, beta-glucosidase; luminescent molecules such as green flourescent protein and firefly luciferase; and auxotrophic markers such as His3p and Ura3p. (See, e.g., Chapter 9 in Ausubel, F. M., et al. Current Protocols in Molecular Biology, John Wiley & Sons, Inc., (1998)).

160. In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR- cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

161. The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1: 327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### C. Methods of using the compositions

162. The transposon system of this invention has applications to many areas of biotechnology. Development of transposable elements for vectors in animals permits the following: 1) efficient insertion of genetic material into animal chromosomes using the methods given in this application; 2) identification, isolation, and characterization of genes involved with growth and development through the use of transposons as insertional mutagens (e.g., see Kaiser et al., 1995, "Eukaryotic transposable elements as tools to study gene structure and function." In Mobile Genetic Elements, IRL Press, pp. 69-100); 3) identification, isolation and characterization of transcriptional regulatory sequences controlling growth and development; 4) use of marker constructs for quantitative trait loci (QTL) analysis; and 5) identification of genetic loci of economically important traits, besides those for growth and development, i.e., disease resistance (e.g., Anderson et al., 1996, Mol. Mar. Biol. Biotech., 5, 105-113).

### 1. Methods of gene modification and gene disruption

163. Due to their inherent ability to move from one chromosomal location to another within and between genomes, transposable elements have been exploited as genetic vectors for genetic manipulations in several organisms. Transposon tagging is a technique in which transposons are mobilized to "hop" into genes, thereby inactivating them by insertional mutagenesis. These methods are discussed by Evans et al., TIG 1997 13,370-374. In the process, the inactivated genes are "tagged" by the transposable element which then can be used to recover the mutated allele. The ability of the human and other genome projects to acquire gene sequence data has outpaced the ability of scientists to ascribe biological function to the new genes. Therefore, the present invention provides an efficient method for introducing a tag into the genome of a cell. Where the tag is inserted into a location in the cell that disrupts expression of a protein that is associated with a particular phenotype, expression of an altered phenotype in a cell containing the nucleic acid of this invention permits the association of a particular phenotype with a particular gene that has been disrupted by the nucleic acid fragment of this invention. Here the nucleic acid fragment functions as a tag. Primers designed to sequence the genomic DNA flanking the nucleic acid fragment of this invention can be used to obtain sequence information about the disrupted genre.

164. The nucleic acid construct can also be used for gene discovery. In one example, the nucleic acid fragment in combination with the chimeric transposase or nucleic acid encoding the chimeric transposase is introduced into a cell. The nucleic acid construct preferably comprises a nucleic acid sequence positioned between at least two inverted repeats, wherein the inverted repeats bind to the Chimeric transposase protein and wherein the nucleic acid fragment integrates into the DNA of the cell in the presence of the chimeric transposase protein. In a preferred embodiment, the nucleic acid sequence includes a marker protein, such as GFP and a restriction endonuclease recognition site, preferably a 6-base recognition sequence. Following integration, the cell DNA is isolated and digested with the restriction endonculease. Where a restriction endonuclease is used that employs a 6-base recognition sequence, the cell DNA is cut into about 4000- bp fragments on average. These fragments can be either cloned or linkers can be added to the ends of the digested fragments to provide complementary sequence for PCR primers. Where linkers are added, PCR reactions are used to amplify fragments using primers from the linkers and primers binding to the direct repeats of the inverted repeats in the nucleic acid fragment. The amplified fragments are then sequenced and the DNA flanking the direct repeats is used to search computer databases such as (GenBank.

165. The invention can be used for site-directed tagging. For example, by incorporating a similiar host gene sequence (but non-functional) in a transposon based plasmid allows for tagging of that gene as described above. One application of the invention is to determine the function of a specific protein. For example, cDNA (reverse transcribed mRNA), genomic DNA, or RNA/DNA hybrids (chimeraplast) can be inserted in a transposon-based palsmid after site-directed mutagenesis so that the coding region can be inactivated This altered cDNA or genomic DNA can be inserted into a tranposon-based plasmid as described herein. The transposon-based vector containing host-like sequence docks to the host DNA through hybridization. Expression of the transposase and subsequent integration occurs at the desired target. Another embodiment of the invention is making a chimeric transposase without site-selectivity for the purposes described above. For example, if a given transposase in a certain cell does not have the DNA directing factor for that cell then the efficiency of integration is markedly reduced. By providing the transposase with a required DNA directing factor then the integration is significantly enhanced which results in an obvious improvement over the "conventional" transposase.

166. Also disclosed is a method for mobilizing a nucleic acid sequence in a cell. In this method the nucleic acid construct of this invention is incorporated into DNA in a cell, as provided in the discussion above. Additional chimeric transposase or nucleic acid encoding the chimeric transposase is introduced into the cell and the protein is able to mobilize (i.e. move) the nucleic acid fragment from a first position within the DNA of the cell to a second position within the DNA of the cell. The DNA of the cell can be genomic DNA or extrachromosomal DNA. The method permits the movement of the nucleic acid fragment from one location in the genome to another location in the genome, or for example, from a plasmid in a cell to the genome of that cell.

167. The disclosed compositions and methods can be used for targeted gene disruption and modification in any animal that can undergo these events. Gene modification and gene disruption refer to the methods, technique, and compositions that surround the selective removal or alteration of a gene or stretch of chromosome in an animal, such as a mammal, in a way that propagates the modification through the germ line of the mammal. In general, a cell is transformed with a vector which is designed to homologously recombine with a region of a particular chromosome contained within the cell, as for example, described herein. This homologous recombination event can produce a chromosome which has exogenous DNA introduced, for example in frame, with the surrounding DNA. This type of protocol allows for very specific mutations, such as point mutations, to be introduced into the genome contained within the cell. Methods for performing this type of homologous recombination are disclosed herein.

168. One of the preferred characteristics of performing homologous recombination in mammalian cells is that the cells should be able to be cultured, because the desired recombination events occur at a low frequency.

169. Once the cell is produced through the methods described herein, an animal can be produced from this cell through either stem cell technology or cloning technology. For example, if the cell into which the nucleic acid was transfected was a stem cell for the organism, then this cell, after transfection and culturing, can be used to produce an organism which will contain the gene modification or disruption in germ line cells, which can then in turn be used to produce another animal that possesses the gene modification or disruption in all of its cells. In other methods for production of an animal containing the gene modification or disruption in all of its cells, cloning technologies can be used. These technologies generally take the nucleus of the transfected cell and either through fusion or replacement fuse the transfected nucleus with an oocyte which can then be manipulated to produce an animal. The advantage of procedures that use cloning instead of ES technology is that cells other than ES cells can be transfected. For example, a fibroblast cell, which is very easy to culture can be used as the cell which is transfected and has a gene modification or disruption event take place, and then cells derived from this cell can be used to clone a whole animal.

170. To modify a gene of interest nucleic acids can be cloned into a vector designed for example, for homologous recombination. This gene could be, for example, a heterologous or synthetic regulatory sequence of an antioncogene (e.g. p53 and retinoblastoma). A variety of other genes are being tested for gene therapy including CFTR for cystic fibrosis, adenosine deaminase (ADA) for immune disorders, factor IX, factor VIII and interleukin-2 (IL-2) for blood cell diseases, alpha-1-antitrypsin for lung disease, and tumor necrosis factor, endostatin, sodium/iodide symporter, angiostatin, and multiple drug resistance (MDR) for cancer therapies. Other examples gene include e.g., bax, bak, E2F-1, BRCA-1, BRCA-2, bak, ras, p21, CDKN2A, pHyde, FAS-ligand, TNF-related apoptosis inducing ligand, DOC-2, E-cadherin, caspases, clusterin, ATM, granulocyte macrophage colony stimulating factor, B7, tumor necrosis factor-alpha, interleuken 12, interleuken 15, interferon-gamma, interferon-beta, MUC-1, PSA, WT1, WT2, myc, MDM2, DCC, VEGFB, VEGFC, VWF, NEFL, NEF3, TUBB, MAPT, SGNE1, RTN1, GAD1, PYGM, AMPD1, TNNT3, TNNT2, ACTC, MYH7, SFTPB, TPO, NGF, connexin 43.

### 2. Methods of performing gene delivery

171. Gene delivery is performed in vitro (e.g., electroporation or other techniques well known in the art) or in vivo. In vivo techniques include intravenous administration, direct injection into the desired site, or by inhalation.

### 3. Methods of treating disease

172. Disclosed are methods of treating a subject with a condition comprising administering to the vector of the invention.

173. The disclosed compositions can be used to treat any disease where uncontrolled cellular proliferation occurs such as cancers. A non-limiting list of different types of cancers is as follows: lymphomas (Hodgkins and non-Hodgkins), leukemias, carcinomas, carcinomas of solid tissues, squamous cell carcinomas, adenocarcinomas, sarcomas, gliomas, high grade gliomas, blastomas, neuroblastomas, plasmacytomas, histiocytomas, melanomas, adenomas, hypoxic tumours, myelomas, AIDS-related lymphomas or sarcomas, metastatic cancers, or cancers in general.

174. A representative but non-limiting list of cancers that the disclosed compositions can be used to treat is the following: lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, kidney cancer, lung cancers such as small cell lung cancer and non-small cell lung cancer, neuroblastoma/glioblastoma, ovarian cancer, pancreatic cancer, prostate cancer, skin cancer, liver cancer, melanoma, squamous cell carcinomas of the mouth, throat, larynx, and lung, colon cancer, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers; testicular cancer; colon and rectal cancers, prostatic cancer, or pancreatic cancer.

175. Also disclosed are methods of the invention, wherein the condition is a viral infection. The viral infection can be selected from the list of viruses consisting of Herpes simplex virus type-1, Herpes simplex virus type-2, Cytomegalovirus, Epstein-Barr virus, Varicella-zoster virus, Human herpesvirus 6, Human herpesvirus 7, Human herpesvirus 8, Variola virus, Vesicular stomatitis virus, Hepatitis A virus, Hepatitis B virus, Hepatitis C virus, Hepatitis D virus, Hepatitis E virus, Rhinovirus, Coronavirus, Influenza virus A, Influenza virus B, Measles virus, Polyomavirus, Human Papilomavirus, Respiratory syncytial virus, Adenovirus, Coxsackie virus, Dengue virus, Mumps virus, Poliovirus, Rabies virus, Rous sarcoma virus, Yellow fever virus, Ebola virus, Marburg virus, Lassa fever virus, Eastern Equine Encephalitis virus, Japanese Encephalitis virus, St. Louis Encephalitis virus, Murray Valley fever virus, West Nile virus, Rift Valley fever virus, Rotavirus A, Rotavirus B, Rotavirus C, Sindbis virus, Simian Immunodeficiency cirus, Human T-cell Leukemia virus type-1, Hantavirus, Rubella virus, Simian Immunodeficiency virus, Human Immunodeficiency virus type-1, and Human Immunodeficiency virus type-2.

176. Also disclosed are methods of the invention, wherein the transgene is an antigen from a virus. The viral antigen can be selected from the group of viruses consisting of Herpes simplex virus type-1, Herpes simplex virus type-2, Cytomegalovirus, Epstein-Barr virus, Varicella-zoster virus, Human herpesvirus 6, Human herpesvirus 7, Human herpesvirus 8, Variola virus, Vesicular stomatitis virus, Hepatitis A virus, Hepatitis B virus, Hepatitis C virus, Hepatitis D virus, Hepatitis E virus, Rhinovirus, Coronavirus, Influenza virus A, Influenza virus B, Measles virus, Polyomavirus, Human Papilomavirus, Respiratory syncytial virus, Adenovirus, Coxsackie virus, Dengue virus, Mumps virus, Poliovirus, Rabies virus, Rous sarcoma virus, Yellow fever virus, Ebola virus, Marburg virus, Lassa fever virus, Eastern Equine Encephalitis virus, Japanese Encephalitis virus, St. Louis Encephalitis virus, Murray Valley fever virus, West Nile virus, Rift Valley fever virus, Rotavirus A, Rotavirus B, Rotavirus C, Sindbis virus, Simian Immunodeficiency cirus, Human T-cell Leukemia virus type-1, Hantavirus, Rubella virus, Simian Immunodeficiency virus, Human Immunodeficiency virus type-1, and Human Immunodeficiency virus type-2.

177. Also disclosed are methods of the invention, wherein the condition is a bacterial infection. The bacterial infection can be selected from the list of bacterium consisting of *M. tuberculosis, M. bovis, M. bovis* strain BCG, BCG substrains, *M. avium, M intracellulare, M. africanum, M kansasii, M marinum, M. ulcerans, M avium* subspecies *paratuberculosis, Nocardia asteroides,* other *Nocardia* species, *Legionella pneumophila,* other *Legionella* species, *Salmonella typhi,* other *Salmonella* species, *Shigella* species, *Yersinia pestis, Pasteurella haemolytica, Pasteurella multocida,* other *Pasteurella species, Actinobacillus pleuropneumoniae, Listeria monocytogenes, Listeria ivanovii, Brucella abortus,* other *Brucella* species, *Cowdria ruminantium, Chlamydia pneumoniae, Chlamydia trachomatis, Chlamydia psittaci, Coxiella burnetti,* other *Rickettsial* species, *Ehrlichia* species, *Staphylococcus aureus, Staphylococcus epidermidis, Streptococcus pyogenes, Streptococcus agalactiae, Bacillus anthracis, Escherichia coli, Vibrio cholerae, Campylobacter* species, *Neiserria meningitidis, Neiserria gonorrhea, Pseudomonas aeruginosa,* other *Pseudomonas* species, *Haemophilus influenzae, Haemophilus ducreyi,* other *Hemophilus species, Clostridium tetani,* other *Clostridium* species, *Yersinia enterolitica, and* other *Yersinia species.*

178. Also disclosed are methods of the invention, wherein the transgene is an antigen from a bacterium. The bacterial antigen can be selected from the group consisting of *M*. *tuberculosis, M. bovis, M. bovis* strain BCG, BCG substrains, *M*. *avium, M. intracellulare, M. africanum, M. kansasii, M marinum, M. ulcerans, M. avium* subspecies *paratuberculosis, Nocardia asteroides,* other *Nocardia* species, *Legionella pneumophila,* other *Legionella* species, *Salmonella typhi,* other *Salmonella* species, *Shigella* species, *Yersinia pestis, Pasteurella haemolytica, Pasteurella multocida, other Pasteurella species, Actinobacillus pleuropneumoniae, Listeria monocytogenes, Listeria ivanovii, Brucella abortus, other Brucella* species, *Cowdria ruminantium, Chlamydia pneumoniae, Chlamydia trachomatis, Chlamydia psittaci, Coxiella burnetti,* other *Rickettsial* species, *Ehrlichia* species, *Staphylococcus aureus, Staphylococcus epidermidis, Streptococcus pyogenes, Streptococcus agalactiae, Bacillus anthracis, Escherichia coli, Vibrio cholerae, Campylobacter* species, *Neiserria meningitidis, Neiserria gonorrhea, Pseudomonas aeruginosa,* other *Pseudomonas* species, *Haemophilus influenzae, Haemophilus ducreyi,* other *Hemophilus* species, *Clostridium tetani,* other *Clostridium species,Yersinia enterolitica,* and other *Yersinia species.*

179. Also disclosed are methods of the invention, wherein the condition is a parasitic infection. The parasitic infection can be selected from the list of parasites consisting of *Toxoplasma gondii, Plasmodium falciparum, Plasmodium vivax, Plasmodium malariae,* other *Plasmodium* species., *Trypanosoma brucei, Trypanosoma cruzi, Leishmania major,* other *Leishmania* species., *Schistosoma mansoni,* other *Schistosoma* species., and *Entamoeba histolytica.*

180. Also disclosed are methods of the invention, wherein the transgene is an antigen from a parasite. The parasitic antigen can be selected from the group consisting of *Tosoplasma gondii, Plasmodium falciparum, Plasmodium vivax*, *Plasmodium malariae,* other *Plasmodium* species., *Trypanosoma brucei, Trypanosoma cruzi, Leishmania major,* other *Leishmania* species., *Schistosoma mansoni,* other *Schistosoma* species., and *Entamoeba histolytica.*

181. Also disclosed are methods of the invention, wherein the condition is cancer.

182. The disclosed vectors and vector containing compositions can be used to treat any disease where uncontrolled cellular proliferation occurs such as cancers. A non-limiting list of different types of cancers is as follows: lymphomas (Hodgkins and non-Hodgkins), leukemias, carcinomas, carcinomas of solid tissues, squamous cell carcinomas, adenocarcinomas, sarcomas, gliomas, high grade gliomas, blastomas, neuroblastomas, plasmacytomas, histiocytomas, melanomas, adenomas, hypoxic tumours, myelomas, AIDS-related lymphomas or sarcomas, metastatic cancers, or cancers in general.

183. A representative but non-limiting list of cancers that the disclosed compositions can be used to treat is the following: lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, kidney cancer, lung cancers such as small cell lung cancer and non-small cell lung cancer, neuroblastoma/glioblastoma, ovarian cancer, pancreatic cancer, prostate cancer, skin cancer, liver cancer, melanoma, squamous cell carcinomas of the mouth, throat, larynx, and lung, colon cancer, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers; testicular cancer; colon and rectal cancers, prostatic cancer, or pancreatic cancer.

184. Also disclosed are methods of the invention, wherein the transgene is a tumor antigen. The tumor antigen can be selected from the list consisting of human epithelial cell mucin (Muc-1; a 20 amino acid core repeat for Muc-1 glycoprotein, present on breast cancer cells and pancreatic cancer cells), the Ha-ras oncogene product, p53, carcino-embryonic antigen (CEA), the raf oncogene product, gp100/pmel17, GD2, GD3, GM2, TF, sTn, MAGE-1, MAGE-3, BAGE, GAGE, tyrosinase, gp75, Melan-A/Mart-1, gyp100, HER2/neu, EBV-LMP 1 & 2, HPV-F4, 6, 7, prostate-specific antigen (PSA), HPV-16, MUM, alpha-fetoprotein (AFP), C017-1A, GA733, gp72, p53, the ras oncogene product, HPV E7, Wilm's tumor antigen-1, telomerase, and melanoma gangliosides.

185. Disclosed are methods of treating a condition in a subject comprising administering to the subject the vector of the invention, wherein the condition is due to a mutated, disregulated, disrupted, or deleted gene; autoimmunity; or inflammatory diseases.

186. Disclosed are methods of treating a condition in a subject, wherein the condition can be selected from list consisting of cystic fibrosis, asthma, multiple sclerosis, muscular dystrophy, diabetes, tay-sachs, spinobifida, sickle cell anemia, hereditary hemochromatosis, cerebral palsy, parkinson's disease, lou gehrigg disease, alzheimer's, systemic lupus erythamatosis, hemophelia, Addsion's disease, Huntington's disease, and Cushing's disease.

187. Disclsosed are methods of treating a condition, wherein the transgene is comprises a functioning gene to replace a mutated gene associated with a genetic disorder. Also disclosed are methods of treating a condition, wherein the transgene can be selected from the list of genes consisting of cystic fibrosis transmembrane conductance regulator, HFE, and HBB.

### 4. Pharmaceutical carriers/. Delivery of pharamceutical products

188. As described above, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

189. The compositions may be administered orally, parenterally (e.g., intravenously), by intramuscular injection, by intraperitoneal injection, transdermally, extracorporeally, topically or the like, although topical intranasal administration or administration by inhalant is typically preferred. As used herein, "topical intranasal administration" means delivery of the compositions into the nose and nasal passages through one or both of the nares and can comprise delivery by a spraying mechanism or droplet mechanism, or through aerosolization of the nucleic acid or vector. The latter may be effective when a large number of animals is to be treated simultaneously. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

190. Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795.

191. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews 129:57-80, (1992); and Roffler, et al., Biochem. Pharmacol. 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo*. The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient, uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology, 10:6, 399-409 (1991)).

### a) Pharmaceutically Acceptable Carriers

192. The compositions, including antibodies, can be used therapeutically in combination with a pharmaceutically acceptable carrier.

193. Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

194. Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

195. The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdernally.

196. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

197. Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

198. Compositions for oral administration include powders or granules, suspensions or solutions in water or non-aqueous media, capsules, sachets, or tablets. Thickeners, flavorings, diluents, emulsifiers, dispersing aids or binders may be desirable.

199. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### b) Therapeutic Uses

200. The dosage ranges for the administration of the compositions are those large enough to produce the desired effect in which the symptoms disorder are effected. The dosage should not be so large as to cause adverse side effects, such as unwanted cross-reactions, anaphylactic reactions, and the like. Generally, the dosage will vary with the age, condition, sex and extent of the disease in the patient and can be determined by one of skill in the art. The dosage can be adjusted by the individual physician in the event of any counterindications. Dosage can vary, and can be administered in one or more dose administrations daily, for one or several days.

201. Other vectors which do not have a specific pharmacuetical function, but which may be used for tracking changes within cellular chromosomes or for the delivery of diagnositc tools for example can be delivered in ways similar to those described for the pharmaceutical products.

202. The nucleic acid constructs of the invention can also be used for example as tools to isolate and test new drug candidates for a variety of diseases. They can also be used for the continued isolation and study, for example, the cell cycle. There use as exogenous DNA delivery devices can be expanded for nearly any reason desired by those of skill in the art.

### 5. Sequence similarities

203. It is understood that as discussed herein the use of the terms homology and identity mean the same thing as similarity. Thus, for example, if the use of the word homology is used between two non-natural sequences it is understood that this is not necessarily indicating an evolutionary relationship between these two sequences, but rather is looking at the similarity or relatedness between their nucleic acid sequences. Many of the methods for determining homology between two evolutionarily related molecules are routinely applied to any two or more nucleic acids or proteins for the purpose of measuring sequence similarity regardless of whether they are evolutionarily related or not.

204. In general, it is understood that one way to define any known variants and derivatives or those that might arise, of the disclosed genes and proteins herein, is through defining the variants and derivatives in terms of homology to specific known sequences. This identity of particular sequences disclosed herein is also discussed elsewhere herein. In general, variants of genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent homology to the stated sequence or the native sequence. Those of skill in the art readily understand how to determine the homology of two proteins or nucleic acids, such as genes. For example, the homology can be calculated after aligning the two sequences so that the homology is at its highest level.

205. Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

206. The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989 for at least material related to nucleic acid alignment. It is understood that any of the methods typically can be used and that in certain instances the results of these various methods may differ, but the skilled artisan understands if identity is found with at least one of these methods, the sequences would be said to have the stated identity, and be disclosed herein.

207. For example, as used herein, a sequence recited as having a particular percent homology to another sequence refers to sequences that have the recited homology as calculated by any one or more of the calculation methods described above. For example, a first sequence has 80 percent homology, as defined herein, to a second sequence if the first sequence is calculated to have 80 percent homology to the second sequence using the Zuker calculation method even if the first sequence does not have 80 percent homology to the second sequence as calculated by any of the other calculation methods. As another example, a first sequence has 80 percent homology, as defined herein, to a second sequence if the first sequence is calculated to have 80 percent homology to the second sequence using both the Zuker calculation method and the Pearson and Lipman calculation method even if the first sequence does not have 80 percent homology to the second sequence as calculated by the Smith and Waterman calculation method, the Needleman and Wunsch calculation method, the Jaeger calculation methods, or any of the other calculation methods. As yet another example, a first sequence has 80 percent homology, as defined herein, to a second sequence if the first sequence is calculated to have 80 percent homology to the second sequence using each of calculation methods (although, in practice, the different calculation methods will often result in different calculated homology percentages).

### 6. Hybridization/selective hybridization

208. The term hybridization typically means a sequence driven interaction between at least two nucleic acid molecules, such as a primer or a probe and a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.

209. Parameters for selective hybridization between two nucleic acid molecules are well known to those of skill in the art. For example, in some embodiments selective hybridization conditions can be defined as stringent hybridization conditions. For example, stringency of hybridization is controlled by both temperature and salt concentration of either or both of the hybridization and washing steps. For example, the conditions of hybridization to achieve selective hybridization may involve hybridization in high ionic strength solution (6X SSC or 6X SSPE) at a temperature that is about 12-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art. (Sambrook et al., Molecular Cloning: A Laboratory Manual, 2nd Ed., Cold Spring Harbor Laboratory, Cold Spring Harbor, New York, 1989; Kunkel et al. Methods Enzymol. 1987:154:367, 1987 for material at least related to hybridization of nucleic acids). A preferable stringent hybridization condition for a DNA: DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or A-T richness of any area wherein high homology is desired, all as known in the art.

210. Another way to define selective hybridization is by looking at the amount (percentage) of one of the nucleic acids bound to the other nucleic acid. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting primer is in for example, 10 or 100 or 1000 fold excess. This type of assay can be performed at under conditions where both the limiting and non-limiting primer are for example, 10 fold or 100 fold or 1000 fold below their k_{d}, or where only one of the nucleic acid molecules is 10 fold or 100 fold or 1000 fold or where one or both nucleic acid molecules are above their k_{d}.

211. Another way to define selective hybridization is by looking at the percentage of primer that gets enzymatically manipulated under conditions where hybridization is required to promote the desired enzymatic manipulation. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer molecules are extended. Preferred conditions also include those suggested by the manufacturer or indicated in the art as being appropriate for the enzyme performing the manipulation.

212. Just as with homology, it is understood that there are a variety of methods herein disclosed for determining the level of hybridization between two nucleic acid molecules. It is understood that these methods and conditions may provide different percentages of hybridization between two nucleic acid molecules, but unless otherwise indicated meeting the parameters of any of the methods would be sufficient. For example if 80% hybridization was required and as long as hybridization occurs within the required parameters in any one of these methods it is considered disclosed herein.

213. It is understood that those of skill in the art understand that if a composition or method meets any one of these criteria for determining hybridization either collectively or singly it is a composition or method that is disclosed herein.

### 7. Nucleic acids

214. There are a variety of molecules disclosed herein that are nucleic acid based, including for example the nucleic acids that encode, for example a chimeric transposase, as well as various functional nucleic acids. The disclosed nucleic acids are made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if, for example, an antisense molecule is introduced into a cell or cell environment through for example exogenous delivery, it is advantagous that the antisense molecule be made up of nucleotide analogs that reduce the degradation of the antisense molecule in the cellular environment.

### a) In vivo/ex vivo

215. As described above, the compositions can be administered in a pharmaceutically acceptable carrier and can be delivered to the subject=s cells in vivo and/or *ex vivo* by a variety of mechanisms well known in the art (e.g., uptake of naked DNA, liposome fusion, intramuscular injection of DNA via a gene gun, endocytosis and the like).

216. If *ex vivo* methods are employed, cells or tissues can be removed and maintained outside the body according to standard protocols well known in the art. The compositions can be introduced into the cells via any gene transfer mechanism, such as, for example, calcium phosphate mediated gene delivery, electroporation, microinjection or proteoliposomes. The transduced cells can then be infused (e.g., in a pharmaceutically acceptable carrier) or homotopically transplanted back into the subject per standard methods for the cell or tissue type. Standard methods are known for transplantation or infusion of various cells into a subject.

### 8. Peptides

### a) Protein variants

217. As discussed herein there are numerous variants of the chimeric integrating enzymes and that are known and herein contemplated. In addition, there are derivatives of the chimeric integrating enzymes which also function in the disclosed methods and compositions. Protein variants and derivatives are well understood to those of skill in the art and in can involve amino acid sequence modifications. For example, amino acid sequence modifications typically fall into one or more of three classes: substitutional, insertional or deletional variants. Insertions include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Immunogenic fusion protein derivatives, such as those described in the examples, are made by fusing a polypeptide sufficiently large to confer immunogenicity to the target sequence by cross-linking in vitro or by recombinant cell culture transformed with DNA encoding the fusion. Deletions are characterized by the removal of one or more amino acid residues from the protein sequence. Typically, no more than about from 2 to 6 residues are deleted at any one site within the protein molecule. These variants ordinarily are prepared by site specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known, for example M13 primer mutagenesis and PCR mutagenesis. Amino acid substitutions are typically of single residues, but can occur at a number of different locations at once; insertions usually will be on the order of about from 1 to 10 amino acid residues; and deletions will range about from 1 to 30 residues. Deletions or insertions preferably are made in adjacent pairs, i.e. a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary mRNA structure. Substitutional variants are those in which at least one residue has been removed and a different residue inserted in its place. Such substitutions generally are made in accordance with the following Tables 1 and 2 and are referred to as conservative substitutions.

218.

**TABLE 1:Amino Acid Abbreviations**

| **Amino Acid** | **Abbreviations** |
|---|---|
| alanine | Ala; A |
| allosoleucine | Alle |
| arginine | Erg; R |
| asparagine | Asn; N |
| aspartic acid | Asp; D |
| cysteine | Cys; C |
| glutamic acid | Glu; E |
| glutamine | Gln; Q |
| glycine | Gly; G |
| histidine | His; H |
| isolelucine | Ile; I |
| leucine | Leu; L |
| lysine | Lys; K |
| phenylalanine | Phe; F |
| proline | Pro; P |
| pyroglutamic acidp | Glu |
| serine | Ser; S |
| threonine | Thr; T |
| tyrosine | Tyr; Y |
| tryptophan | Trp; W |
| valine | Val; V |

| TABLE 2:Amino Acid Substitutions |
|---|
| Original Residue Exemplary Conservative Substitutions, others are known in the art. |
| Ala; Ser |
| Arg; Lys, Gln |
| Asn; Gln; His |
| Asp; Glu |
| Cys; Ser |
| Gln; Asn, Lys |
| Glu; Asp |
| Gly; Pro |
| His; Asn; Gln |
| Ile; Leu; Val |
| Leu; Ile; Val |
| Lys; Arg; Gln; |
| Met; Leu; Ile |
| Phe; Met; Leu; Tyr |
| Ser; Thr |
| Thr; Ser |
| Trp; Tyr |
| Tyr; Trp; Phe |
| Val; Ile; Leu |

219. Substantial changes in function or immunological identity are made by selecting substitutions that are less conservative than those in Table 2, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions which in general are expected to produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.

220. For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, Ile, Leu; Asp, Glu; Asn, Gln; Ser, Thr; Lys, Arg; and Phe, Tyr. Such conservatively substituted variations of each explicitly disclosed sequence are included within the mosaic polypeptides provided herein.

221. Substitutional or deletional mutagenesis can be employed to insert sites for N-glycosylation (Asn-X-Thr/Ser) or O-glycosylation (Ser or Thr). Deletions of cysteine or other labile residues also may be desirable. Deletions or substitutions of potential proteolysis sites, e.g. Arg, is accomplished for example by deleting one of the basic residues or substituting one by glutaminyl or histidyl residues.

222. Certain post-translational derivatizations are the result of the action of recombinant host cells on the expressed polypeptide. Glutaminyl and asparaginyl residues are frequently post-translationally deamidated to the corresponding glutamyl and asparyl residues. Alternatively, these residues are deamidated under mildly acidic conditions. Other post-translational modifications include hydroxylation of proline and lysine, phosphorylation of hydroxyl groups of seryl or threonyl residues, methylation of the o-amino groups of lysine, arginine, and histidine side chains (T.E. Creighton, Proteins: Structure and Molecular Properties, W. H. Freeman & Co., San Francisco pp 79-86 [1983]), acetylation of the N-terminal amine and, in some instances, amidation of the C-terminal carboxyl.

223. It is understood that one way to define the variants and derivatives of the disclosed proteins herein is through defining the variants and derivatives in terms of homology/identity to specific known sequences. Specifically disclosed are variants of these and other proteins herein disclosed which have at least, 70% or 75% or 80% or 85% or 90% or 95% homology to the stated sequence. Those of skill in the art readily understand how to determine the homology of two proteins. For example, the homology can be calculated after aligning the two sequences so that the homology is at its highest level.

224. Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

225. The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989 for at least material related to nucleic acid alignment.

226. It is understood that the description of conservative mutations and homology can be combined together in any combination, such as embodiments that have at least 70% homology to a particular sequence wherein the variants are conservative mutations.

227. As this specification discusses various proteins and protein sequences it is understood that the nucleic acids that can encode those protein sequences are also disclosed. This would include all degenerate sequences related to a specific protein sequence, i.e. all nucleic acids having a sequence that encodes one particular protein sequence as well as all nucleic acids, including degenerate nucleic acids, encoding the disclosed variants and derivatives of the protein sequences. Thus, while each particular nucleic acid sequence may not be written out herein, it is understood that each and every sequence is in fact disclosed and described herein through the disclosed protein sequence. For example, one of the many nucleic acid sequences that can encode a chimeric transposase obtained from linking a transposase [e.g. Tcl (Reference No. NM_061407, AI878683, AI878522, AI794017); P-element (Rio et al., Cell (1986) 44:21-32; among others)] to a DNA directing factor [e.g., LexA DBD (Accession No. J01643-V0029-V00300, Hin DNA binding domain (Reference No. J03245), STF-1 DNA binding domain (Reference No. S67435, corresponding to a.a. 140-215 described in Leonard et al. (1993) Mol. Endo. 7:1275-1283), among others]. The sequences can be obtained at Entrez Nucleotide Database, or GenBank or other nucleotide or protein search engines.

### 9. Kits

228. Disclosed herein are kits that are drawn to reagents that can be used in practicing the methods disclosed herein. The kits can include any reagent or combination of reagent discussed herein or that would be understood to be required or beneficial in the practice of the disclosed methods. For example, the kits could include primers to perform the amplification reactions discussed in certain embodiments of the methods, as well as the buffers and enzymes required to use the primers as intended.

### 10. Compositions with similar funtions

229. It is understood that the compositions disclosed herein have certain functions, such as directing a transposon to a target nucleic acid or binding to target nucleic acid. Disclosed herein are certain structural requirements for performing the disclosed functions, and it is understood that there are a variety of structures which can perform the same function which are related to the disclosed structures, and that these structures will ultimately achieve the same result.

### D. Methods of making the compositions

230. The compositions disclosed herein and the compositions necessary to perform the disclosed methods can be made using any method known to those of skill in the art for that particular reagent or compound unless otherwise specifically noted.

### 1. Nucleic acid synthesis

231. For example, the nucleic acids, such as, the oligonucleotides to be used as primers can be made using standard chemical synthesis methods or can be produced using enzymatic methods or any other known method. Such methods can range from standard enzymatic digestion followed by nucleotide fragment isolation (see for example, Sambrook et al., Molecular Cloning: A Laboratory Manual, 2nd Edition (Cold Spring Harbor Laboratory Press, Cold Spring Harbor, N.Y., 1989) Chapters 5, 6) to purely synthetic methods, for example, by the cyanoethyl phosphoramidite method using a Milligen or Beckman System 1Plus DNA synthesizer (for example, Model 8700 automated synthesizer of Milligen-Biosearch, Burlington, MA or ABI Model 380B). Synthetic methods useful for making oligonucleotides are also described by Ikuta et al., Ann. Rev. Biochem. 53:323-356 (1984), (phosphotriester and phosphitetriester methods), and Narang et al., Methods Enzymol., 65:610-620 (1980), (phosphotriester method). Protein nucleic acid molecules can be made using known methods such as those described by Nielsen et al., Bioconjug. Chem. 5:3-7 (1994).

### 2. Peptide synthesis

232. One method of producing the disclosed proteins is to link two or more peptides or polypeptides together by protein chemistry techniques. For example, peptides or polypeptides can be chemically synthesized using currently available laboratory equipment using either Fmoc (9-fluorenylmethyloxycarbonyl) or Boc (*tert* -butyloxycarbonoyl) chemistry. (Applied Biosystems, Inc., Foster City, CA). One skilled in the art can readily appreciate that a peptide or polypeptide corresponding to the disclosed proteins, for example, can be synthesized by standard chemical reactions. For example, a peptide or polypeptide can be synthesized and not cleaved from its synthesis resin whereas the other fragment of a peptide or protein can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively, to form an antibody, or fragment thereof. (Grant GA (1992) Synthetic Peptides: A User Guide. W.H. Freeman and Co., N.Y. (1992); Bodansky M and Trost B., Ed. (1993) Principles of Peptide Synthesis. Springer-Verlag Inc., NY (at least for material related to peptide synthesis). Alternatively, the peptide or polypeptide is independently synthesized *in vivo* as described herein. Once isolated, these independent peptides or polypeptides may be linked to form a peptide or fragment thereof via similar peptide condensation reactions.

233. For example, enzymatic ligation of cloned or synthetic peptide segments allow relatively short peptide fragments to be joined to produce larger peptide fragments, polypeptides or whole protein domains (Abrahmsen L et al., Biochemistry, 30:4151 (1991)). Alternatively, native chemical ligation of synthetic peptides can be utilized to synthetically construct large peptides or polypeptides from shorter peptide fragments. This method consists of a two step chemical reaction (Dawson et al. Synthesis of Proteins by Native Chemical Ligation. Science, 266:776-779 (1994)). The first step is the chemoselective reaction of an unprotected synthetic peptide-thioester with another unprotected peptide segment containing an amino-terminal Cys residue to give a thioester-linked intermediate as the initial covalent product. Without a change in the reaction conditions, this intermediate undergoes spontaneous, rapid intramolecular reaction to form a native peptide bond at the ligation site (Baggiolini M et al. (1992) FEBS Lett. 307:97-101; Clark-Lewis I et al., J.Biol.Chem., 269:16075 (1994); Clark-Lewis I et al., Biochemistry, 30:3128 (1991); Rajarathnam K et al., Biochemistry 33:6623-30 (1994)).

234. Alternatively, unprotected peptide segments are chemically linked where the bond formed between the peptide segments as a result of the chemical ligation is an unnatural (non-peptide) bond (Schnolzer, M et al. Science, 256:221 (1992)). This technique has been used to synthesize analogs of protein domains as well as large amounts of relatively pure proteins with full biological activity (deLisle Milton RC et al., Techniques in Protein Chemistry IV. Academic Press, New York, pp. 257-267 (1992)).

### 3. Process for making the compositions

235. Disclosed are processes for making the compositions as well as making the intermediates leading to the compositions. For example, disclosed are nucleic acids for the construction of a chimeric transposase obtained from linking a transposase [e.g. Tcl (Reference No. NM_061407, AI878683, AI878522, AI794017); P-element (Rio et al., Cell (1986) 44:21-32; among others)] to a DNA directing factor [e.g., LexA DBD (Accession No. J01643-V0029-V00300, Hin DNA binding domain (Reference No. J03245), STF-1 DNA binding domain (Reference No. S67435, corresponding to a.a. 140-215 described in Leonard et al. (1993) Mol. Endo. 7:1275-1283), among others]. The sequences of these and other known transposases can be obtained at Entrez Nucleotide Database, or GenBank or other nucleotide or protein search engines. There are a variety of methods that can be used for making these compositions, such as synthetic chemical methods and standard molecular biology methods. It is understood that the methods of making these and the other disclosed compositions are specifically disclosed.

236. Disclosed are nucleic acid molecules produced by the process comprising linking in an operative way a nucleic acid comprising the sequence set forth in a chimeric transposase obtained from linking a transposase [e.g. Tcl (Reference No. NM_061407, AI878683, AI878522, AI794017); P-element (Rio et al., Cell (1986) 44:21-32; and among others listed herein. The sequences can be obtained at Entrez Nucleotide Database, or GenBank or other nucleotide or protein search engines])] to a DNA directing factor [e.g., LexA DBD (Accession No. J01643-V0029-V00300, Hin DNA binding domain (Reference No. J03245), STF-1 DNA binding domain (Reference No. S67435, corresponding to a.a. 140-215 described in Leonard et al. (1993) Mol. Endo. 7:1275-1283), and among others listed herein. The sequences can be obtained at Entrez Nucleotide Database, or GenBank or other nucleotide or protein search engines]] and a sequence controlling the expression of the nucleic acid.

237. Also disclosed are nucleic acid molecules produced by the process comprising linking in an operative way a nucleic acid molecule comprising a sequence having 80% identity to a sequence set forth in a chimeric transposase obtained from linking a transposase [e.g. Tcl (Reference Nos. NM_061407, AI878683, AI878522, AI794017); P-element (Rio et al., Cell (1986) 44:21-32; and among others listed herein. The sequences can be obtained at Entrez Nucleotide Database, or GenBank or other nucleotide or protein search engines])] to a DNA directing factor [e.g., LexA DBD (Accession No. J01643-V0029-V00300, Hin DNA binding domain (Reference No. J03245), STF-1 DNA binding domain (Reference No. S67435, corresponding to a.a. 140-215 described in Leonard et al. (1993) Mol. Endo. 7:1275-1283), and among others listed herein. The sequences can be obtained at Entrez Nucleotide Database, or GenBank or other nucleotide or protein search engines], and a sequence controlling the expression of the nucleic acid.

238. Disclosed are nucleic acid molecules produced by the process comprising linking in an operative way a nucleic acid molecule comprising a sequence that hybridizes under stringent hybridization conditions to a sequence of a transposase set forth in a chimeric transposase obtained from linking a transposase [e.g. Tcl (Reference Nos. N_061407, AI878683, AI878522, AI794017); P-element (Rio et al., Cell (1986) 44:21-32; and among others listed herein. The sequences can be obtained at Entrez Nucleotide Database, or GenBank or other nucleotide or protein search engines])] to a DNA directing factor [e.g., LexA DBD (Accession No. J01643-V0029-V00300, Hin DNA binding domain (Reference No. J03245), STF-1 DNA binding domain (Reference No. S67435, corresponding to a.a. 140-215 described in Leonard et al. (1993) Mol. Endo. 7:1275-1283), and among others listed herein. The sequences can be obtained at Entrez Nucleotide Database, or GenBank or other nucleotide or protein search engines] and a sequence controlling the expression of the nucleic acid.

239. Disclosed are nucleic acid molecules produced by the process comprising linking in an operative way a nucleic acid molecule comprising a comprising a sequence encoding a fusion polypeptide containing two DNA binding domains (or a DNA binding and a protein binding domain) [e.g., LexA DBD (Accession No. J01643-V0029-V00300, Hin DNA binding domain (Reference No. J03245) linked to the STF-1 DNA binding domain (Reference No. S67435, corresponding to aa. 140-215 described in Leonard et al. (1993) Mol. Endo. 7:1275-1283) and among others listed herein which can be combined. The sequences can be obtained at Entrez Nucleotide Database, or GenBank or other nucleotide or protein search engines] and a sequence controlling an expression of the nucleic acid molecule.

240. Disclosed are nucleic acid molecules produced by the process comprising linking in an operative way a nucleic acid molecule comprising a sequence encoding a fusion polypeptide containing two DNA binding domains (or a DNA binding and a protein binding domain) [e.g., LexA DBD (Accession No. J01643-V0029-V00300, Hin DNA binding domain (Reference No. J03245) linked to the STF-1 DNA binding domain (Reference No. S67435, corresponding to a.a. 140-215 described in Leonard et al. (1993) Mol. Endo. 7:1275-1283) and among others listed herein which can be combined. The sequences can be obtained at Entrez Nucleotide Database, or GenBank or other nucleotide or protein search engines.] having 80% identity to a peptide and a sequence controlling an expression of the nucleic acid molecule.

241. Disclosed are cells produced by the process of transforming the cell with any of the disclosed nucleic acids. Disclosed are cells produced by the process of transforming the cell with any of the non-naturally occurring disclosed nucleic acids.

242. Disclosed are any of the disclosed peptides produced by the process of expressing any of the disclosed nucleic acids. Disclosed are any of the non-naturally occurring disclosed peptides produced by the process of expressing any of the disclosed nucleic acids. Disclosed are any of the disclosed peptides produced by the process of expressing any of the non-naturally disclosed nucleic acids.

243. Disclosed are animals produced by the process of transfecting a cell within the animal with any of the nucleic acid molecules disclosed herein. Disclosed are animals produced by the process of transfecting a cell within the animal any of the nucleic acid molecules disclosed herein, wherein the animal is a mammal. Also disclosed are animals produced by the process of transfecting a cell within the animal any of the nucleic acid molecules disclosed herein, wherein the mammal is mouse, rat, rabbit, cow, sheep, pig, or primate.

244. Also disclose are animals produced by the process of adding to the animal any of the cells disclosed herein.

### E. Examples

247. The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1

### a) Preparation of Nucleic Acid Constructs Encoding Invention

### Chimeric Proteins

248. Chimeric transposases [e.g. Tc1 (Reference No. NM_061407, AI878683, AI878522, AI794017); P-element (Rio et al., Cell (1986) 44:21-32; among others)] containing the DNA-binding domain at the"amino-terminal" or "carboxyl-terminal" are constructed using fusion PCR (see, e.g., Vallette, et al., 1989, NAR, 17:723-733; and Yon and Fried, 1989, NAR, 17:4895). The transposase coding region constructed as described and the DNA binding domain (e.g., zif268 coding region) constructed as described are separately amplified by PCR. Primers are designed employing well-known methods to contain a region of overlap that encodes the desired fusion junction. PCR products from the two separate reactions are then purified, mixed, and subjected to a second PCR reaction using primers directed at either side of the overlap region. In the first cycle of the second round, strands from the two reaction products can denature and anneal to allow extension by the polymerase. In the next cycle, the resulting strand can be amplified as in normal PCR.

249. Thus two unrelated sequences can be precisely fused: the transposon-based plasmid [coding for the transgene, transposase, and containing a protein binding site (e.g., λ operators)] and a second plasmid comprising a fusion polypeptide containing two DNA binding domains (or a DNA binding domain and a protein binding domain) [e.g., LexA DBD (Accession No. J01643-V0029-V00300) linked to the STF-1 DNA binding domain (Reference No. S67435; corresponding to a.a. 140-215 described in Leonard et al. (1993) Mol. Endo. 7:1275-1283) and among others listed herein which can be combined]. The sequences can be obtained at Entrez Nucleotide Database, or GenBank or other nucleotide or protein search engines] are constructed by methods utlized in Example 1 (see, e.g., Vallette, et al., 1989, NAR, 17:723-733; and Yon and Fried, 1989, NAR, 17:4895). The plasmids are transfected into pancreas-derived cells.

250. Cell Culture and Transfections. Pancreatic-derived cells are cultured in OptiMEM (GIBCO/BRL) supplemented with 5% serum and 50 µg/ml penicillin/streptomycinat 37°C and 5% CO₂. The tissue culture transposition assay are performed in a similar previously described (Ivics Z, et al. (1997) Cell 91:1-20).

251. After cotransfection the number of G418-resistant colonies are compared with the number obtained after cotransfection of a control transposon-based vector without the PBS. It was shown previously that the increase in the number of resistant colonies is caused by transposase-mediated integration of the transposon (Schouten GJ, et al (1998) Nucleic Acids Res 26:3687-3693 and Ivics Z, et al (1997)Cell:91:1-20) and therefore is indicative of the transposition efficiency.

252. The vectors of this invention are produced by standard methods of restriction enzyme cleavage, ligation and molecular cloning. The general protocol for constructing the subject vectors includes the following steps. First, purified nucleic acid fragments containing desired component nucleotide sequences as well as extraneous sequences are cleaved with restriction endonucleases from initial sources. Fragments containing the desired nucleotide sequences are then separated from unwanted fragments of different size using conventional separation methods, e.g., by agarose gel electrophoresis. The desired fragments are excised from the gel and ligated together in the appropriate configuration so that a circular nucleic acid or plasmid containing the desired sequences, e.g. sequences corresponding to the various elements of the subject vectors, as described above is produced. Where desired, the circular molecules so constructed are then amplified in a prokaryotic host, e.g. E. coli. The procedures of cleavage, plasmid construction, cell transformation and plasmid production involved in these steps are well known to one skilled in the art and the enzymes required for restriction and ligation are available commercially. (See, for example, R. Wu, Ed., Methods in Enzymology, Vol. 68, Academic Press, N.Y. (1979); T. Maniatis, E. F. Fritsch and J. Sambrook, Molecular Cloning: A Laboratory Manual, Cold Spring Harbor Laboratory Press, Cold Spring Harbor, N.Y. (1982); Catalog 1982-83, New England Biolabs, Inc.; Catalog 1982-83, Bethesda Research Laboratories, Inc.)

### 2. Example 2

253. Chimeric transposases are provided comprising known transposases (e.g., Sleeping Beauty, Tn7, Tn916, Tcl/mariner, Tc3, maT, and others listed herein) containing the lexA DNA binding domain (DBD) fused precisely at the N- or C- termini. Examples of known non-chimeric transposases can be found throughout the literature: Sleeping Beauty (Izsvak Z, Ivics Z, and Plasterk RH. (2000) Sleeping Beauty, a wide host-range transposon vector for genetic transformation in vertebrates. J. Mol. Biol. 302:93-102), Tn5 (Bhasin A, et al. (2000) Characterization of a Tn5 pre-cleavage synaptic complex. J Mol Biol 302:49-63), Tn7 (Kuduvalli PN, Rao JE, Craig NL. (2001) Target DNA structure plays a critical role in Tn7 transposition. EMBO J 20:924-932), Tn916 (Marra D, Scott JR. (1999) Regulation of excision of the conjugative tranposon Tn916. Mol Microbiol 2:609-621), Tc1/mariner (Izsvak Z, Ivics Z, Hackett PB. (1995) Characterization of a Tc-1 like transposable element in zebrafish (Danio rerio). Mol. Gen. Genet. 247:312-322), *Minos* and S elements (Franz G and Savakis C. (1991) Minos, a new transposable element from Drosophila hydei, is a member of the Tcl-like family of transposons. Nucl. Acids Res. 19:6646; Merriman PJ, Grimes CD, Ambroziak J, Hackett DA, Skinner P, and Simmons MJ. (1995) S elements: a family of Tcl-like transposons in the genome of Drosophila melanogaster. Genetics 141:1425-1438), Quetzal elements (Ke Z, Grossman GL, Cornel AJ, Collins FH. (1996) Quetzal: a transposon of the Tc1 family in the mosquito Anopheles albimanus. Genetica 98:141-147); Tor elements (Lam WL, Seo P, Robison K, Virk S, and Gilbert W. (1996) Discovery of amphibian Tcl-like transposon families. J Mol Biol 257:359-366*),* Tcl-like transposon subfamilies (Ivics. Z, Izsvak Z, Minter A, Hackett PB. (1996) Identification of functional domains and evolution of Tcl-like transposable elements. Proc. Natl. Acad Sci USA 93: 5008-5013), and Tc3 (Tu Z, Shao H. (2002) Intra- and inter-specific diversity of Tc-3 like tranposons in nematodes and insects and implications for their evolution and transposition. Gene 282:133-142), P-element (Rubin GM and Spradling AC. (1983) Vectors for P element mediated gene transfer in Drosophila. Nucleic Acids Res. 11:6341-6351. These fusion transposases enhance targeted integration into the host genome.

### a) Construction and purification of LexA-transposase fusion proteins.

254. The LexA DBD (Accession No. J01643-V0029-V00300) is fused directly to the N- or C-terminus of these transposases [e.g. Tc1 (Reference No. NM_061407, AI878683, AI878522, AI794017); P-element (Rio et al., Cell (1986) 44:21-32; among others)]. Techniques to construct fusion protein encoding nucleic acids and purification of their products are well known in the literature and to those skilled in the art.

255. Transposon linear and circular constructs are combined with their respective chimeric transposase and added to cellular extracts of different cell lines (e.g. HeLa cells) which contain a target plasmid. A target plasmid containing the LexA-binding sequence provides the DNA substrate for assaying site-selective integration. Depending upon the transposon vector (e.g., Tc1/mariner) being used further modifications are made to the target such as increasing the number of TA sites throughout the plasmid (including regions within, adjacent, and far from its putative target site) to determine the effect on the efficiency of integration. Methods of determining the site of integration have previously been described in literature (40, 84). The chimeric transposase can thus be assessed for its integration level and specificity compared to its respective transposase without a fused DNA binding domain.

### 3. Example 3:Targeted transposition of the maT transposon

### a) Assessing targeted integration of maT in insect cells.

256. *maT is* a member of the *Tc1*/*mariner* superfamily of transposons. Characteristic of mariner-like elements, maT has a DDD catalytic triad. The ITRs of *maT* more closely resemble those of *Tc1* than mariner and structural indications show the N-terminal domain to be unique from either mariner or *Tc1.* Additionally the DNA binding domain more closely resembles *Paxlpaired* transcription factors and *Tc3* transposase than the *Tel*/*mariner* transposases.

257. The ability of a modified, chimeric *maT transposase* to promote transposon integration to either Ga14 or LexA binding sites is assessed. Insect cell lines and insect embryos are transfected with two to three plasmids. The first plasmid, referred to as the donor plasmid, contains a modified *maT* transposon that has its inverted terminal repeats and transposase binding domains intact, but its transposase gene has been replaced or interrupted by a selectable marker gene (an antibiotic resistance gene). The second plasmid expresses both the DNA binding proteins and the *maTtransposase* gene fused either at the 5' or 3' ends to the heterologous DNA binding domains for Gal4 or LexA (or both). The essential elements of these two plasmids are also combined onto a single plasmid, to facilitate the co-transfection of these elements into the cells (see figure 7). The transposase gene is placed under the control of the promoter from the *Drosophila hsp70* gene. The final plasmid, the target plasmid, contains the target sites for Gal4 or LexA. Following delivery of the plasmids into the cells or embryos, expression of the transposase is induced by a heat shock, which promotes the transposition of the modified transposon. The presence of recombinant target plasmids containing the marked *maT* transposon is determined in treated cells/embryos and the integration sites assessed for sitespecific integration. To ensure that the integrations are transposon-dependent, control transfections are performed lacking the transposase plasmid.

### b) Assessing targeted integration of maT in human cells.

258. Human (HeLa) cell lines are transfected with the same two or three plasmids described above, except that the transposase is under the control a CMV promoter and the transposon contains the G418-resistance selectable marker under the control of the SV40 promoter. Recombinant target plasmids are recovered to assess transposon integration proximity relative to a LexA or Gal4 target site.

### c) Assessing targeted integration of maT in Drosophila.

259. In experiments that demonstrate that *maT transposon* integration can be preferentially directed to Gal4 or LexA sites in the cell lines, the donor and transposase plasmids are delivered into transgenic lines of *Drosophila* containing Gal4 target sites to assess targeted integration in an intact organism.

### 4. Example 4: Use of Gal4 and LexA DNA binding domains to achieve

### targeted integration of MosI and PiggyBac transposons.

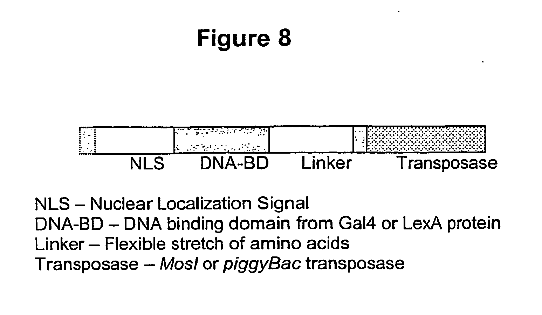

260. Gal4 and LexA DNA recognition sequences have been PCR amplified and cloned into the pGDVI target plasmid. *Mosl* and *piggyBac* transposons are fused to NLS and Linker sequences have been incorporated into oligonucleotides and used in PCR amplification reactions to produce the required DNA-binding domains for both Gal4 and LexA. Double Gal4-LexA DNA binding domain fusions, and modified pGDV1 target plasmids that contain recognition sequences for the Gal4 and LexA DNA binding domains are constructed using this same technique. The separate plasmids are then cut with restriction enzymes and ligated forming the complete construct as shown in Figure 8.

261. Interplasmid transposition assays are performed using the DNA binding domain-transposase helpers, modified donor transposons and modified pGDV1 target plasmids to determine a) a general increase in transposition frequency and b) a specific targeting of transposon integration at or near the recognition sequences.

262. Genetic transformation experiments are performed in *Drosophila melanogaster* strains that contain Gal4 or LexA recognition sequences, using the modified helper and donor transposons to determine a) a general increase in germ-line transformation frequency and b) a specific targeting of integrations to the genomic copies of the recognition sequences.

### 5. Example 5: Chimeric transposases

### a) Plasmids.

263. PCR fragments of the ORFs encoding the transposase proteins of Tc1, Tc3, *Himar1,* and *Mos1* were cloned into the Klenow-treated, 3.8-kb *Not*I fragment of pCMVβ (CLONTECH), resulting in, respectively, pRP1341, pRP1342, pRP1389, and pRP1353.

264. PCR fragments of the ORFs encoding the chimeric transposase proteins of Tc1, Tc3, *Himar1,* and *Mos1,* fused to the DNA binding domain of the 66 kD ALU-DNA repeat binding domain (Luk'yanov, et al. (1999) Biochemistry (Moscow) 64:25-33), the DNA binding domains from two proteins of 120 kDa and 35 kDa isolated from the HeLa cell proteins that specificaly bind to the Alu-element (Chiang Y and Vishwanatha JK (1996) Molecular and Cellular Biochemistry 155:131-138, 1996), or others contained herein and designed by methods provided by Kim, JS, et al. (Patent Application No. 20020061512) at the C-terminal domain of the transpoase, were cloned into the Klenow-treated, 3.8-kb *Not*I fragment of pCMVβ (CLONTECH), resulting in, respectively, pRP1341*, pRP1342*, prop1389*, and pRP1353* plasmid groups. The template plasmids were, respectively, pRP470 (Vos J, et al (1993) Genes Development 7,:1244-1253), pRP716 (Van Luenen, HGAM et al (1993) EMBO J.12:2513-2520), pMar27fH (Lampe DJ, et al (1999) PNAS 96:1142S-11433), and *pMos1* (Medhora M, et al (1991) Genetics 128:311-318). The mutations in the Tc3, *Mos1,* and *Hintar1* chimeric transposase ORFs are introduced either by site-directed mutagenesis using mutagenic primers or by a PCR-ligation-PCR method (Ali SA BioTechniques 18:746-750). The following cytomegalovirus (CMV) expression vectors are constructed: pRP2301* (Tc3 N225D/DBD), pRP2302* (Tc3 V41E N225D/DBD), pRP1390* (*Mos1* F344L/DBD), pRP1398* (*Himar1* H267R/DBD), pRP1399* *(Himar1* Q131R E137K/DBD), and *pRP2300**(*Himar1* Q131R E137K H267R/DBD) plasmid groups. Also, the plasmids which contain site-directed mutagenesis to the transposases of *Tc3* (pRP2301 and pRP23 02), *Mos1* (pRP1390), *Himar1* (pRP1398, pRP1399, and pRP2300) are tested as described below (Fischer SE (2001) PNAS 98:6759-6764).

265. A simian virus 40 (SV40)-G418 resistance cassette (a blunt-ended 1.6-kb *Bam*HI-*Eco*RI fragment ofpRc/CMV (Invitrogen)) is cloned into Tc1 [into the blunt-ended StyI sites of pRP1212 (Ketting RF, et al (1997) Nucleic Acids Res 25:4041-4047)], resulting in pRP1349, into Tc3 [into the blunt-ended *Bsp*EI and *Nco*I sites of Tc3 in pRP790 (Fischer SE, et al (1999) Mol Gen Genet 262:268-274)], resulting in pRP1351, *into Himar1* [the *Himar1* transposon is cut out from pMarKan (Lampe DJ, et al (1999) PNAS 96:11428-11433) by using *Not*I and *Eco*RI and cloned into the *Sma*I site of pUC19, the SV40-G418 resistance cassette is then cloned into the *Hinc*II and *Bst*EII sites], resulting in pRP 1347, and into *Mos1* [the *Mos1* transposon was PCR amplified from *pMos1 (pMos1* (Medhora M, et al (1991) Genetics 128:311-318) and cloned into the *Sma*I site of pUC19; the SV40-G418 resistance cassette is then cloned into the *Nru*I site]*,* resulting in pRP 1388.

### b) Cell Culture and Transfections.

266. Human HeLa cells are cultured in OptiMEM (GIBCO/BRL) supplemented with 5% serum and 50 µg/ml penicillin/streptomycin at 37°C and 5% CO₂. The tissue culture transposition assay are performed in a similar previously described (Ivics Z, et al. (1997) Cell 91:1-20).

### c) Efficiencies of Tc1/marinier Elements Compared in Human HeLa Cells.

267. The efficiencies of transposition of the *C*. *elegans* transposons Tc1 and Tc3, and the insect transposons *Hintar1,* and *Mos1* in human HeLa cells are compared. All transposase ORFs are cloned in identical restriction sites in a CMV expression vector. The corresponding transposons all are disrupted by an SV40-G418-resistance cassette. The transposase expression vector and the corresponding transposon vector are cotransfected into human HeLa cells. After cotransfection the number of G418-resistant colonies are compared with the number obtained after cotransfection of a control expression vector together with the transposon vector. It was shown previously that the increase in the number of resistant colonies is caused by transposase-mediated integration of the transposon (Schouten GJ, et al (1998) Nucleic Acids Res 26:3687-3693 and Ivics Z, et al (1997)Cell:91:1-20) and therefore is indicative of the transposition efficiency.

### 6. Example 6

### a) Chimeric transposases containing λR DNA binding domain (DBD) fused precisely at the N- or C-termini.

268. The λR DBD is fused directly to the N- or C-tenninus of a transposase [e.g. Tc1 (Reference No. NM_061407, AI878683, AI878522, AI794017); P-element (Rio et al., Cell (1986) 44:21-32; among others)]. Techniques to construct fusion protein encoding nucleic acids and purification of their products are well known in the literature and to those skilled in the art.

269. A transposon linear and circular construct is combined with their respective chimeric transposase and then added to cellular extracts of different cell lines (e.g. HeLa cells) which contain the target plasmid. The DNA substrate for assaying site-selective integretion of this vector is a plasmid containing the λ operators. Depending upon the transposon vector (e.g., Tc1/mariner) being used further modifications are made to the target such as increasing the number of TA sites throughout die plasmid (including regions within, adjacent, and far from its putative target site) to determined the effect on the efficiency of integration. This Chimeric transposase can be assessed for its integration level and specificity compared to its respective transposase without a fused DNA binding domain.

### 7. Example 7

### a) Transposon vector including a chimeric transposase containing λR DNA binding domain (DBD) fused precisely at the N- or C-termini.

270. The λR DBD is fused directly to the N- or C-terminus of a transposase [e.g. Tc1 (Reference No. NM_061407, AI878683, AI878522, AI794017); P-element (Rio et al., Cell (1986) 44:21-32; among others)]. Techniques to construct fusion protein encoding nucleic acids and purification of their products are well known in the literature and to those skilled in the art.

271. The vector is a plasmid which contains a transgene (e.g. antibiotic resistance, p53, or factor VIII gene) flanked by the tenninal repeats of a transposon which also contains a chimeric transposase (e.g., Sleeping Beauty) containing λR DNA binding domain (DBD) fused precisely at the N- or C-termini. The DNA substrate for assaying site-selective integration is a plasmid containing the I operators and antibiotic resistant gene which had previously been introduced into a cell line (e.g. HeLa cells). Depending upon the transposon construct used in the vector being used further modifications are made to the target plasmid such as interspersing TA sites throughout the plasmid (including regions within, adjacent, or far from its putative target site) as would be required in the Tc1/mariner transposon family. Upon transfection, cleavage and integration of the transgene flanked by the terminal repeats would occur as previously described in Figure 1. This chimeric transposase can be assessed for its integration level and specificity compared to its respective transposase without a fused DNA binding domain.

### 8. Examples 8

### a) Modification of Existing DNA-Binding Domains to Recognize New Target Recognition Sequences

### (1) Zinc Finger Modification

272. Modification of existing DNA-binding domains permits the recognition many new sequences. Many zinc finger proteins consist of several tandem finger repeats. In some of these proteins each finger recognizes three adjacent DNA bases (Desjarlais and Berg, 1993, PNAS, USA, 90:2256-2260; and Pavletich and Pabo, 1991, Science, 252:809). It has been found that these zinc fingers can be "mixed and matched" to yield new DNA binding specificities. For example, several zinc finger proteins containing the same three zinc fingers, but in rearranged order have been prepared (Desjarlais and Berg, 1993, PNAS. USA, 90:2256-2260). The modified proteins recognized the same 9 base pair sites, but with the three base pair units rearranged in the predicted order. Thus, new specificities are created by rearranging the order of zinc fingers in multi-finger proteins. Furthermore, in vivo selection methods for identifying zinc finger domain that recognize any given target site and other amino acid sequences of zinc finger domains that recognize a particular site are contained in Patent Application No. 20020061512.

273. In addition, new DNA-binding specificities have been conferred on individual Cys2 -His2 fingers (Rebar and Pabo, 1994, Science, 263:671-673). For example, phage display libraries have been used to isolate new fingers that recognize a preselected sequence from a library containing randomized recognition sequences. The zif-268 finger protein, which contains three zinc fingers, was randomized in the amino acids of one finger involved in DNA-contacting, and was expressed on the surface of a bacteriophage. Phage capable of binding a mutant DNA site were isolated by applying the phage display library to a dish coated with the DNA site of interest. After washing, bound phage were eluted with high salt and grown up. The selection was then repeated several more times, using as starting material for the next round the phage recovered from the previous round. At the end of this procedure, the remaining phage encoded zif268 derivatives with high affinities for the new DNA sequences. Those of skill in the art will recognize that combining the ability to evolve individual fingers to recognize new sites with the ability to mix and match different fingers allows the design of zinc finger proteins that recognize any DNA sequence.

### (2) Zif268 DNA-Binding Domain Modification to Recognize an L1 Element

274. L1 element DNA is an attractive binding domain recognition site because disruptions in such sequences are known to be harmless. The zif268 protein can be modified to bind to the sequence 5'GGGGCAGGG3', which is found near the 3' end of L1 elements (Hattori et al., 1985, NAR, 13:7813-7827). By following the rules of Desjarlais and Berg (Desjarlais and Berg, 1993, PNAS, USA, 90:2256-2260), and using a new recognition element from the work of Rebar and Pabo (Rebar and Pabo, 1994, Science, 263:671-673), a new specificity is designed to recognize this L1 sequence. Specifically, changing the critical recognition amino acids in the three fingers (13, 16, and 19 in each finger according to the numbering of Desjarlais and Berg) to contain the sequence RHR (finger 1), QGS (finger 2), and RHR (finger 3) produces a protein that recognizes the desired L1 sequence.

### (3) Modification of the Hin DNA-Binding Domain to Recognize an L1 Element

275. When a relatively small DNA-binding domain is desired (e.g. approximately 50 amino acids), directed in vitro evolution may be employed to modify the 52 amino acid Hin DNA-binding domain to recognize the L1 sequence or other desired sequences. For example, DNA encoding the Hin DNA-binding domain can be cloned into the fUSE2 vector (Parmley and Smith, 1988, Gene, 73:305-318), and selections carried out using the methods of Rebar and Pabo described above.

### 9. Example 9

### a) Preparation of a Protein-Binding Domain that Indirectly Attaches to Target Nucleic Acid by Binding to STF-1.

276. To produce a protein-binding first domain of a chimeric protein that binds to STF-1, the phage display library method is used to create an approximately 19 amino acid peptide that binds tightly to STF-1. The STF-1 protein-binding domain peptide is then fused to transposase to form a chimeric protein (as described herein). The transposase-STF-1 protein-binding domain chimeric protein is assayed (as described herein) to determine whether it targets integration to DNA bound to STF-1 in vitro. This chimeric protein is also incorporated into a transposon-based plasmid as described herein and tested intracellularly in cells expressing STF-1 (e.g., pancreas-derived cells).

### 10. Example 10

277. By utilizing similar host-sequences (i.e., which are nonfunctional) flanked by the terminal repeats in a transposon based system, one can target a gene and disrupt it with a higher efficiency than conventional methods. The potential for this technology, obviously extends to developing knock-out models, determining functions of genes, etc. Here we utilize, a transposon-based plasmid that contains the inverted terminal repeats of a transposon and homologous host sequences between and/or outside the terminal repeats. A transposase contained on another plasmid (or its mRNA) is microinjected into cells (e.g., mouse embryos) as described by Dupuy and colleagues (Dupuy AJ et al. Mammalian germ-line trangenesis by transposition. PNAS 99:4495-4499). Methods of determining the site of integration have previously been described in literature (40,84 as in references currently). The transposon-based plasmid with homologous sequences is compared to a control without homologous sequences.

### 11. Example 11

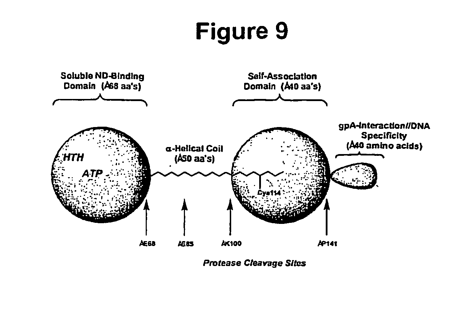

278. Replication of viral DNA in many double-stranded DNA viruses occurs via a rolling circle mechanism which yields linear concatemers of the viral genome (Furth ME and Wickner SH (1983) Lambda II, 145-155). The assembly of an infectious virus requires excision of a single genome from the concatemer, and concomitant insertion of the DNA into a preformed capsid (Casjen, S and Hendix, R (1988) The Bacteriophages, 15-92; Black LW (1989) Annu. Rev. Microbiol. 43, 267-292). Terminase enzymes are common to these viruses and are responsible for "packaging" of viral DNA (Black LW (1989) Annu. Rev. Microbiol. 43, 267-292; Fujisawa, H and Morita, M (1997) Genes to Cells 2, 537-545; Catalano, CE (2000) Cellular and Molecular Life Sciences, 57, 128-148). The small subunit of bacteriophage lambda terminase, gpNu1, is responsible for site-specific assembly of the holoenzyme at *cos,* the packaging initiation site of the lambda genome (Catalano, CE (2000) Cellular and Molecular Life Sciences, 57, 128-148). Specific binding interactions between gpNul and repeated "R-elements" within *cos* have been demonstrated. The domain organization of gpNul is described in Figure 9. The C-terminal ≈40 residues are involved in protein-protein interactions with the larger gpA subunit, while residues ≈100-140 define a hydrophobic self-assembly domain of the protein. The N-terminal ≈55 residues of the protein define the minimal DNA binding domain (DBD) of the protein while residues ≈55 - 100 form an extended helical coil that connect the DBD and the self-association domain of the protein.

279. Analysis of deletion constructs of the protein demonstrated that the DBD retains cos-specific DNA binding interactions; however, deletion of the self-association domain decreased DNA binding affinity by three orders of magnitude (Yang, Q et al. (1999) Biochemistry 38, 465-477; Yang, Q et al. (1999) Biochemistry 38, 14238-14247).

280. The integrase protein ofbacteriophage lambda is required for site-specific integration of viral DNA into the bacterial chromosome during lysogeny (Landy, A (1989) Ann. Rev. Biochem. 58, 913-949). The protein binds site-specifically to *"att"* sites in both the bacterial *(attB)* and viral *(attP)* genomes. Each *att* site contains an inverted pair of "core-type" binding sites (9 bp each) separated by an "overlap" region of 7 bp. Additional "arm" DNA binding elements flank the core-type binding sites. A domain organization of for phage lambda integrase has been defined, as follows. An amino-terminal domain (residues 1-64) binds with high affinity to the arm-type sites *of att,* while a carboxy-terminal domain (C65, residues 65-356) binds with low affinity to the core-type sites. This domain also possesses a sequence independent topoisomerase activity. A catalytic domain of the protein has been identified which comprises residues ≈170-356 (IntC 170), but this domain does not form stable complexes with att-containing DNA.

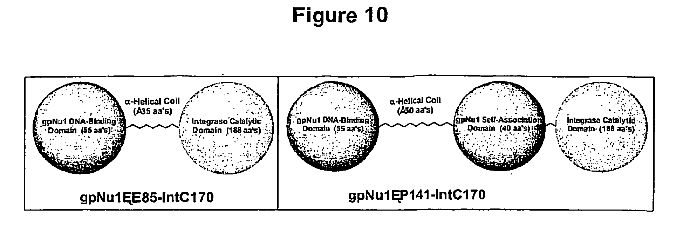

281. A construct comprising the N-terminal 85 residues of gpNu1 (gpNu1ΔE85) constitute a highly soluble DNA binding domain of the protein (Yang, Q et al. (1999) Biochemistry 38, 465-477; Yang, Q et al. (1999) Biochemistry 38, 14238-14247; *Structural and biophysical studies on this and related constructs have demonstrated that while residues 1 - 55 form a fully folded globular domain, residues ≈55 - 85 form an extended and flexible helical structure (Bain, 2001 #686);* de Beer, T et al. (2002) Mol. Cell 9, 981-991). This protein is thus ideal for the construction of a chimeric protein that provides a site-specific N-terminal DNA binding domain, linked via the flexible helix to the catalytic domain of integrase (IntC170). The construct comprising gpNu1ΔE85 and IntC170 is referred to as gpNu1ΔE85-IntC170 (Figure 10).

282. While gpNu1ΔE85 binds with specificity to *cos-DNA,* the affinity of this construct for viral DNA is relatively weak. Thus, gpNu1ΔP141-IntC170, a chimera that consists of the N-terminal 141 residues of gpNu1 linked to IntC170 (Figure 10) is constructed. It has been demonstrated that gpNu1ΔP141 binds to cos-containing DNA with an affinity equal to that of full-length protein (Yang, Q et al. (1999) Biochemistry 38, 14238-14247). Thus, the gpNu1ΔP141-IntC170 construct can provide a chimeric protein with high specificity and affinity for cos-containing DNA substrates.

283. Both of the chimeric constructs specifically target the IntC170 catalytic domain to cos-containing DNA substrates. The isolated IntC170 domain possesses a topoisomerase activity that is non-specific for any DNA sequence. Thus, the chimeric proteins possess a topoisomerase activity that is significantly enhanced in the presence of the cos sequence of phage lambda DNA.

284. Construction of the chimeric proteins gpNulΔE85-IntC170 and gpNu1ΔP141-IntC170 using PCR technology reveals that the former chimera possesses the highly soluble gpNul DNA binding domain, and the latter chimera further contains the gpNul self-association domain that provides high-affinity binding. Mature lambda DNA was used as a template to amplify viral DNA sequences encoding the N-terminal sequence of gpNul extending from Metl to Glu85, and Met1 to Pro141, respectively. The primers used are listed in Table 3. Note that amplification of DNA using these primers provides *EcoR*I and *Hpa*I restriction sequences at the upstream and downstream ends of the PCR product, respectively. Similarly, primers were synthesized that allow the amplification of the C-terminal sequence of lambda integrase extending from Ala170 to Lys356 (C170, Table 3). Amplification of IntCI70 using these primers provided *Not*I and *Hind*III restriction sequences at the upstream and downstream ends of the PCR product, respectively.

| **Primer** | **Sequence** | **Product** |
|---|---|---|
| gpNu1 Forward | TTCTCC-*GAATTC*-ATG-GAA-GTC-AAC-AAA-AAG-C (*Eco*RI) | - |
| gpNul-E85 Reverse | TCCTTC-*GTTAAC*-TTC-GTA-CTC-AAT-AGT-TCC-T (*Hpa*I) | 258 bp |
| gpNul-P141 Reverse | TCCTTC-*GTTAAC*-CGG-AAA-ACG-CCG-CTG-C (*Hpa*I) | 426 bp |
| Integrase Forward | AAGAAT-*GCGGCCGC*-GCA-GCA-AAA-TCA-GAG-GTA (*Not*I) | - |
| Integrase Reverse | ATTAAT-*AAGCTT*-TTA-TTT-GAT-TTC-AAT-TTT-GTC-C | 590 bp |
| | (*Hind*III) | |
| **Table 3. Primers used for PCR amplification.** Italicized sequence indicates the relevant restriction enzyme recognition sequences. | | |

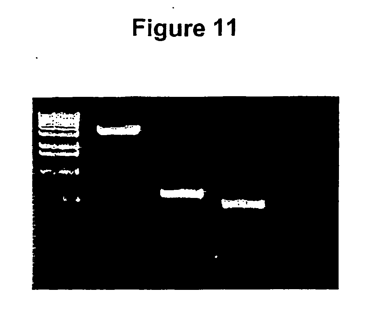

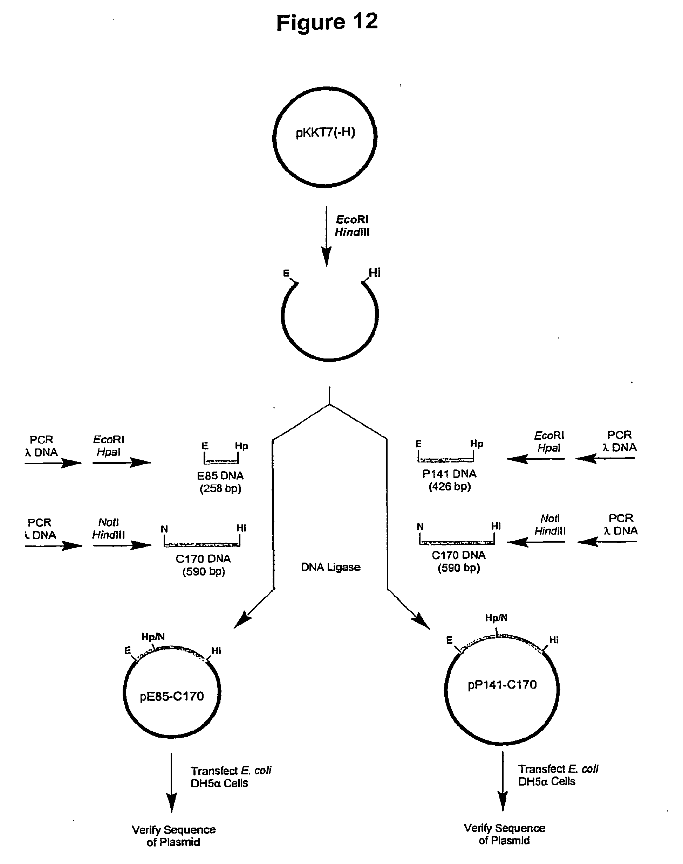

285. Figure 11 shows that amplification of all the appropriate sequences has been successfully accomplished. The PCR products have been purified, digested with the appropriate restriction endonucleases, and again purified by agarose gel electrophoresis. These PCR products are cloned into the plasmid pKKT7(-H) using the protocol presented in Figure 12.

286. To construct the protein expression vectors as outlined in Figure 12, *E.coli* DH5α cells are transformed with the ligation mixture and plasmid DNA isolated from ampicillin resistant colonies. The plasmids are analyzed by restriction digestion analysis and DNA sequencing to verify the presence of the appropriate inserts. Once the sequence of the vector has been verified, the plasmids are used to transform *E*. *coli* BL21(DE3) cells and the chimeric proteins expressed. Initial studies will examine the solubility and stability of the protein.

### 12. Example 12: Transposase-DNA binding domain transposition in cell lines

### a) Transformation of cell lines with neomycin resistance

287. Selecting for survival in the presence of G418, cell lines are transfected with a neomycin resistance transposon-based vector ± native transposase, to measure the frequency of transposition and heterologous recombination. The frequency of homologous recombination is measured using a vector that includes sequences homologous to the intended human genomic site. Next, cell lines are transfected with neomycin resistance transposon vectors plus chimeric transposase. Seven-day survival frequency greater than background is understood as putative evidence for transposition beyond the background levels of homologous or heterologous recombination. Sites of insertion are determined to test the hypothesis of site-specific transposition.

### b) Transformation of cell lines with beta-galactosidase

288. Cell lines are transfected with a CMV/beta-galactosidase transposon-based vector ± chimeric transposase or the native transposase. Seven-day beta-galactosidase specific activity in cellular extracts is understood as putative evidence for transposition. Sites of insertion are determined to test the hypothesis of site-specific transposition.

### c) Transformation of cell lines with luciferase

289. Cell lines are transfected with CMV/luciferase transposon-based vector ± chimeric transposase or the native transposase. Seven-day luciferase specific activity in cellular extracts is understood as putative evidence for transposition. Sites of insertion are determined to test the hypothesis of site-specific transposition.

### d) Transformation of cell lines with green fluorescent protein

290. Cell lines are transfected with a cocktail of a dexamethasone-inducible MMTV/EGFP vector ± chimeric transposase or the native transposase. Seven-day inducible EGFP fluorescence intensity in live cells is understood as putative evidence for transposition. Sites of insertion are determined to test the hypothesis of site-specific transposition.

### 13. Example 13: Transposase-TnsD targeted integration transposition in cell lines

### a) Transformation of cell lines with neomycin resistance

291. Selecting for survival in the presence of G418, cell lines are transfected with a neomycin resistance Tn7 vector, to measure the background frequency of heterologous recombination. The frequency of homologous recombination is measured using a vector that includes sequences homologous to the intended human genomic site. Next, cell lines are transfected with neomycin resistance Tn7 vectors plus TnsA, TnsB, TnsC, and TnsD transposition proteins of Tn7. Seven-day survival frequency greater than background is understood as putative evidence for transposition beyond the background levels of homologous or heterologous recombination. Sites of insertion are determined to test the hypothesis of sitespecific transposition. The DNA sequence recognition domain of TnsD is altered to optimize recognition of the cognate human target sequence and the minimum DNA binding domain of TnsD is determined.

### b) Transformation of cell lines with beta-galactosidase

292. After optimizing TnsD affinity for its human target and purifying the second generation protein, cell lines are transfected with a cocktail of a CMV/beta-galactosidase vector ± purified TnsA, TnsB, TnsC, and TnsD transposition proteins. Seven-day beta-galactosidase specific activity in cellular extracts is understood as putative evidence for transposition. Sites of insertion are determined to test the hypothesis of site-specific transposition.

### c) Transformation of cell lines with luciferase

293. A panel of expression vectors to produce TnsD DNA binding domain-transposase chimeras are constructed. The most efficient TnsD DNA binding domain-transposase chimera is truncated, particularly in the TnsD domain, to determine the minimum size active chimera. Similarly, spacing between TnsD and transposase domains is varied to determine the most efficient spacing for insertion at the desired site. For the transposase, TnsB (i.e., the catalytic domain of Tn7), maT, Tn5 transposase, λ phage intergrase, µ phage integrase, or HIV integrase can be used. TnsD may be oriented 3' or 5' of the transposase. Then the cell lines are transfected with a cocktail of a CMV/luciferase vector ± purified TnsD DNA binding domain-transposase. Seven-day luciferase specific activity in cellular extracts is understood as putative evidence for transposition. Sites of insertion are determined to test the hypothesis of site-specific transposition.

### d) Transformation of cell lines with green fluorescent protein

294. Cell lines are transfected with a cocktail of a dexamethasone-inducible MMTV/EGFP vector ± purified TnsD-transposase proteins. Seven-day inducible EGFP fluorescence intensity in live cells is understood as putative evidence for transposition. Sites of insertion are determined to test the hypothesis of site-specific transposition.

### 14. Example 14: Design and analysis of a recombinase with altered site-specificity

### a) Results

295. The precise modification of mammalian genomes is of major importance in gene therapy (J.M. Kaminski, et al., (2002) Faseb J. 16: 1242-1247). At present the site-specific modification of eukaryotic genomes relies on homologous recombination, which is too inefficient to be of use in gene therapy approaches. Therefore the site-specificity of the most efficient site-specific recombinase namely, Cre has been altered. Cre is a 34kDa gene product of bacteriophage P1 and interacts with sites termed loxP as part of the bacteriophage life-cycle (K. Abremski, et al., (1983) Cell 32: 1301-1311). It does not require any co-factors and catalyses DNA double strand exchanges in vitro and in cells of any origin. The protein surface with which Cre interacts with the loxP site is distributed over a large portion of the protein (F. Guo, et al., (1997) Nature 389: 40-46) and attempts to alter the site-specificity by mutagenesis of the aminoacids involved in the DNA protein interaction have not been successful.

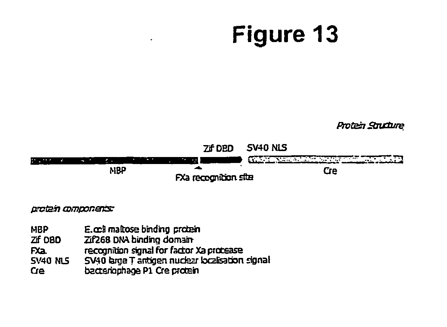

296. Therefore attempts were made to augment rather than replace the DNA binding ability of Cre by adding the DNA binding domain (DBD) of the human Zinc finger transcription factor Zif268 (H.A. Greisman, et al., (1997) Science 275: 657-661). The Zif268 DBD was fused to the N-terminus of the Cre recombinase such that a contiguous open reading frame is generated (Fig. 13). For ease of purification and stabilisation of the protein a fragment of the E. coli maltose binding protein (MBP) open reading frame was added to the N-terminus of the protein (Fig. 13). MBP-Cre fusion protein shows improved stability while retaining full catalytic activity in vitro and in vivo (A.F. Kolb and S.G. Siddell, (1996) Gene 183: 53-60).

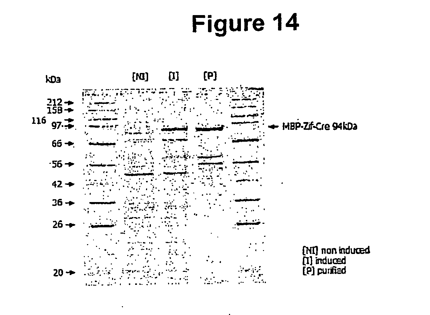

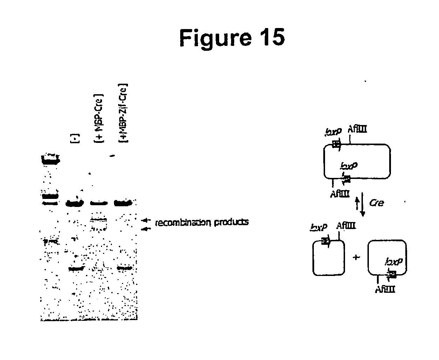

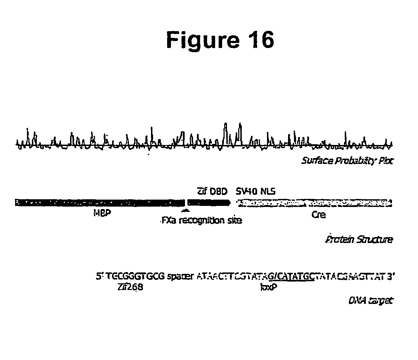

297. The MBP-Zif-Cre protein was purified via an amylose column (Fig. 14) and analysed for its ability to catalyse site-specific recombination between loxP sites. This analysis demonstrates that the addition of the Zif268 DBD to the MBP-Cre protein does not abolish its catalytical activity (Fig. 15). Chimeric binding sites were generated consisting of loxP sites and Zif268 binding sites, which are separated by spacer segments of varying length (Fig. 15). The binding domains were orientated such that they were compatible with the juxtaposed DNA binding domains of the MBP-Zif-Cre protein. The chimeric binding sites can be analysed for affinity to the fusion protein and for their ability to be recombined by the fusion protein. The completed construct is shown if Figure 16.

### b) Methods

298. The plasmid pMALc2-Cre encoding the MBP-Cre fusion protein has been described before. The Zif268 DNA binding domain has been excised from the plasmid pB-Zif268 as a 283bp SpeI/PstI fragment and ligated with the plasmid pMALc2-Cre digested with PstI/XbaI. The resulting plasmid pMALc2-Zif-Cre was transformed into E. coli TB1 cells. IN order to produce the MBP-Zif-Cre protein bacteria were grown to an OD600 of 0.5 in a total volume of 100ml and induced with a final concentration of 0.3mM of IPTG. Cells were lysed by sonication in a buffer containing 20mM Tris-HCl pH7.4, 200mM NaCl and 1mM EDTA. The lysate was cleared by centrifugation and the protein was purified via an amylose column as described (A.F. Kolb and S.G. Siddell, (1996) Gene 183: 53-60).

### 15. Example 15

299. The compositions described herein can utilize integrase derived from the Mu bacteriophage and other elements comprising an active cleaved donor complex (CDC) and further comprising a targeting mechanism whereby integration of a Mu transposable cassette may be directed to a predetermined target site within a host organism's genome. These integration vectors comprise a Mu transposable cassette and chimeric bacteriophage muA. Methods of the invention utilize the integration vectors of the invention to insert the Mu transposable cassette into a target site of an organism's genome. This insertion occurs in the absence of the MuB accessory protein. The methods are useful for modulating activity of known genes and for targeting integration of nucleotide sequences of interest into a specific location of an organism's genome. Accordingly, the methods may also be used to create gene disruptions and knockouts.

300. These integration vectors comprise a Mu cleaved donor complex (CDC) and a "chimeric transposase" that provides for transposition of the Mu transposable cassette in a sitespecific manner and in the absence of the accessory protein MuB (Suzuki, Hideki ; et al United States Patent Application 20020132350).

301. Active cleaved donor complexes (CDCs) can be obtained using an in vitro transposition reaction and a mini-Mu plasmid as the transposon donor. By "mini-Mu plasmid" is intended a plasmid comprising a Mu transposable cassette flanked by a nonMU plasmid DNA domain. Such mini-Mu plasmids can be constructed using molecular biology techniques well known in the art. See particularly Sambrook et al. (1989) Molecular Cloning: A Laboratory Manual (2d ed.; Cold Spring Harbor Laboratory Press, Plainview, N.Y.); and Ausubel et al., eds. (1995) Current Protocols in Molecular Biology (Greene Publishing and Wiley-Interscience, New York).

302. Compositions of the invention comprise novel integration vectors that are derived from CDCs of the temperate bacteriophage Mu, a bacterial class III transposon of Escherichia coli. This transposon exhibits extremely high transposition frequency (Toussaint and Rsibois (1983) in Mobile Genetic Elements, ed. Shapiro (Academic Press, New York), pp. 105-158). The Mu bacteriophage with its approximately 37 kb genome is relatively large compared to other transposons. Mu encodes two gene products that are involved in the transposition process: MuA transposase, a 70 kDa, 663 amino-acid multidomain protein, and MuB, an accessory protein of approximately 33 kDa. This transposable element has left end and right end MuA recognition sequences (designated "L" and "R", respectively) that flank the Mu transposable cassette, the region of the transposon that is ultimately integrated into the target site. Unlike other transposons known in the art, these ends are not inverted repeat sequences. The Mu transposable cassette, when necessary, may include a transpositional enhancer sequence (also referred to herein as the internal activating sequence, or "IAS") located approximately 950 base pairs inward from the left end recognition sequence.

303. The left and right end recognition sequences of the Mu transposon each encompass three 22-base-pair "end-type" MuA transposase binding sites, designated attL1 ("L1"), attL2 ("L2"), and attL3 ("L3"); and attR1 ("R1"), attR2 ("R2"), and attR3 ("R3"), which are numbered from the extreme ends of the Mu transposable cassette inwards (see FIG. 1). Two dinucleotide DNA cleavage sites reside outside the Mu transposable cassette, positioned 6 bp away from the end-most MuA-binding sites L1 and R1. The Mu transpositional enhancer sequence also binds the MuA transposase, but at a different domain of the protein than that used to bind the left and right end recognition sequences. MuA transposase interacts with the flanking left and right end recognition sequences and the transpositional enhancer sequence to bring about insertion of the Mu transposable cassette into a target DNA sequence.

304. Transposition is an essential feature of the life cycle of bacteriophage Mu. Integration of infecting Mu DNA into a host chromosome to form a stable lysogen occurs by nonreplicative simple insertion (Liebart et al. (1982) Proc. Natl. Acad. Sci. USA 79:4362-4366; Harshey (1984) Nature 311:580-581. During lytic growth, Mu generates multiple copies of its genome by repeated rounds of replicative transposition (Ljungquist and Bukhari (1977) Proc. Natl. Acad. Sci. USA 74:3143-3147) via a cointegrate pathway (Chaconas et al. (1981) J. Mol. Biol. 150:341-359). Both types of transposition are facilitated by the MuA transposase and accessory MuB protein. E. coli-encoded proteins such as histone-like protein ("HU") and integration host factor (IHF) assist in early conformational changes that ultimately lead to the transfer of the Mu transposable cassette into a target host DNA sequence.

305. The details of Mu transposition have been elucidated using an in vitro transposition reaction (Mizuuchi (1983) Cell 35:785-794; Mizuuchi (1984) Cell 39:395-404; Craigie and Mizuuchi (1985) Cell 41:867-876; Craigie et al. (1985) Proc. Natl. Acad. Sci. USA 82:750-7574; reviewed by Chaconas et al. (1996) Curr. Biol. 6:817-820; Craigie (1996) Cell 85:137-140; Lavoie and Chaconas(1995) Curr. Topics Microbiol. Immunol. 204:83-99; and Mizuuchi (1992) Annu. Rev. Biochem. 61:1011-1051). In this in vitro reaction, for example, the transposon donor is a mini-Mu plasmid, and another DNA molecule, commonly .phi.X174 replicative form DNA, serves as the target of transposition. The mini-Mu plasmid is constructed such that it comprises two DNA domains. The first of these DNA domains is a Mu transposable cassette, which is flanked by the second DNA domain, referred to herein as the non-Mu plasmid DNA domain.

306. Using an in vitro system, it has been shown that normally MuA transposase exists in its inert monomeric state which does not recognize the DNA cleavage sites adjacent to the left end and right end recognition sequences of the Mu transposable cassette. In the presence of HU, IHF, and divalent metal ions, particularly Mg²⁺, MuA transposase initially binds to the Mu transpositional enhancer sequence and to the left and right end recognition sequences. Following this binding, the mini-Mu plasmid undergoes a series of conformational changes that ultimately result in formation of the cleaved donor complex (CDC).

307. In normal bacteriophage Mu transposition, the structural and functional core of the CDC is a tetrameric unit of MuA molecules (Lavoie et al. (1991) EMBO J. 10:3051-3059; Mizuuchi (1992) Annu. Rev. Biochem. 61:1011-1051; Baker et al. (1993) Cell 74:723-733, hereinafter referred to as the MuA tetrameric core. The three end-type MuA transposase binding sites designated attL1, attR1, and attR2 are considered the core binding sites, as they are stably bound by the MuA tetramer. MuA protein interacting with the other three end-type MuA transposase binding sites (attL2, attL3, and attR3) is loosely bound. These loosely bound MuA molecules can be removed either by heparin, high salt (0.5 M NaCl), or excess Mu end competitor DNA (Kuo et al. (1991) EMBO J. 10:1585-1591; Lavoie et al. (1991) EMBO J. 10:3051-3059; Mizuuchi et al. (1991) Proc. Natl. Acad. Sci. USA 88:9031-9035). The three sites L1, L2, and L3 are considered accessory sites, as they are dispensable individually and are not required for the intermolecular strand transfer reaction (Allison and Chaconas (1992) J. Biol. Chem. 267:19963-19970; Lavoie et al. (1991) EMBO J. 10:3051-3059; and Mizuuchi et al. (1991) Proc. Natl. Acad. Sci. USA 88:9031-9035). However, sites R1, R2 and R3 may be interchanged with sites L1, L2, and L3 for use in constructing plasmids and in preparing the active cleaved donor complexes of this invention.

308. In the in vitro system, as well as in bacterial cells, the Mu-encoded protein MuB binds to target DNA in a non-specific manner in the presence of ATP. Accordingly, in the in vitro system, MuB binds to the target DNA molecule, while in vivo it binds to host DNA. The DNA-bound form of MuB has a strong affinity for the Mu CDC, and thus, when present, MuB introduces the CDC to the target molecule or host genome wherever MuB is bound. Because of the non-specific binding of MuB, CDC introduction occurs with little target preference. MuB also stimulates the DNA-breakage and DNA-joining activities of MuA (Adzuma and Mizuuchi (1988) Cell 53:257-266; Baker et al. (1991) Cell 65:1003-1013; Maxwell et al. (1987) Proc. Natl. Acad. Sci. USA 84:699-703; Surette and Chaconas (1991) J. Biol Chem. 266:17306-17313; Surette et al. (1991) J. Biol. Chem. 266:3118-3124; and Wu and Chaconas (1992) J. Biol. Chem. 267:9552-9558; and Wu and Chaconas, (1994) J. Biol. Chem. 269:28829-28833). Thus, MuB bound DNA molecules are preferential targets of Mu transposition. In the absence of MuB, introduction of the CDC to a target DNA site still occurs but is mainly limited to intramolecular reactions which take place in adjacent regions outside of Mu DNA.

309. The actual transfer of the Mu transposable cassette from the CDC into a target DNA site is mediated by the bound chimeric MuA transposase within the CDC. While the invention is not bound by any theory or mechanism of action, it is believed that the exposed 3' OH ends of the CDC act as nucleophiles, attacking the phosphodiester bond on the backbone of the target DNA. This attacking of a phosphate group by the exposed 3' OH group forms a bond between the 3' ends of the Mu DNA and the 5' ends of the target DNA. This process is referred to as strand transfer and results in formation of a strand transfer complex (STC). This stable nucleoprotein complex is involved in both cointegration and simple insertion (see generally, Haren et al. (1999) Ann. Rev. Microbiol 53:245-281). Cointegrates are made by replication of the Mu transposable cassette portion of the STC, using the free 3' ends of the target DNA as primers for leading-strand DNA synthesis. Simple inserts are formed from the STC by degradation of the non-Mu plasmid DNA domain that flanked the Mu transposable cassette portion of the donor molecule, followed by gap repair.

310. The integration vectors of the present invention comprise Mu bacteriophage "active" cleaved donor complexes (CDCs) with the chimeric muA transposase such that insertion of the Mu transposable cassette within the genome of a host organism occurs in a site-specific manner and in the absence of the accessory protein MuB. This integration can occur in the absence of in vivo expression of chimeric MuA transposase because active CDC has the intact chimeric MuA tetrameric core attached. These novel integration vectors allow for insertion of the entire Mu transposable cassette within a predetermined target site in any host organism's genome and thus may be referred to as "targeted CDCs." By "predetermined target site" is intended a desired location within the genome of the host organism for insertion of the Mu transposable cassette. Desired locations in the genome include, for example, locations in chromosomal DNA sequences, episomal sequences (e.g., replicable plasmids or viral replication intermediates), and chloroplast and mitochondrial DNA sequences. By "predetermined" is intended that the target site may be selected by the practitioner on the basis of known or predicted sequence information.

311. Active cleaved donor complexes (CDCs) can be obtained using an in vitro transposition reaction and a mini-Mu plasmid as the transposon donor. By "mini-Mu plasmid" is intended a plasmid comprising a Mu transposable cassette flanked by a nonMU plasmid DNA domain. Such mini-Mu plasmids can be constructed using molecular biology techniques well known in the art. See particularly Sambrook et al. (1989) Molecular Cloning: A Laboratory Manual (2d ed.; Cold Spring Harbor Laboratory Press, Plainview, N.Y.); and Ausubel et al., eds. (1995) Current Protocols in Molecular Biology (Greene Publishing and Wiley-Interscience, New York).

312. Any plasmid or mini-Mu plasmid can be used to obtain the CDCs, so long as it comprises the necessary elements within the Mu transposable cassette for formation of an active CDC. By "active CDC" is intended a CDC that is capable of carrying out intermolecular or intramolecular strand transfer in an in vitro transposition reaction. Such active CDCs, when modified to obtain the integration vectors of the present invention, will support intermolecular strand transfer in vivo. The necessary elements for active CDC formation depend upon the reaction conditions used during in vitro formation of the CDC (see, for example, Baker and Mizuuchi (1992) Genes and Develop. 6:2221-2232; Wu and Chaconas (1997) J. Mol. Biol. 267:132-141). However, it is possible to obtain an active CDC using a Mu transposable cassette the ends of which are defined by either the left or right MuA recognition sequences. Further, if precleaved cassettes are used, it is possible to obtain integration into the genome (i.e., an active CDC) which retains less than the full set of three binding sites of either the left or right MuA recognition sequence(s).

313. Thus, also disclosed is that an active CDC is obtained using a wild-type mini-Mu plasmid. By "wild-type mini-Mu plasmid" is intended the mini-Mu plasmid has a Mu transposable cassette that comprises the complete Mu left and right end recognition sequences in their natural (i.e., inverted) orientation; these recognition sequences flank an internal nucleotide sequence comprising the Mu transpositional enhancer sequence. By "complete Mu left and right end recognition sequences" is intended each of the end recognition sequences comprising the three naturally occurring 22-base-pair end-type MuA transposase binding sites. Thus, the left end recognition sequence comprises the attL1 attL2, attL3 end-type MuA transposase binding sites, while the right end recognition sequence comprises the attR1 attR2, and attR3 end-type MuA transposase binding sites. When present, the complete end recognition sequences allow for formation of an active CDC having the chiermic MuA transposase stably bound to the core binding sites attL1, attR1, and attR2 to form the MuA tetrameric core, and chimeric MuA transposase monomers loosely bound to the accessory end-type MuA transposase binding sites attL2, attL3, and attR3. The base pair sequences for the complete Mu left and right end recognition sequences and the Mu transpositional enhancer are known in the art. See Kahmann and Kamp (1979) Nature 280:247-250 and Allet (1978) Nature 274:553-558 for the Mu left end and right end recognition sequences; note, however, that both of these references contain sequencing errors. The connect sequence is found in Genbank Accession No. AF083977 (bacteriophage Mu sequence, contributed by Grimaud (Virology 217: 200-210 (1996) and Morgan et al., direct submission (Aug. 13, 1998)). See also, Mizuuchi and Mizuuchi (1989) Cell 58:399-408 for the Mu transpositional enhancer sequence. However, one of skill in the art will realize that the exact nucleotide sequence of these recognition sequences may vary slightly, and there is not an exact sequence requirement for individual binding domains. Thus, for example, the left end recognition sequence comprises three end-type MuA transposase binding sites that reside within nucleotides 1-180 of Genbank Accession No. AF083977, and the right end recognition sequence comprises three end-type MuA transposase binding sites that reside within nucleotides 36641-36662 of Genbank Accession No. AF083977. In, one embodiment of the invention, the MuA transposase binding sites in the left end recognition sequence are represented by nucleotides 6-27 (attL1), 111-132 (attL2), and 151-172 (attL3), respectively, of Genbank Accession No. AF083977; and the MuA transposase binding sites in the right end recognition sequence are represented by nucleotides 36691-36712 (attR1), 36669-36690 (attR2), and 36641-36662 (attR3), respectively, of Genbank Accession No. AF083977. One of skill will realize that variations of these sequences may be employed in the invention so long as the desired result is achieved. Thus, sequences having at least 65%, 70%, 75%, 80%, 85%, 90%, 91%, 92%, 93%, 94%, 95%, 96%, 97%, 98%, 99% or more sequence identity to the native Mu sequences may be employed.

314. Use of a wild-type mini-Mu plasmid to form an active CDC allows for the in vitro transposition reaction to be carried out under standard reaction conditions. For standard reaction conditions, see Mizuuchi et al. (1992) Cell 70:303-311 and Surette and Chaconas (1992) Cell 68:1101-1108. When a wild-type mini-Mu plasmid is used in the in vitro transposition reaction under standard conditions, the mini-Mu plasmid must be negatively supercoiled to form an active CDC. However, this requirement for supercoiling under standard reaction conditions can be relieved under other reaction conditions, for example, by including DMSO in the reaction mixture. See Baker and Mizuuchi (1992) Genes and Develop. 6:2221-2232.

315. Also disclosed is that an active CDC is obtained using a derivative mini-Mu plasmid. By "derivative mini-Mu plasmid" is intended a mini-Mu plasmid having a Mu transposable cassette that lacks one or more of the features of the Mu transposable cassette found in a wild-type mini-Mu plasmid. By "features" is intended the following: (1) a complete left end recognition sequence, (2) a complete right end recognition sequence, (3) left and right end recognition sequences in their natural orientation (i.e., inverted), and (4) a Mu transpositional enhancer sequence within the internal nucleotide sequence that is flanked by the left and right end recognition sequences. Thus, for example, a derivative mini-Mu plasmid lacking a complete left or right end recognition sequence lacks one or more of the end-type MuA transposase binding sites within its Mu transposable cassette.

316. Where a derivative mini-Mu plasmid is used to obtain an active CDC, the reaction conditions required in an in vitro transposition reaction will depend upon what wild-type mini-Mu plasmid feature is missing from the Mu transposable cassette. Thus, where the only feature missing is the accessory end-type MuA transposase binding site attR3, standard reaction conditions will yield an active CDC that supports intermolecular strand transfer (Baker and Mizuuchi (1992) Genes and Develop. 6:2221-2232).

317. Other derivative mini-Mu plasmids having additional features deleted from the Mu transposable cassette can be used to obtain an active CDC by varying the in vitro reaction conditions. For example, when dimethylsulfoxide (DMSO) is included in the transposition reaction under standard reaction conditions, mini-Mu plasmids lacking the Mu transpositional enhancer, carrying only a complete Mu left end or right end recognition sequence, carrying only a single end-type MuA transposase binding site adjacent to a DNA cleavage site with or without the Mu transpositional enhancer, or having left and right end recognition sequences in direct orientation (rather than inverted orientation) can be used to form a CDC that is active in the DNA cleavage and strand transfer steps required for intermolecular transposition. See Baker and Mizuuchi (1992) Genes and Develop. 6:2221-2232. Also disclosed is that, the DNA cleavage site can be a site which is recognized and cleaved by the chimeric MuA protein, or it may be a site which is a restriction enzyme recognition site; thus, the DNA cleavage sites used may be native to the DNA sequence in which they are located or they may be engineered or added artificially to the sequence in which they are located.

318. Accordingly, any plasmid or mini-Mu plasmid that yields an active CDC may be used as the basis for obtaining the integration nucleic acid constructs of the invention. Examples of wild-type mini-Mu plasmids that may be used include, but are not limited to, the pBR322-based pBLO7 (7.2 kb; Lavoie (1993) in Structural Aspects of the Mu Transpososome (University of Western Ontario, London, Canada); pUC19-based pBL03 (6.5 kb; Lavoie and Chaconas (1993) Genes Dev. 7:2510-2519; pMK:586 (Mizuuchi et al. (1991) Proc. Natl. Acad. Sci. USA 88:9031-9035); pMK108 (Mizuuchi (1983) Cell 35:785-794; Craigie and Mizuuchi (1986) Cell 45:793-800; pCL222 (Chaconas et al. (1981) Gene 13:37-46); and pBR322-based pGG215 (7.1 kb; Surette et al. (1987) Cell 49:253-262). Examples of derivative mini-Mu plasmids having one or more MuA binding sites and/or the transpositional enhancer sequence include, but are not limited to, pBL05 (MuA transposase binding site attR3 deleted from pBL03; Allison and Chaconas (1992) J. Biol. Chem. 267:19963-19970); pMK426 (carrying two Mu right end recognition sequences; Craigie and Mizuuchi (1987) Cell 51:493-501); pMK412 (pMK108 with the Mu transpositional enhancer sequence removed; Mizuuchi and Mizuuchi (1989) Cell 58:399-408); and pMK395 (mini-Mu with wrong relative orientation of the two Mu end sequences; Craigie and Mizuuchi (1986) Cell 45:793-800; and others described in Mizuuchi and Mizuuchi (1989) Cell 58:399-408. Also suitable for formation of an active mutant CDC are pUC19 derivatives carrying specific MuA-binding sites, such as the derivatives described by Baker and Mizuuchi et al. (1992) Genes and Develop. 6:2221-2232. All of the foregoing references describing such mini-Mu plasmids.

319. Where in vitro production of active CDCs is desired, the resulting mini-Mu plasmid is then subjected to the initial steps of the in vitro transposition reaction to form an active cleaved donor complex (CDC). Methods for producing active CDCs are well known in the art. See particularly Craigie et al. (1985) Proc. NatL. Acad. Sci. USA 82:7570-7574; Wu and Chaconas (1997) J. Mol. Biol. 267:132-141. The transposition reaction may be carried out under standard reaction conditions (Craigie et al. (1985) Proc. Natl. Acad. Sci. USA 82:7570-7574,) or under modified reaction conditions (such as with the addition of DMSO or glycerol; see, for example, Mizuuchi and Mizuuchi (1989) Cell 58:399-408) to obtain an active CDC.

320. Active CDCs may be obtained in vivo (i.e., in the host cell) where chimeric MuA is introduced into or expressed in a cell in which DNA from a mini-Mu plasmid or other plasmid capable of forming an active CDC is also present. In some embodiments, for example, formation of active CDCs from DNA of a mini-Mu plasmid previously integrated into the genome of the host organism could result in deletion of most of the previously integrated DNA and could also result in reintegration of the newly-formed active CDC into a different location of the host genome.

321. For example, where in vitro production of active CDCs is desired, a mini-Mu plasmid of interest is incubated with the purified chimeric MuA transposase protein and the E. coli HU protein, or biologically active variants or fragments thereof as defined below, in the presence of a divalent metal ion such as Mg2+ or Mn2+ (Mizuuchi et al. 1992 Cell 70:303-311). Where the Mu transposable cassette comprises a Mu transpositional enhancer sequence, the purified E. coli protein IHF or variant thereof is also included in the incubation reaction. Following formation of the CDC, the reaction is terminated by addition of EDTA (see Wu and Chaconas (1997) J. Mol. Biol. 267:132-141) to obtain the stable active CDC. Further spontaneous rearrangements of the CDC can also be inhibited by incubation at 0 degrees C. (see Surette et al. (1987) Cell 49:253-262)). Where the CDC has been derived from a wild-type mini-Mu plasmid, the loosely bound MuA transposase molecules may be removed to obtain a stripped-down version of the active CDC (Wu and Chaconas (1997) J. Mol. Biol. 267:132-141). This stripped-down active CDC may be used for preparing the integration vectors of the invention. However, when the active CDC comprises the MuA transposase molecules loosely bound to the accessory binding sites attL2, attL3, and attR3, intermolecular strand transfer occurs four times faster than with the stripped-down CDC (Wu and Chaconas (1997), supra). Thus, when a stripped-down CDC is to be used, additional chimeric MuA protein can be codelivered into the host cell to promoter intermolecular strand transfer. Additional chimeric MuA can be codelivered directly using a technique such as microinjection or particle bombardment, or it can be codelivered indirectly by delivering an expression vector comprising the chimeric MuA coding sequence operably linked to regulatory elements that promote expression in the host cell. Since the chimeric MuA must be imported into the nucleus, such a DNA construct would further comprise a sequence encoding a nuclear localization signal, such as the SV40 NLS, fused in frame with the chimeric MuA coding sequence. In addition to the chimeric MuA, other proteins or compounds may be helpful in achieving the desired results of increased frequency of non-random integration of the CDC, and such proteins or compounds may also be codelivered into the host cell with the vectors of the present invention.

322. Thus, a mini-Mu plasmid of interest and the chimeric MuA transposase, HU, and IHF proteins, or biologically active variants or fragments thereof, may be used in an in vitro reaction under standard or modified reaction conditions to obtain a stable active CDC that is capable of intermolecular transposition. During formation of this CDC, a nick has been introduced at each end of the Mu transposable cassette, exposing 3'--OH groups, relaxing the non-Mu plasmid DNA domain of the mini-Mu plasmid. This stable CDC may then be modified within the non-Mu plasmid DNA domain to obtain novel integration vectors of the invention.

323. Thus, the novel integration vectors of the invention may be obtained using mini-MU plasmids and any other necessary or helpful proteins, such as, for example, the native MuA transposase, the bacterial proteins HU, IHF, and a RecA-like protein, or biologically active variants or fragments thereof. Such proteins may be produced in vivo by the host genome, for example as the result of previous genetic engineering of the genome, or the proteins may be introduced along with the integration vectors during or after transformation of the host genome with the integration vectors. Such introduction may be direct or indirect (for example, by cotransformation of an integration vector with another DNA sequence encoding the native MuA transposase). Thus, active CDCs may be formed within the host cell where the appropriate elements and sequences exist within the cell.

324. Where purified proteins are to be used, methods for obtaining these purified native proteins or biologically active variants or fragments thereof are known in the art. See, for example, Craigie and Mizuuchi (1985) J. Biol. Chem. 260:1832-1835 (cloning of the MuA gene and purification of MuA); Craigie et al. (1985) Proc. Natl. Acad. Sci. USA 82:7570-7574, Rouviere-Yaniv and Gros (1975) Proc. Natl. Acad. Sci. USA 72:3428-3432, Dixon and Komberg (1984) Proc. Natl. Acad. Sci. USA 81:424-428, and Surette et al. Cell 49:253:226 (purification of HU); Wu and Chaconas (1994) J. Biol. Chem. 269:28829-28833, and the references cited therein (MuA, HU, and IHF); Yang et al. (1995) EMBO J 14:2374-2384 (native MuA and variants thereof, and HU); and Shibita et al. (1982) J. Biol. Chem. 257:370, Shibita et al. (1983) Methods Enzymol. 100:197, Cox et al. (1981) J. Biol. Chem. 256(9):4676, and Cox et al. (1981) Proc. Natl. Acad. Sci. USA 78:3433 (purified RecA). Methods of constructing chimeric integrating enzymes are described herein.

325. By "fragment" is intended a portion of the amino acid sequence and hence protein encoded thereby. For example, a biologically active portion of the MuA, HU, IHF, or RecA-like protein can be prepared by isolating a portion of their respective coding sequences, expressing the encoded portion of the respective protein (e.g., by recombinant expression in vitro), and assessing the activity of the encoded portion of the respective protein. The coding sequences for these proteins are known in the art. See, for example, Grimaud (1996) Virology 217(1):200-210 for the nucleotide sequence for the Mu bacteriophage (GenBank Accession No. AF083977), which identifies the coding sequence for the MuA transposase (GenBank Accession No. AAF01083); Miller (1984) Cold Spring Harb. Symp. Quant. Biol. 49:691-698 for the coding sequence for the IHF alpha-subunit (GenBank Accession No. P06984) and Flamm and Weisberg (1985) J. Mol. Biol. 183(2): 117-128 for the coding sequence for the IHF beta-subunit (GenBank Accession No. P08756); GenBank Accession No. U82664, nucleotides 40901-41173, which code for the HU protein (GenBank Accession No. AAB40196); and Keener et al. (1984) J. Bacteriol. 160(1):153-160 and the references cited elsewhere herein for coding sequences for RecA-like proteins.

### 16. Example 16

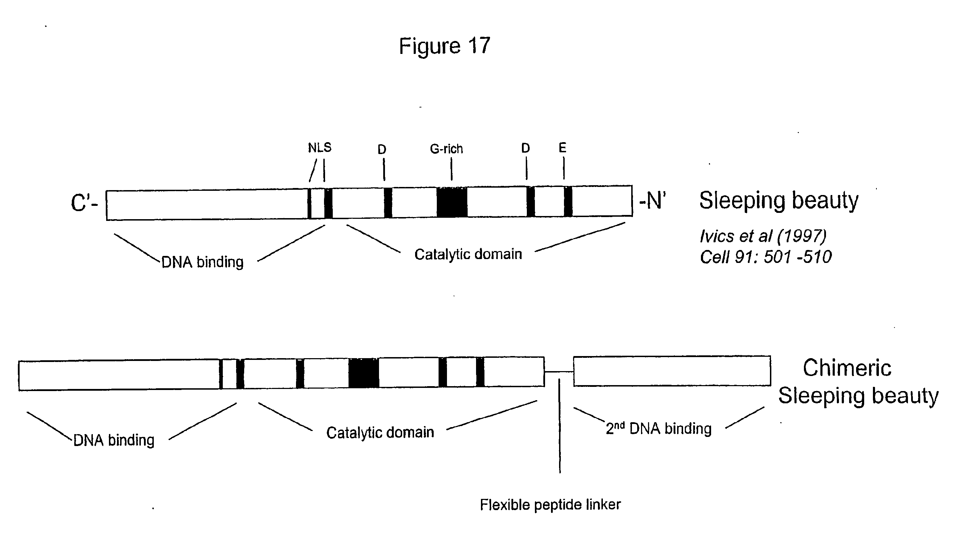

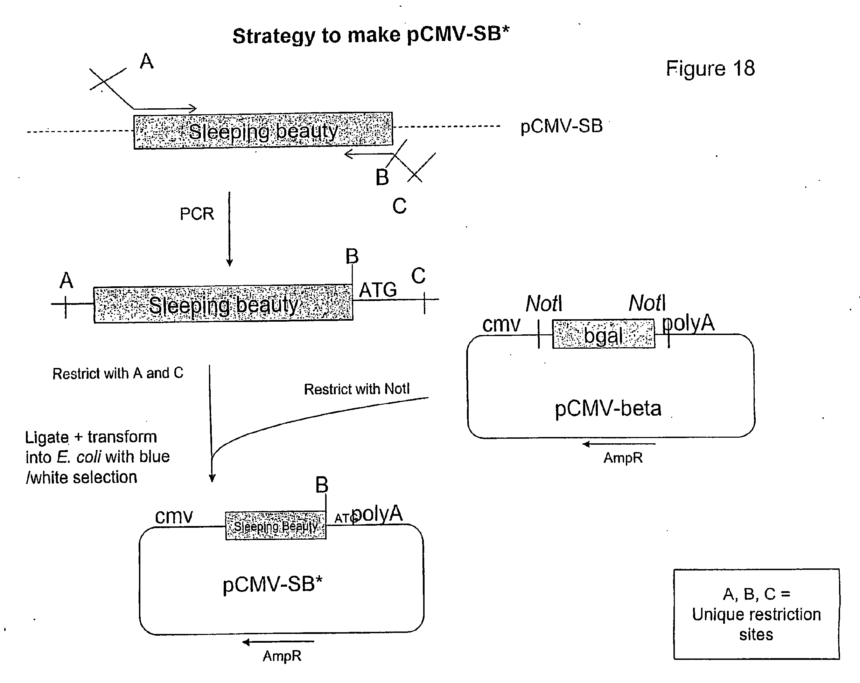

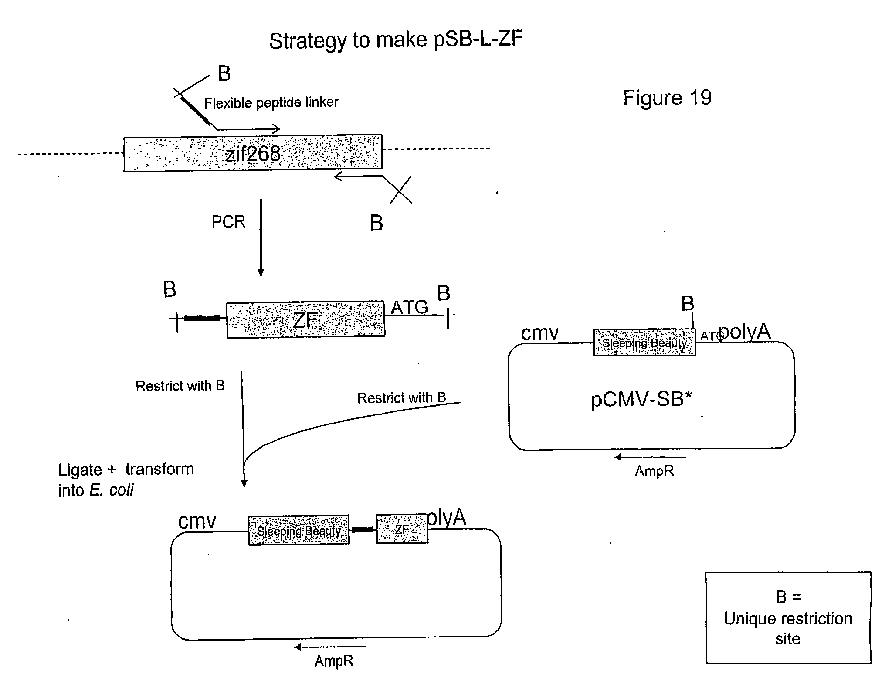

326. Development of a chimeric transposase uses techniques well-known in the art of molecular biology. For example a DNA binding domain from an exogenous source may be introduced onto the Sleeping beauty transposase (Figure 17). The strategy involves two cloning steps; both are PCR-based and involve primer-directed mutagenesis. The first step introduces a unique restriction site at the N-terminus of Sleeping Beauty (Figure 18). The second step introduces a DNA binding domain (either from zif268 or similar), along with a flexible peptide linker, into that restriction site (Figure 19). This flexible peptide linker can help the protein to adopt the necessary conformation to bind DNA and catalyse the transposition event simultaneously. The clones must be sequenced after each step, to select a vector that is free from mutations. With this strategy, unique restriction sites are also retained around the expression cassette (including promoter and poly-A signal) in the final vector, to allow this to be subcloned into other vectors (e.g. a suicide plasmid). By-products of this strategy are pCMV-antisenseSB*, and Sleeping Beauty with the DNA binding domain-flexible peptide linker fused in an antisense orientation.

### 17. Example 17: Expression vector for Transposase-DNA binding domain fusion protein

327. Two well-characterized DNA binding domains were chosen: those of Leu3p and Uga3p. Both Leu3p and Uga3p are transcriptional activators in *S*. *cerevisiae.* They bind to specific DNA sequences found in target genes. Their DNA binding domain consists of a zinc finger and a dimerization domain that allow homodimeric binding to DNA. Uga3p and Leu3p are highly related, but distinct DNA targets (Noel and Turcotte (1998) J. Biol. Chem. 273: 17463.), for example, the purified DNA binding domain of Leu3p (amino acids 1 to 147) binds in vitro to the DNA sequence SEQ ID No: 1 TCCGGCCGGAACCGGCTTT (Hellauer, et al.. (1996) Mol. Cell. Biol. 16:6096.)

328. Sequences corresponding to the DNA binding domain of Leu3p were amplified by PCR using S. cerevisiaegenomic DNA as a template and the following oligos:
Y10
   CG GGATCCCACCTATGGAAGGAAGATCAGATTT SEQ ID No: 2
Y11
   AGATTACTCGAGTCAAAGTGTTTTGTATGATGTCG SEQ ID No: 3

329. The PCR product was cut with BamHI and XhoI and subcloned into the plasmid pGRTEMP2. It was then cut with the compatible enzymes BglII and SalI. The transposase open reading frame contains a BglII site just before the stop codon and a SalI site just downstream of the stop codon (AAGATCTGATCCGTCGAC SEQ ID No: 4 with stop codon underlined). The fusion protein should be Tn5-Pro-Pro-Leu3p (a.a. 1-147). Plasmid encoding a transposase Uga3p (a.a.1-124) fusion was obtained similarly except that the oligos used for PCR were:
U11
   CGGGATCCCACCTATGAATTATGGCGTGGAGAA SEQ ID No: 5
U12
   AGATTACTCGAGTCAGTTGTACAGCTGCAATCCCA SEQ ID No: 6

330. Plasmids of the appropriate size were obtained. Constructs that were sequenced (using primer GGAAGCCCTGCAAAGTAAA SEQ ID No: 7) had Leu3p or Uga3p sequences inserted into pGRTEMP2. However, all the constructs sequenced had mutations that resulted in frameshifts in the coding region of Leu3p or Uga3p. As such, no full-length fusion proteins could be produced by bacteria transformed with the plasmids.

### 18. Example 18

331. Disclosed herein are examples of constructs of the present invention made in accordance with the teaching herein. The terminology used has the following meaning: TR = terminal repeat; E/P = Enhancer/ Promoter; Transgene = Nucleic acid inserted in the target DNA. The constructs all have the basic formula of terminal repeat enhancer/ promoter-transgene-terminal repeat - enhancer/ promoter ---chimeric transposase (transposase/ DNA docking factor), wherein the transposase would be represented by the name of the corresponding transposon (e.g., Sleeping Beauty and Tcl/mariner) and the DNA docking factor is represented by LexA, STF-1, Zif268, or any other docking factor disclosed herein. Additionally, the terminal repeats represent the repeat corresponding to the identified transposase. All genes represent the nucleic acid encoding the identified protein.
TR-E/P)-Transgene-TR-E/P------lambda integrase / Gal4 --------------
TR-E/P-Transgene-TR-E/P-----Gal4 / lambda integrase -------------
TR-E/P-Transgene-TR-E/P-----lambda integrase / LexA -----
TR-E-P-Transgene-TR-E/P----- LexA / lambda integrase -----------
TR-E/P-Transgene-TR-E/P--------piggybac/Gal4------------
TR-E/P-Transgene-TR-E/P-----Gal4 /piggybac-----
TR-E/P-Transgene-TR-E/P-----piggybac/LexA -----------
TR-E/P-Transgene-TR-E/P-----LexA /piggybac-----
TR-E/P-Transgene-TR-E/P-----mos1/Gal4 -------------
TR-E/P-Transgene-TR-E/P----- Gal4 mos1-------------
TR-E/P-Transgene-TR-E/P-----------mos1/LexA -------------
TR-E/P-Transgene-TR-E/P---------------LexA / mos1 ------------
TR-EIP-Transgene-TR-E/P------------Cre / Zif268--------------
TR-E/P-Transgene-TR-E/P------------Zif268/ Cre -------------
TR-E/P-Transgene-TR-E/P------------sleeping beauty/ LexA ----------------
TR-E/P-Transgene-TR-E-P-------------- LexA sleeping beauty ---------------
TR-E/P-Transgene-TR-E/P------------sleeping beauty /STF-1--------------
TR-E/P-Transgene-TR-E/P---------------STF-1/sleeping beauty -------------
TR-E/P-Transgene-TR-E/P-----------sleeping beauty / Zif268-------------
TR-E/P-Transgene-TR-E/P-------------- Zif268/sleeping beauty --------------
TR-E/P-Transgene-TR-E/P-------------sleeping beauty /XFin -------------
TR-E/P-Transgene-TR-E/P-------------Xfin/ sleeping beauty ---------------
TR-E/P-Transgene-TR-E/P------------sleeping beauty /glucocorticoid receptor---
TR-E/P-Transgene-TR-E/P--------------glucocorticoid receptor/ sleeping beauty ---
TR-E/P-Transgene-TR-E/P--------------sleeping beauty /434-cro --------------
TR-E/P-Transgene-TR-E/P---------434-cro/ sleeping beauty ------------
TR-E/P-Transgene-TR-E/P----------------sleeping beauty /MRF4---------------
TR-E/P-Transgene-TR-E/P----------------MRF4/sleeping beauty -------------------
TR-E/P-Transgene-TR-E/P-------------------sleeping beauty /PAL2-------------------
TR-E/P-Transgene-TR-E/P------------------PAL2/sleeping beauty -----------------
TR-E/P-Transgene-TR-E/P------------------sleeping beauty /GCN4--------------
TR-E/P-Transgene-TR-E/P----------------GCN4/sleeping beauty --------------
TR-E/P-Transgene-TR-E/P----------------sleeping beauty jun --------------
TR-E/P-Transgene-TR-E/P---------------jun /sleeping beauty --------------
TR-E/P-Transgene-TR-E/P---------------sleeping beauty / pin --------------
TR-E/P-Transgene-TR-E/P-------------- pin /sleeping beauty --------------
TR-E/P-Transgene-TR-E/P-----sleeping beauty /HSV-VP16--------------
TR-E/P-Transgene-TR-E/P-------------- HSV-VPl6/sleeping beauty --------------
TR-E/P-Transgene-TR-E/P--------------sleeping beauty / HOX --------------
TR-E/P-Transgene-TR-E/P-------------- HOX/ sleeping beauty --------------
TR-E/P-Transgene-TR-Egr-1--------------sleeping beauty/ LexA --------------
TR-E/P-Transgene-TR-Egr-1-------------- LexA / sleeping beauty --------------
TR-E-P-Transgene-TR-Egr-1--------------sleeping beauty /STF-1--------------
TR-E/P-Transgene-TR-Egr-1-------------- STF-1/sleeping beauty --------------
TR-E/P-Transgene-TR-Egr-1--------------sleeping beauty / Zif268--------------
TR-E/P-Transgene-TR-Egr-1--------------Zif268/sleeping beauty --------------
TR-E/P-Transgene-TR-Egr-1--------------sleeping beauty /XFin --------------
TR-E/P-Transgene-TR-Egr-1-------------- Xfin/sleeping beauty --------------
TR-E/P-Transgene-TR-Egr-1--------------sleeping beauty / glucocorticoid receptor-
TR-E/P-Transgene-TR-Egr-1-------------- glucocorticoid receptor/ sleeping beauty-
TR-E/P-Transgene-TR- Egr-1--------------sleeping beauty /434-cro --------------
TR-E/P-Transgene-TR-Egr-1--------------434-cro/ sleeping beauty --------------
TR-E/P-Transgene-TR- Egr-1--------------sleeping beauty /MRF4--------------
TR-E/P-Transgene-TR-Egr-1-------------- MRF4/sleeping beauty --------------
TR-E/P-Transgene-TR-Egr-1--------------sleeping beauty /PAL2--------------
TR-E/P-Transgene-TR-Egr-1---------------PAL2/sleeping beauty --------------
TR-E/P-Transgene-TR-Egr-1--------------sleeping beauty /GCN4--------------
TR-E/P-Transgene-TR-Egr-1-------------- GCN4/sleeping beauty --------------
TR-E/P-Transgene-TR- Egr-1--------------sleeping beauty / jun --------------
TR-E/P-Transgene-TR- Egr-1-------------- jun /sleeping beauty ------------
TR-E/P-Transgene-TR-Egr-1--------------sleeping beauty / pin --------------
TR-E/P-Transgene-TR-Egr-1-------------- pin /sleeping beauty --------------
TR-E/P-Transgene-TR-Egr-1--------------sleeping beauty /HSV-VP16--------------
TR-E/P-Transgene-TR-Egr-1-------------- HSV-VP16/sleeping beauty --------------
TR-E/P-Transgene-TR-Egr-1--------------sleeping beauty / HOX --------------
TR-E/P-Transgene-TR-Egr-1-------------- HOX/ sleeping beauty --------------
TR-E/P-Transgene-TR-GFAP--------------sleeping beauty/ LexA --------------
TR-E/P-Transgene-TR-GFAP-------------- LexA / sleeping beauty --------------
TR-E/P-Transgene-TR- GFAP--------------sleeping beauty /STF-1--------------
TR-E1P-Transgene-TR- GFAP--------------STF-1/sleeping beauty --------------
TR-E/P-Transgene-TR- GFAP----------sleeping beauty / ZifZ68--------------
R-E/P-Transgene-TR- GFAP-------------- Zif268/sleeping beauty -------------- --------------
TR-E/P-Transgene-TR-GFAP-----sleeping beauty /XFin -----
TR-E/P-Transgene-TR-GFAP-----Xfin/ sleeping beauty ------ -----
TR-E/P-Transgene-TR- GFAP-----sleeping beauty / glucocorticoid receptor-----------
TR-E/P-Transgene-TR-GFAP-----glucocorticoid receptor/ sleeping beauty-----------
TR-E/P-Transgene-TR- GFAP-----sleeping beauty /434-cro -----------
TR-E/P-Transgene-TR-GFAP-----434-cro/ sleeping beauty -----------
TR-E/P-Transgene-TR-GFAP-----sleeping beauty /MRF4-----------
TR-E/P-Transgene-TR- GFAP-----MRF4/sleeping beauty -----------
TR-E/P-Transgene-TR GFAP-----sleeping beauty /PAL2-----------
TR-E/P-Transgene-TR- GFAP---------- PAL2/sleeping beauty -----------
TR-E/P-Transgene-TR- GFAP-----------sleeping beauty /GCN4-----------
TR-E/P-Transgene-TR- GFAP-----GCN4/sleeping beauty -----------
TR-E/P-Transgene-TR-GFAP----------sleeping beauty / jun -----------
TR-E/P-Transgene-TR- GFAP-----jun /sleeping beauty -----------
TR-E/P-Transgene-TR- GFAP---------sleeping beauty / pin -----------
TR-E/P-Transgene-TR- GFAP-----pin /sleeping beauty -----------
TR-E/P-Transgene-TR- GFAP--------sleeping beauty /HSV-VP16-----------
TR-E/P-Transgene-TR- GFAP-----HSV-VP16/sleeping beauty -----------
TR-E/P-Transgene-TR- GFAP-----sleeping beauty / HOX -----------
TR-E/P-Transgene-TR- GFAP----------- HOX/ sleeping beauty -----------
TR-E/P-Transgene-TR-E/P-----Tc1/mariner/ LexA -----------
TR-E/P-Transgene-TR-E/P-----LexA / Tc1/mariner -----------
TR-E/P-Transgene-TR-E/P-----Tc1/mariner /STF-1-----------
TR-E/P-Transgene-TR-E/P-----STF-1/Tc1/mariner -----------
TR-E/P-Transgene-TR-E/P-----Tc1/mariner / Zif268-----
TR-E/P-Transgene-TR-E/P-----Zif268/Tc1/mariner ------- -----------
TR-E/P-Transgene-TR-E/P-----Tc1/mariner /XFin -----------
TR-E/P-Transgene-TR-E/P----- Xfin/ Tc1/mariner -----------
TR-E/P-Transgene-TR-E/P-----Tc1/mariner / glucocorticoid receptor-----------
TR-E/P-Transgene-TR-E/P-----glucocorticoid receptor/ Tc1/mariner -----------
TR-E/P-Transgene-TR-E/P-----Tc1/mariner/434-cro -----------
TR-E/P-Transgene-TR-E/P-----434-cro/ Tc1/mariner -----------
TR-E/P-Transgene-TR-E/P-----Tc1/mariner /MRF4------------
TR-E/P-Transgene-TR-E/P-----MRF4/Tc1/mariner -----------
TR-E/P-Transgene-TR-E/P-----Tc1/mariner/PAL2-----------
TR-E/P-Transgene-TR-E/P-----PAL2/Tc1/mariner -----------
TR-E/P-Transgene-TR-E/P-----Tc1/mariner /GCN4-----------
TR-E/P-Transgene-TR-E/P-----GCN4/Tc1/mariner-----------
TR-E/P-Transgene-TR-E/P-Tel/mariner / jun -----------
TR-E/P-Transgene-TR-E/P-----jun /Tc1/mariner -----------
TR-E/P-Transgene-TR-E/P-----Tc1/mariner / pin -----------
TR-E/P-Transgene-TR-E/P-----pin /Tc1/mariner -----------
TR-E/P-Transgene-TR-E/P-----Tc1/mariner /HSV-VP16-----------
TR-E/P-Transgene-TR-E/P-----HSV-VP16fTc1/mariner-----------
TR-E/P-Transgene-TR-E/P-----Tc1/mariner / HOX -----------
TR-E/P-Transgene-TR-E/P-----HOX/ Tc1/mariner -----------
TR-E/P-Transgene-TR-Egr-1-----Tc1/mariner/ LexA ------------
TR-E/P-Transgene-TR-Egr-1-----LexA / Tc1/mariner -----------
TR-E/P-Transgene-TR- Egr-1-----Tc1/mariner/STF-1-----------
TR-E/P-Transgene-TR-Egr-1-----STF-1/Tc1/mariner-----------
TR-E/P-Transgene-TR-Egr------Tc1/mariner / Zif268-----------
TR-E/P-Transgene-TR-Egr-1-----Zif268/Tcl/mariner -----------
TR-E/P-Transgene-TR-Egr-1-----Tc1/mariner /XFin-----------
TR-E/P-Transgene-TR-Egr-1-----Xfin/ Tc1/marin -----------
TR-E/P-Transgene-TR-Egr-1-----Tc1/mariner / glucocorticoid receptor-
TR-E/P-Transgene-TR-Egr-1-----glucocorticoid receptor/ Tc1/mariner--
TR-E/P-Transgene-TR-Egr-1-----Tc1/mariner /434-cro -----------
TR-E/P-Transgene-TR-Egr-1-----434-cro/Tc1/mariner -----------
TR-E/P-Transgene-TR-Egr-1-----Tc1/mariner /MRF4-----------
TR-E/P-Transgene-TR-Egr-1-----MRF4/Tc1/mariner-----------
TR-E/P-Transgene-TR-Egr-1-----Tc1/mariner /PAL1-----------
TR-E/P-Transgene-TR-Egr-1-----PAL2/Tc1/mariner -----------
TR-E/P-Transgene-TR-Egr-1------Tc1/mariner /GCN4-----------
TR-E/P-Transgene-TR-Egr-1-----GCN4/Tc1/mariner -----------
TR-E/P-Transgene-TR-Egr-1-----Tc1/mariner / jun -----------
TR-E/P-Transgene-TR- Egr-1-----jun /Tc1/mariner -----------
TR-E/P-Transgene-TR-Egr-1-----Tc1/mariner / pin -----------
TR-E/P-Transgene-TR-Egr-1-----pin /Tc1/mariner -----------
TR-E/P-Transgene-TR-Egr-1-----Tc1/mariner /HSV-VP16-----------
TR-E/P-Transgene-TR-Egr-1-----HSV-VP16/Tc1/mariner -----------
TR-E/P-Transgene-TR-Egr-1-----Tc1/mariner / HOX -----------
TR-E/P-Transgene-TR-Egr-1-----HOX/ Tc1/mariner -----------
TR-E/P-Transgene-TR-GFAP-----Tc1/mariner/ LexA -----------
TR-E/P-Transgene-TR-GFAP-----LexA / Tc1/mariner -----------
TR-E/P-Transgene-TR-GFAP-----Tc1/mariner/STF-1-----------
TR-E/P-Transgene-TR- GFAP----------- STF-1/Tc1/mariner-----------
TR-E/P Transgene-TR- GFAP-----------Tc1/mariner / Zif268-----------
TR-E/P-Transgene-TR- GFAP-----------Zif268/Tc1/mariner-----------
TR-E/P-Transgene-TR- GFAP-----------Tc1/mariner /XFin-----------
TR-E/P-Transgene-TR- GFAP-----------Xfin/ Tc1/mariner-----------
TR-E/P-Transgene-TR- GFAP-----------Tc1/mariner / glucocorticoid receptor---
TR-E/P-Transgene-TR- GFAP-----------glucocorticoid receptor/ Tc1/mariner ---
TR-E/P-Transgene-TR- GFAP-----------Tc1/mariner /434-cro-----------
TR-E/P-Transgene--TR-GFAP-----------434-cro/ Tc1/mariner-----------
TR-E/P-Transgene-TR- GFAP-----------Tc1/mariner /MRF4-----------
TR-E/P-Transgene-TR- GFAP-----------MRF4/Tc1/mariner-----------
TR-E/P-Transgene-TR- GFAP-----------Tc1/mariner /PAL2-----------
TR-E/P-Transgene-TR- GFAP-----------PAL2/Tc1/mariner-----------
TR-E/P-Transgene-TR- GFAP-----------Tc1/mariner /GCN4-----------
TR-E/P-Transgene-TR- GFAP-----------GCN4/Tc1/mariner-----------
TR-E/P-Transgene-TR- GFAP-----------Tc1/mariner / jun-----------
TR-E/P-Transgene-TR- GFAP-----------jun /Tc1/mariner-----------
TR-E/P-Transgene-TR- GFAP-----------Tc1/mariner / pin-----------
TR-E/P-Transgene-TR- GFAP-----------pin /Tc1/mariner-----------
TR-E/P-Transgene-TR- GFAP-----------Tc1/mariner /HSV-VP16-----------
TR-E/P-Transgene-TR- GFAP-----------HSV-VP16/Tc1/mariner-----------
TR-E/P-Transgene-TR- GFAP-----------Tc1/mariner / HOX-----------
TR-E/P-Transgene-TR- GFAP----------HOX/ Tc1/mariner-----------

332. While the invention has been described in detail with reference to certain preferred embodiments thereof, it will be understood that modifications and variations are within the spirit and scope of that which is described and claimed.

### F. References

1. Wu, G.Y., Zhan, P., Sze, L.L., Rosenberg, A.R., and Wu, C.H. (1994) Incorporation of adenovirus into a ligand-based DNA carrier system results in retention of original receptor specificity and enhances targeted gene expression. J. Biol. Chem. 269:11542-11546.
2. Chowdhury, N.R., Wu, C.H., Wu, G.Y., Yemeni, P.C., Bommineni, V.R, and Chowdhury, J.R. (1993) Fate of DNA targeted to the liver by asialoglycoprotein receptor-mediated endocytosis in vivo. J. Biol. Chem. 268: 11265-11271.

3. Farhood, H., Gao, X., Son, K., Yang, Y.Y., Lazo, J.S., Huang, L., Barsoum, J., Bottega, R., and Epand, R.M. (1994) Cationic liposomes for direct gene transfer in therapy of cancer and other diseases. Ann. NY Acad. Sci. 716:23-35.
4. Curiel, D.T. (1994) High efficiency gene transfer mediated by adenovirus-polylysine-DNA complexes. Ann. NY Acad. of Sci. 716:36-58.
5. Cotten, M., Wagner, E., Zatloukal, K., and Birnstiel ML. (1993) Chicken adenovirus (CELO virus) particles augment receptor-mediated DNA delivery to mammalian cells and yield exceptional levels of stable transformants. J. Virology 67: 3777-3785.
6. Schagen, F.H., Rademaker, H.J., Cramer, S.J., van Ormondt, H., van der Eb, A.J., van de Putte, P., and Hoeben, R.C. (2000) Towards integrating vectors for gene therapy: expression of functional bacteriophage MuA and MuB proteins in mammalian cells. Nucleic Acids Res. 28: E104.
7. Lestina, B.J., Sagnella, S.M., Xu, Z., Shive, M.S., Richter, N.J., Jayaseharan, J., Case, A.J., Kottke-Marchant, K., Anderson, J.M., and Marchant, R.E. (2002) Surface modification of liposomes for selective cell targeting in cardiovascular drug delivery. J. Control Release 78:235-247.
8. Moreira, J.N., Gaspar, R., and Allen, T.M. (2001) Targeting stealth liposomes in a murine model of human small cell lung cancer. Biochim. Biophys. Acta. 1515:167-176.
9.Xu, L., Tang, W.H., Huang, C.C., Alexander, W., Xiang, L.M., Pirollo, K.F., Rait, A., and Chang, E.H. (2001) Systemic p53 gene therapy of cancer with immunolipoplexes targeted by anti-transferrin receptor scFv. Mol. Med. 7:723-734.
10. Sudhan Shaik, M., Kanikkannan, N., and Singh, M. (2001) Conjugation of anti-My9 antibody to stealth monensin liposomes and the effect of conjugated liposomes on the cytotoxicity of immunotoxin. J. Control Realease 76:285-295.
11. Li, X., Stuckert, P., Bosch, I., Marks, J.D., and Marasco, W.A. (2001) Single-chain antibody-mediated gene delivery into ErbB2-positive human breast cancer cells. Cancer Gene Ther. 8:555-565.
12. Park, J.W., Kirpotin, D.B., Hong, K., Shalaby, R., Shao, Y., Nielsen, U.B., Marks, J.D., Papahadjopoules, D., and Benz, C.C. (2001) Tumor targeting using anti-her2 immunoliposomes. J. Control Release 74:95-113.
13. Lee, S.E., Jin, R.J., Lee, S.G., Yoon, S.J., Park, M.S., Heo, D.S., and Choi, H. (2000) Development of a new plasmid vector with PSA-promoter and enhancer expressing tissuespecificity in prostate carcinoma cell lines. Anticancer Res. 20:417-422.
14. Gottschalk, S., Cristiano, R.J., Smith, L.C., and Woo, S.L. (1993) Folate receptor mediated DNA delivery into tumor cells: potosomal disruption results in enhanced gene expression. Gene Ther 1:185-191.
15. Boulikas, T. (1997) Gene therapy of prostate cancer: p53, suicidal genes, and other targets. Anticancer Res. 17:1471-1505.
16. Kaneda, Y., Iwai, K., and Uchida, T. (1989) Increased expression of DNA cointroduced with nuclear protein in adult rat liver. Science 243:375-378.
17. Izsvak, Z., Ivics, Z., and Plasterk, R.H. (2000) Sleeping Beauty, a wide host-range transposon vector for genetic transformation in vertebrates. J. Mol. Biol. 302:93-102.
18. Sauer, B. (1994) Site-specific recombination: developments and applications. Curr. Opin. Biotechnol. 5:521-527.
19. Diaz, V., Rojo, F., Martinez-A, C., Alonso, J.C., and Bernard, A. (1999) The prokaryotic beta-recombinase catalyzes site-specific recombination in mammalian cells. J. Biol. Chem. 274:6634-6640.
20. O'Gorman, S., Fox, D.T., and Wahl, G.M. (1991) Recombinase-mediated gene activation and site-specific integration in mammalian cells. Science 251:1351-1355.
21. Waters, J.S., Webb, A., Cunningham, D., Clare, P.A., Raynaud, F., di Stefano, F., and Cotter, F.E. (2000) Phase I clinical and pharmacokinetic study of bcl-2 antisense oligonucleotide therapy in patients with non-Hodgkin's lymphoma. J. Clin. Oncol. 18:1812-1823*.*
22. Nabel, E.G., Yang, Z., Muller, D., Chang, A.E., Gao, X., Huang, L., Cho, K.J., and Nabel, G.J. (1994) Safety and toxicity of catheter gene delivery to the pulmonary vasculature in a patient with metastatic melanoma. Hum. Gene Ther. 5:1089-1094.
23. Nabel, G.J., Nabel, E.G., Yang, Z.Y., Fox, B.A., Plautz, G.E., Gao, X., Huang, L., Shu, S., Gordon, D., and Chang, A.E. (1993) Direct gene transfer with DNA-liposome complexes in melanoma: expression, biologic activity, and lack of toxicity in humans. Proc. Natl. Acad. Sci. U. S. A. 90:11307-11311.
24. Caplen, N.J., Alton, E.W., Middleton, P.G., Dorin, J.R., Stevenson B.J., Gao X., Durham S.R., Jeffrey P.K., Hodson M.E., Coutelle C. et al. (1995) Liposome-mediated CFTR gene transfer to the nasal epithelium of patients with cystic fibrosis. Nat. Med. 1:39-46.
25. Kalpana, G.V., Marmon, S., Wang, W., Crabtree, G.R., and Goff, S.P. (1994) Binding and stimulation of HIV-1 integrase by a human homolog of yeast transcription factor snf5. Science 266:2002-2006.
26. Bushman FD, Fujiwara T, and Cragie R. (1995) Retroviral DNA integration directed by HIV integration protein in vitro. Science 249:1555-1558.
27. Lampe, D.J., Churchill, M.E., and Robertson, H.M. (1996) A purified mariner transposase is sufficient to mediate transposition in vitro. EMBO J. 15:5470-5479.
28. Vos, J.C., De Baere, I., and Plasterk, R.H. (1996) Transposase is the only nematode protein required for in vitro transposition of Tc1. Genes Dev. 10:755-761.
29. Skalka, A.M. (1993)Retroviral DNA integration: lessons for transposon shufffing. Gene 135: 175-182.
30. Ellison, V. and Brown, P.O. (1994). A stable complex between integrase and viral DNA ends mediates human immunodeficiency virus integration in vitro. Proc. Natl. Acad. U.S.A. 91: 7316-7320.
31. Engelman, A. (1999) In vivo analysis of retroviral integrase structure and function. Adv. Virus Res. 52:411-426.
32. Fischer, S.E., Wienholds, E., and Plasterk, R.H. (2001) Regulated transposition of a fish transposon in the mouse germ line. Proc. Natl. Acad. Sci. U.S.A 98:6759-6764.
33. Flint, S.J., Enquist, L.W., Krug, R.M., Racaniello, V.R., and Skalka, A.M. (2000) Reverse transription and integration: hallmarks of the retroid viruses. In Principles of Virology: Molecular Biology, Pathogenesis, and Control. pp. 199-234, ASM Press, Washington, DC.
34. Goldhaber-Gordon, I., Williams, T.L., and Baker, T.A. (2001) DNA recognition sites activate MuA transposase to perform transposition of non-Mu DNA. J. Biol. Chem. 277:7694-7702.
35. Yant, S.R., Meuse, L., Chin, W., Ivics, Z., Izsvak, Z., and Kay, M.A. (2000) Somatic integration and long-term transgene expression in normal and haemophilic mice using a DNA transposon system. Nat. Genet. 25:35-41.
36. Bushman, F. (1995) Targeting retroviral integration. Science 267:1443-1444.
37. Kirchner, J., Connolly, C.M., and Sandmeyer, S.B. (1995) Requirement of RNA polymerase III transcription factors for in vitro position-specific integration of a retroviruslike element. Science 267:1488-1491.
38. Thyagarajan, B., Olivares, E.C., Hollis, R.P., Ginsburg, D.S., and Calos, M.P. (2001) Sitespecific genomic integration in mammalian cells mediated by phage φC31 integrase. Mol. Cell Biol. 21:3926-3934.
39. Rubin, G.M. and Spradling, A.C. (1983) Vectors for P element mediated gene transfer in Drosophila. Nucleic Acids Res. 11:6341-6351.
40. Bushman, F.D. (1994) Tethering human immunodeficiency virus 1 integrase to a DNA site directs integration to nearby sequences. Proc. Natl. Acad. Sci. U.S.A. 91:9233-9237.
41. Kulkosky, J., Katz, R., Merkel, G., and Skalka, A.M. (1995) Activities and substrate specificity of the evolutionarily conserved central domain of retroviral integrase. Virology 206:44.8-456.
42. Logie, C. and Stewart, F.A. (1995) Ligand-regulated site-specific recombination. Proc. Natl. Acad. Sci. U.S.A. 92:5940-5944.
43. Katz, R.A., Merkel, G., and Skalka, A.M. (1996) Targeting ofretroviral integrase by fusion to a heterologous DNA binding domain: in vitro activities and incorporation of a fusion protein into viral particles. Virology 217:178-190.
44. Bushman FD, and Miller MD. (1997) Tethering human immunodeficiency virus type 1 preintegration complexes to target DNA promotes integration at nearby sites. J. Virol. 71:458-464.
45. Holmes-Son, M.L., Appa, R.S., and Chow, S.A. (2001) Molecular genetics and target site specificity ofretroviral integration. Adv. Genet. 43:33-69.
46. Holmes-Son, M.L., and Chow, S.A. (2000) Integrase-LexA fusion proteins incorporated into human immunodeficiency virus type I that contains a catalytically inactive integrase gene are functional to mediate integration. J. Virol. 7.4:11548-11556.
47. Kaminski, J.M., Nguyen, K., Buyyounouski, M., and Pollack, A. (2002) Prostate cancer gene therapy and the role of radiation. Cancer Treat. Rev. 28:49-64.
48. Yant, S.R., Meuse, L., Chiu, W., Ivics, Z., Izsvak, Z., and Kay, M.A. (2000) Somatic integration and long-term transgene expression in normal and haemophilic mice using a DNA transposon system. Nature Genetics 25:35-41.
49. Loukeris, T.G., Livadaras, I., Arca, B., Zabalou, S., and Savakis, C. (1995) Gene transfer into the medfly, Ceratitis capitata, with a Drosophila hydei transposable element. Science 270:2002-2005.
50. Loukeris, T.G., Area, B., Livadaras, I., Dialektaki, G., and Savakis, C. (1995) Introduction of the transposable element Minos into the germline of Drosophila melanogaster. Proc. Natl. Acad. Sci. U.S.A. 92:9485-9489.
51. Lidholm, D.A., Lohe, A.R., and Hartl, D.L. (1993) The transposable element mariner mediates germline transformation in Drosophila melanogaster. Genetics 134:859-868.
52. Lohe, A.R.and Hart1, D.L. (1996) Germline transformation of Drosophila virilis with the transposable element mariner. Genetics 143:365-374.
53. Coates, C.J., Turney, C.L., Frommer, M., and O'Brochta, D.A. (1997) Interplasmid transposition of the mariner transposable element in non-drosophilid insects. Mol. Gen. Genet 253:728-733.
54. Coates, C.J., Jasinskiene, N., Miyashiro, L., and James, A.A. (1998) Mariner transposition and transformation of the yellow fever mosquito, Aedes aegypti. Proc. Natl. Acad. Sci. U.S.A. 95:3748-3751.
55. Rubin, E..J., Akerley, B.J., Novik ,V.N., Lampe, D.J., Husson, R.N., and Mekalanos, J.J. (1999) In vivo transposition of mariner-based elements in enteric bacteria and mycobacteria. Proc. Natl. Acad. Sci. U.S.A. 96:1645-1650.
56. Gueiros-Filho, F.J. and Beverly, S.M. (1997) Trans-kingdom transposition of the Drosophila element mariner within the protozoan Leishmania. Science 276:1716-1719.
57. Sherman, A., Dawson, A., Mather, C., Gilhooley, H., Li, Y., Mitchell, R., Finnegan, D., and Sang, H. (1998) Transposition of the Drosophila element mariner into the chicken germ line. Nat. Biotech. 16:1050-1053*.*

58. Raz, E., van Luenen, H.G., Schaerringer, B., Plasterk, R.H., Driever, W. (1998) Transposition of the nematode Caenorhabditis elegans Tc3 element in the zebrafish Danio rerio. Curr. Biol 8:82-88.
59. Fadool, J.M., Hartl, D.L., and Dowling, J.E.(1998) Transposition of the mariner element from Drosophila mauritiana in zebrafish. Proc. Natl. Acad. Sci. U.S.A. 95:5182-5186.
60. Luo, G., Ivics, Z., Izsvak, Z., and Bradley, A. (1998) Chromosomal transposition of a Tc1 /mariner-like element in a mouse embryonic stem cell. Proc. Natl. Acad. Sci. U.S.A. 95:10769-10773.
61. Ivics, Z., Hackett, P.B., Plasterk, R.H., and Izsvak, Z. (1997) Molecular reconstruction of Sleeping Beauty, a Tc1-like tranposon from fish, and its transposition in human cells. Cell 91:501-510.
62. Schouten, G.J., van Luenen, H.G., Verra, N.C., Valerio, D., and Plasterk, RH. (1998) Transposon Tc1 of the nematode Caenorhabditis elegans jumps in human cells. Nucleic Acids Res. 26:3013-3017.
63. Zhang, L., Sankar, U., Lampe, D.J., Robertson, H.M., and Graham, F.L. (1998) The Himar1 mariner transposase cloned in a recombinant adenovirus vector is functional in mammalian cells. Nucleic Acids Res. 16:3687-3693.
64. Kay, M.A., Meuse ,L., Gown, A.M., Linsley, P., Hollenbaugh, D., Aruffo, A., Ochs, H.D., and Wilson, C.B. (1997) Transient immunomodulation with anti-CD40 ligand antibody and CTLA4Ig enhances persistence and secondary adenovirus-mediated gene transfer into mouse liver. Proc. Natl. Acad. Sci. U.S.A. 94:4684-4691.
65. Hernandez, Y.J., Wang, J., Kearns, W.G., Loiler, S., Poirier, A., and Flotte, T.R. (1999) Latent adeno-associated virus infection elicits humoral but not cell-mediated immune responses in a nonhuman primate model J. Virol. 73:8549-8558.
66. Koduri, R.K., Miller, J.T., and Thammana P. (2001) An efficient homolgous recombination vector pTV(I) contains a hot spot for increased recombinant protein expression in Chinese hamster ovary cells. Gene 280:87-95.
67. Kang, Y.K. Park, J.S., Lee, C.S., Yeom, Y., Chung, A.S., and Lee, K.K. (1999) Efficient integration of short interspersed element-flanked foreign DNA via homologous recombination. J. Biol. Chem. 274:36585-36591.
68. Stevens, S.W. and Griffith, J.D. (1994) Human immunodeficiency virus type 1 may preferentially integrate into chromatin occupied by L1Hs repetitive elements. Proc. Natl. Acad. Sci. U.S.A. 91:5557-5561.
69. Stevens, S.W., and Griffith, J.D. (1996) Sequence analysis of the human DNA flanking sites of Human Immunodeficiency Virus Type 1 integration. J. Virol. 70:6459-6462.
70.Cox, G.S., Gutkin, D.W., Haas, M.J., and Cosgrove, D.E. (1998) Isolation of an Alu repetitive DNA binding protein and effect of CpG methylation on binding to its recognition sequence. Biochim. Biophys. Acta. 1493:302-318.
71. Chesnokov, I.N., and Schmid, C.W. (1995) Specific Alu binding protein from human sperm chromatin prevents DNA methylation. J. Biol. Chem. 270:18539-18542.
72. de Belle, I., Cai, S., and Kohwi-Shigematsu, T. (1998) The genomic sequences bound to special AT-rich sequence-binding protein 1 (SATB 1) in vivo in Jurkat T cells are tightly associated with the nuclear matrix at the bases of the chromatin loops. J. Cell. Biol. 141:335-348.
73. Chiang, Y., and Vishwanatha, J.K. Characterization of the HeLa cell 35 kDa Alu-element binding protein. Mol. Cell Biochem. 155:131-138.
74. Jurka, J., Walichiewicz, J., and Milosavljevic, A. (1992) Prototypic sequences for human repetitive DNA. J. Mol. Evol. 35:286-291.
75. Zhang, L., Dawson, A., and Finnegan, D.J. (2001) DNA-binding activity and subunit interaction of the mariner transposase. Nucleic Acids Res. 29:3566-3575.
76. Vega MA. (1991) Prospects for homologous recombination in human gene therapy Human Genet. 87:245-253.
77. Kren, B.T., Bandyopahyay, P., and Steer, C.L. (1998) In vivo site-directed mutagenesis of the factor IX gene by chimeric RNA/DNA oligonucleotides. Nat. Med. 4:285-290.
78. Strauss, M. (1998) The site-specific correction of genetic defects. Nat.Med. 4:274-275.
79. Van der Steege, G., Schuilenga-Hut, P.H., Buys, C.H., Scheffer, H., Pas, H.H., and Jonkman, M.F. (2001) Persistent failures in gene repair. Nat. Biotechnol. 19:305-306.
80. Albuquerque-Silva, J., Vassart, G., Lavinha, J., and Abramowicz, M.J. (2001) Chimeraplasty validation. Nat. Biotechnol. 19:1011*.*
81. Groth, A.C., Olivares, E.C., Thyagarajan, B., and Calos, M.P. (2000) A phage integrase directs efficient site-specific integration in human cells. Proc. Natl. Acad. Sci. U.S.A. 97:5995-6000.
82. Kirchner, J., Connolly, C. M., and Sandmeyer, S. B. Science 267, 1488-1491 (1995).
83. Morozov, A., Yung, E., and Kalpana, G. V. Prow. Natl. Acad. Sci. USA 95, 1120-1125 (1998).
84. Goulaouic H and Chow SA. (1996) Directed integration of viral DNA mediated by fusion proteins consisting of human immunodeficiency virus type 1 integrase and E. coli LexA protein. J Virol 70:37-46.
85. Manome Y, Kunieda T, et al. (1998) Trangene expression in maliganant glioma using a replication-defective adenoviral vector containing the Egr-1 promoter: activation by ionizing radiation or uptakr of radioactive iododeoxyuridine. Human Gene Ther 9:1409-1417.
86. Borrelli MJ, et al. (2001) Heat-activated transgene expression from adenoviru vectors infected into human prostate cancer cells. Cancer Res 61:1113-1121.
87. Xie X, Zhao X, et al (2001) Robust prostate-specific expression for targeted gene therapy based on the human kallikrein 2 promoter. Human Gene Ther 12:549-561.
88. Furth PA, et al. (1994) Temporal control of gene expression in transgenic mice by a tetracycline-responsive promoter. Proc. Natl. Acad. Sci. USA 91:9302-9306.
89. Izsvak Z, Ivics Z, Hackett PB. (1995) Characterization of a Tc-1 like transposable element in zebrafish (Danio rerio). Mol. Gen. Genet. 247:312-322.
90. Franz G and Savakis C. (1991) Minos, a new transposable element from Drosophila hydei, is a member of the Tc1-like family of transposons. Nucl. Acids Res. 19:6646.
91. Mernman PJ, Grimes CD, Ambroziak J, Hackett DA, Skinner P, and Simmons MJ. (1995) S elements: a family of Tc1-like transposons in the genome of Drosophila melanogaster. Genetics 141:1425-1438.
92. Ke Z, Grossman GL, Cornel AJ, Collins FH. (1996) Quetzal: a transposon of the Tc1 family in the mosquito Anopheles albimanus. Genetica 98:141-147.
93. Lam WL, Seo P, Robison K, Virk S, and Gilbert W. (1996) Discovery of amphibian Tc1-like transposon families. J Mol Biol 257:359-366.
94. Ivics Z, Izsvak Z, Minter A, Hackett PB. (1996) Identification of functional domains and evolution of Tc1-like transposable elements. Proc. Natl. Acad Sci USA 93: 5008-5013.
95. Richardson PD, Augustin LB, Kren BT, and Steer CJ. (2002) Gene repair and transposon-mediated gene therapy. Stem Cells 20:105-118.
96. Plasterk RH, Izsvak Z, Ivics Z. (1999) Resident aliens: the Tc1/mariner superfamily of transposable elements. Trends Genet 15:326-332.
97. Izsvak Z, Ivics Z, and Plasterk RH. (2000) Sleeping Beauty, a wide host-range transposon vector for genetic transformation in vertebrates. J. Mol. Biol. 302:93-102.
98. Furth, M. E., and Wicker, S. H. (1983) in Lambda II (Hendrix, R. W., Roberts, J. W., Stahl, F. W., and Weisberg, R. A., Eds.) pp 145-155, Cold Spring Harbor Laboratory, Cold Spring Harbor, N.Y.
99. Casjen, S., and Hendrix, R. (1988) in The Bacteriophages (Calendar, R., Ed.) pp 15-92, Plenum, New York, N.Y.
100. Black.L. W. (1989) Ann. Rev. Microbiol. 43, 267-292.
101. Fujisawa, H., and Morita, M. (1997) Genes to Cells 2, 537-545.
102. Catalano, C. E. (2000) Cellular and Molecular Life Sciences 57, 128-148.
103. Yang, Q., de Beer, T., Woods, L., Meyer, J., Manning, M., Overduin, M., and Catalano, C. E. (1999) Biochemistry 38, 465-477.
104. Yang, Q., Berton, N., Manning, M. C., and Catalano, C. E. (1999) Biochemistry 38, 14238-14247.
105. Landy, A. (1989) Ann. Rev. Biochem. 58, 913-949.
106. de Beer, T., Meyer, J., Ortega, M., Yang, Q., Maes, L., Duffy, C., Berton, N., Sippy, J., Overduin, M., Feiss, M., and Catalano, C. E. (2002) Mol. Cell 9, 981-991.
107. J.M. Kaminski, M.R. Huber, J.B. Summers, M.B. Ward, Design of a nonviral vector for site-selective, efficient integration into the human genome, Faseb J. 16 (2002) 1242-1247.
108. K. Abremski, R. Hoess, N. Sternberg, Studies on the properties of P1 site-specific recombination: evidence for topologically unlinked products following recombination, Cell 32 (1983)1301-1311.
109. F. Guo, D.N. Gopaul, G.D. van Duyne, Structure of Cre recombinase complexed with DNA in a site-specific recombination synapse, Nature 389 (1997) 40-46.
110. H.A. Greisman, C.O. Pabo, A general strategy for selecting high-affinity zinc finger proteins for diverse DNA target sites, Science 275 (1997) 657-661.
111. A.F. Kolb, S.G. Siddell, Genomic targeting with an MBP-Cre fusion protein, Gene 183 (1996) 53-60.

### G. Sequences

SEQ ID No: 1 TCCGGCCGGAACCGGCTTT
SEQ ID No: 2 CG GGATCCCACCTATGGAAGGAAGATCAGATTT
SEQ ID No: 3 AGATTACTCGAGTCAAAGTGTTTTGTATGATCTCG
SEQ ID No: 4 AAGATCTGATCCGTCGAC
SEQ ID No: 5 CGGGATCCCACCTATGAATTATGGCGTGGAGAA
SEQ ID No: 6 AGATTACTCGAGTCAGTTGTACAGCTGCAATCCCA
SEQ ID No: 7 GGAAGCCCTGCAAAGTAAA

## Claims

1. A composition comprising a nucleic acid construct comprising a transgene flanked by two terminal repeats and a nucleic acid encoding an integrating enzyme under the control of a promoter element, wherein the integrating enzyme is a chimeric integrating enzyme comprising a host-specific DNA binding domain and wherein the nucleic acid encoding the transgene and the nucleic acid encoding the chimeric integrating enzyme are the same nucleic acid, and wherein the chimeric integrating enzyme is a transposase.

2. The composition of claim 1, wherein the promoter element is a promoter/enhancer.

3. The composition of claim 1, wherein the promoter is a site-specific promoter.

4. The composition of claim 3, wherein the site-specific promoter can be selected from at least the group consisting of the glial fibrillary acetic protein (GFAP) promoter, myelin basic (MBP) promoter, MCK promoter, NSE promoter, nestin promoter, synapsin promoter, Insulin 2 (Ins2) promoter, PSA promoter, albumin promoter, TRP-1 promoter, the tyrosinase promoter, the EIIA promoter, a promoter specific for breast tissue, such as the WAP promoter, a promoter specific for ovarian tissue, such as the ACTB promoter, or a promoter specific for bone tissue.

5. The composition of claim 1, wherein the promoter is inducible.

6. The composition of claim 5, wherein the inducible promoter can be selected from at least one of the group consisting of human heat shock promoter, Egr-1 promoter, tetracycline promoter, cre-lox recombinase system, and the human glandular kallikrein 2 (hK2) promoter.

7. The composition of claim 1, wherein the chimeric transposase can be selected from at least one of the group consisting of Sleeping Beauty (SB), mosl, piggyBac, Himarl, Hermes, To 12 element, Pokey, Tn7, Tn916, maT, Tc1/mariner and Tc3.

8. The composition of claim 1; wherein the host-specific DNA binding domain of the chimeric integrating enzyme is fused to the transposase's N-terminus.

9. The composition of claim 1, wherein the host-specific DNA binding domain of the chimeric integrating enzyme is fused to the transposase's C-terminus.

10. The composition of claim 1, wherein the nucleic acid sequence encoding the chimeric integrating enzyme is located outside the terminal repeats.

11. The composition of any one of the preceding claims, wherein the host-specific DNA binding domain is selected from the group consisting of homeobox proteins, zinc finger proteins, hormone receptors, helix-turn-helix proteins, helix-loop-helix proteins, basic-Zip proteins (bZip), and beta-ribbon factors.

12. A nucleic acid construct comprising i) a transgene flanked by two terminal repeats, ii) a chimeric integrating enzyme in the form of a transposase under the control of a promoter element capable of directing expression of said transposase, and iii) a host-specific DNA binding domain.

13. A composition according to any one of the claims 1-11 or a nucleic acid construct according to claim 12 for use as a medicament.

## Patentansprüche

1. Eine Zusammensetzung, umfassend ein Nukleinsäurekonstrukt, welches ein Transgen, flankiert von zwei terminalen Wiederholungen, und eine Nukleinsäure, welche für ein integrierendes Enzym unter der Kontrolle eines Promotorelements kodiert, umfasst, wobei das integrierende Enzym ein chimäres integrierendes Enzym, umfassend eine wirtsspezifische DNA-Bindungsdomäne, ist, und wobei die Nukleinsäure, welche für das Transgen kodiert, und die Nukleinsäure, welche für das chimäre integrierende Enzym kodiert, dieselbe Nukleinsäure sind, und wobei das chimäre integrierende Enzym eine Transposase ist.

2. Die Zusammensetzung nach Anspruch 1, wobei das Promotorelement ein Promotor/Verstärker ist.

3. Die Zusammensetzung nach Anspruch 1, wobei der Promotor ein ortsspezifischer Promotor ist.

4. Die Zusammensetzung nach Anspruch 3, wobei der ortsspezifische Promotor ausgewählt sein kann aus mindestens der Gruppe bestehend aus saurem Gliafaserprotein (glial fibrillary acetic protein, GFAP)-Promotor, basischem Myelin (myelin basic, MBP)-Promotor, MCK-Promotor, NSE-Promotor, Nestin-Promotor, Synapsin-Promotor, Insulin 2 (Ins2)-Promotor, PSA-Promotor, Albumin-Promotor, TRP-1-Promotor, dem Tyrosinase-Promotor, dem EIIA-Promotor, einem für Brustgewebe spezifischen Promotor, wie z.B. dem WAP-Promotor, einem für Ovarialgewebe spezifischen Promotor, wie z.B. dem ACTB-Promotor, oder einem für Knochengewebe spezifischen Promotor.

5. Die Zusammensetzung nach Anspruch 1, wobei der Promotor induzierbar ist.

6. Die Zusammensetzung nach Anspruch 5, wobei der induzierbare Promotor ausgewählt sein kann aus mindestens einem aus der Gruppe bestehend aus humanem Hitzeschock-Promotor, Egr-1-Promotor, Tetrazyklin-Promotor, cre-lox-Rekombinasesystem und dem humanen Drüsen-Kallikrein2 (hK2)-Promotor.

7. Die Zusammensetzung nach Anspruch 1, wobei die chimäre Transposase ausgewählt sein kann aus mindestens einer aus der Gruppe bestehend aus Sleeping Beauty (SB), mos1, piggyBac, Himarl, Hermes, To12-Element, Pokey, Tn7, Tn916, maT, Tc1/Mariner und Tc3.

8. Die Zusammensetzung nach Anspruch 1, wobei die wirtsspezifische DNA-Bindungsdomäne des chimären integrierenden Enzyms an den N-Terminus der Transposase fusioniert ist.

9. Die Zusammensetzung nach Anspruch 1, wobei die wirtsspezifische DNA-Bindungsdomäne des chimären integrierenden Enzyms an den C-Terminus der Transposase fusioniert ist.

10. Die Zusammensetzung nach Anspruch 1, wobei sich die Nukleinsäuresequenz, welche für das chimäre integrierende Enzym kodiert, außerhalb der terminalen Wiederholungen befindet.

11. Die Zusammensetzung nach einem der vorangehenden Ansprüche, wobei die wirtsspezifische DNA-Bindungsdomäne ausgewählt ist aus der Gruppe bestehend aus Homeobox-Proteinen, Zinkfinger-Proteinen, Hormonrezeptoren, Helix-Tum-Helix-Proteinen, Helix-Loop-Helix-Proteinen, basischen Zip-Proteinen (basic-Zip proteins, bZip), und Beta-Ribbon-Faktoren.

12. Ein Nukleinsäurekonstrukt umfassend i) ein Transgen flankiert von zwei terminalen Wiederholungen, ii) ein chimäres integrierendes Enzym in Form einer Transposase unter der Kontrolle eines Promotor-Elements, das imstande ist, die Expression von besagter Transposase zu steuern, und iii) eine wirtsspezifische DNA-Bindungsdomäne.

13. Die Zusammensetzung nach einem der Ansprüche 1 bis 11 oder ein Nukleinsäurekonstrukt nach Anspruch 12 zur Verwendung als Medikament.

## Revendications

1. Composition comportant un complexe d'acide nucléique comprenant un transgène flanqué de deux répétitions terminales et un acide nucléique codant une enzyme intégratrice sous le contrôle d'un élément promoteur, où l'enzyme intégratrice est une enzyme intégratrice chimérique comprenant un domaine de liaison à l'ADN spécifique à l'hôte et où l'acide nucléique codant le transgène et l'acide nucléique codant l'enzyme intégratrice chimérique constituent le même acide nucléique, et où l'enzyme intégratrice chimérique est une transposase.

2. La composition de la revendication 1, où l'élément promoteur est un promoteur / amplificateur.

3. La composition de la revendication 1, où le promoteur est un promoteur spécifique au site.

4. La composition de la revendication 3, où le promoteur spécifique au site peut être sélectionné dans au moins le groupe comportant le promoteur de la protéine acide fibrillaire gliale (GFAP), le promoteur de la protéine basique de la myéline (MBP), le promoteur de la MCK, le promoteur du NSE, le promoteur de la nestine, le promoteur de la synapsine, le promoteur de l'Insuline 2 (Ins2), le promoteur du PSA, le promoteur de l'albumine, le promoteur du TRP-1, le promoteur de la tyrosinase, le promoteur de l'EIIA, un promoteur spécifique au tissu mammaire tel que le promoteur de la WAP, un promoteur spécifique au tissu ovarien tel que le promoteur de l'ACTB, ou un promoteur spécifique au tissu osseux.

5. La composition de la revendication 1, où le promoteur est inductible.

6. La composition de la revendication 5, où le promoteur inductible peut être sélectionné parmi au moins l'un des membres du groupe comprenant le promoteur du choc thermique humain, le promoteur de l'Egr-1, le promoteur de la tétracycline, le système de recombinase cre-lox, et le promoteur de la kallicréine glandulaire humaine 2 (hK2).

7. La composition de la revendication 1, où la transposase chimérique peut être sélectionnée parmi au moins l'un des membres du groupe comprenant Sleeping Beauty (SB), mosl, piggyBac, Himarl, Hermes, l'élément To12, Pokey, Tn7, Tn916, maT, Tc1/mariner et Tc3.

8. La composition de la revendication 1, où le domaine de liaison à l'ADN spécifique à l'hôte de l'enzyme intégratrice chimérique est fusionné au terminus N de la transposase.

9. La composition de la revendication 1, où le domaine de liaison à l'ADN spécifique à l'hôte de l'enzyme intégratrice chimérique est fusionné au terminus C de la transposase.

10. La composition de la revendication 1, où la séquence d'acide nucléique codant l'enzyme intégratrice chimérique est située en dehors des répétitions terminales.

11. La composition de l'une quelconque des revendications précédentes, où le domaine de liaison à l'ADN spécifique à l'hôte est sélectionné dans le groupe comprenant les protéines homéobox, les protéines à doigt de zinc, les récepteurs hormonaux, les protéines hélice-tour-hélice, les protéines hélice-boucle-hélice, les protéines à motif Zip basique (bZip) et les facteurs de type ruban bêta.

12. Complexe d'acide nucléique comportant i) un transgène flanqué de deux répétitions terminales, ii) une enzyme intégratrice chimérique sous la forme d'une transposase sous le contrôle d'un élément promoteur apte à diriger l'expression de ladite transposase, et iii) un domaine de liaison à l'ADN spécifique à l'hôte.

13. Composition selon l'une quelconque des revendications 1 à 11 ou complexe d'acide nucléique selon la revendication 12 destiné(e) à être utilisé(e) comme médicament.
